(19) **Europäisches Patentamt** / **European Patent Office** / **Office européen des brevets**

(11) **EP 1 810 610 B1**

(12) **EUROPEAN PATENT SPECIFICATION**

(45) Date of publication and mention of the grant of the patent:
**14.09.2016 Bulletin 2016/37**

(51) Int Cl.:
**A61B 5/00** *(2006.01)*

(21) Application number: **07001327.1**

(22) Date of filing: **22.01.2007**

(54) **Method and apparatus for analyzing characteristic information of object with the use of mutual interaction between ultrasound wave and light**

Verfahren und Vorrichtung zur Analyse von Objekteigenschaftsinformationen mittels gegenseitiger Interaktion zwischen Ultraschallwellen und Licht

Procédé et appareil d'analyse des informations caractéristiques d'un objet grâce à l'utilisation de l'interaction réciproque entre l'onde ultrasonique et la lumière

(84) Designated Contracting States:
**DE FR GB IT**

(30) Priority: **20.01.2006 JP 2006012898**
**15.01.2007 JP 2007005680**
**15.01.2007 JP 2007005678**
**15.01.2007 JP 2007005679**

(43) Date of publication of application:
**25.07.2007 Bulletin 2007/30**

(60) Divisional application:
**10012569.9 / 2 275 022**

(73) Proprietor: **Olympus Corporation**
**Hachioji-shi**
**Tokyo**
**192-8507 (JP)**

(72) Inventor: **Igarashi, Makoto**
**Tokyo 192-8507 (JP)**

(74) Representative: **von Hellfeld, Axel**
**Wuesthoff & Wuesthoff**
**Patentanwälte PartG mbB**
**Schweigerstrasse 2**
**81541 München (DE)**

(56) References cited:
**JP-A- 2005 224 399 US-B1- 6 245 015**

• **MAHAN G D ET AL: "ULTRASONIC TAGGING OF LIGHT: THEORY" PROCEEDINGS OF THE NATIONAL ACADEMY OF SCIENCES OF USA, NATIONAL ACADEMY OF SCIENCE, WASHINGTON, DC, US, vol. 95, 1998, pages 14015-14019, XP001028174 ISSN: 0027-8424**
• **Peter O'Shea: "Chapter 41: Phase Measurement" In: "The Measurement, Instrumentation and Sensors Handbook", 1999, Crc Press Llc**

**Description**

CROSS REFERENCES TO RELATED APPLICATIONS

This application is based on and claims the benefit of priorities from earlier Japanese Patent Application No. 2006-012898 filed January 20, 2006, and Japanese Patent Application No. 2007-005680 filed January 15, 2007 , Japanese Patent Application No. 2007-005678 filed January 15, 2007 , and Japanese Patent Application No. 2007-005679 filed January 15.

Background of the Invention

(Field of the Invention)

The present invention relates to a technique for analyzing an internal structure of an object to be diagnosed, and in particular, to a method and apparatus for analyzing information indicative of a characteristic of a region to be examined of an object to be diagnosed with the use of mutual interaction between the ultrasound waves and light, and an endoscope system that uses such an analyzing technique.

(Description of Related Art)

Recently, various technologies are suggested and put into practice as optical tomography imaging system for living organism, such as optical CT or optical coherence tomography (hereinafter referred to as "OCT") and photoacoustic tomography.

The optical CT utilizes near-infrared light of a wavelength ranging from 700 nm to 1200 nm, whose light scattering influence on a living organism is comparatively weak. Therefore, the optical CT enables to obtain tomograms of deep part in a living organism, such as up to several centimeters under the mucous membrane.

The OCT utilizing interference enables to obtain tomography images of a living organism up to a depth of about 2 mm with high resolution (up to several tens of $\mu$m) in short time. The OCT is a technology that has already been put into practice in diagnosing retinopathy in an ophthalmic field, and has attracted very keen interest in the medical world.

Although the optical CT can provide information on a deep part of a living organism, its spatial resolution is as low as several millimeters. On the other hand, it is difficult for the OCT to enable observation at a depth of about 2 mm or more under the mucous membrane and to provide good quality image of tumor tissue, such as cancer.

This is because the optical coherence is extremely disturbed by the influence of absorption or serious scattering of blood in the deep part of a living organism and tumor tissue.

A paper entitled "Optical tomogram observation at deep part of a scatterer utilizing interaction of pulsed ultrasound wave and light" by Maki HISAKA et al., Optics, Vol. 29, 10, pp., 631-634 (2000) reports an example of attempting to perform optical imaging for absorbed light to a depth of about 1 cm in and under the cortical layer of the mucous membrane, by radiating pulsed ultrasound waves and light beams to living organism and by detecting the radiated light beams that have been modulated by the pulsed ultrasound waves.

Further, Japanese Patent Laid-Open Publication No. 2005-224399 also discloses a system for obtaining imaging of absorbed light in and under the cortical layer of the mucous membrane by radiating ultrasound pulses and light beams to a living organism and by detecting the radiated light beams that have been modulated by the pulsed ultrasound waves.

However, the prior art references mentioned above are only specialized in light-absorbing imaging and do not provide a technique for obtaining information on scattering light caused by the configuration or structure of tissue.

In other words, light scattering characteristics (hereinafter referred to as "scattering characteristics") are altered, in particular, by the alteration of the biological structure of the tissue of a living organism, such as the alteration in the concentration of nuclear chromatin accompanying the canceration of a tumor and the alteration in the spatial distribution of nuclei. Accordingly, it is desirable to obtain imaging information on light scattering having high correlativity to the alteration of tissue structure associated, for example, with cancer tissue.

It will be appreciated that light scattering having high correlativity to the alteration of tissue structure derives from the real part of a complex refractive index in the tissue region. On the other hand, the imaginary part of the complex refractive index relates to light absorption. Thus, perception of the real and imaginary parts of the complex refractive index may help obtain two-dimensional or three-dimensional information on the scattering and absorption characteristics.

Japanese Patent Laid-Open Publication No. 2000-197635 discloses a method based on a diffusion-type wave formula, in which a scattering coefficient and an absorption coefficient are recorded by radiating ultrasound waves focusing on a living organism and also by radiating light beams from a plurality of light sources, such as lasers, from various directions to detect scattered light in a region where the ultrasound waves have been focused on, with the aid of a plurality of detectors arranged around the living organism.

The plurality of light sources and detectors employed in the prior art disclosed in this Japanese Patent Laid-Open Publication No. 2000-197635 raise some problems, for example, that lots of labor and time have to be consumed in setting the light sources and detectors so as to be ready for measurement, and that, in a signal processing system downstream of the detectors, adjustment may be required according to the arrangement of the detectors, thus imposing some load on users.

Further, in this prior art, no control means is provided for adequately receiving light by the detectors in case the light sources or a region to be examined are positionally changed for scanning. This raises a problem that imaged information, for example, cannot be readily obtained.

Therefore, a system and a method have been desired, in which high spatial resolution can be ensured to produce characteristics information on a living organism, or an object, including scattering light information (hereinafter referred to as "scattering information"), in case a region to be examined is located at a deep part of the object.

Since the conventional system is comparatively large in size, more compact system has been desired. Further, since a larger spatial region in which interference is caused between light beams and ultrasound waves will result in lower resolution, it has also been desired to extract the scattering information with good resolution only on the region to be examined. In addition, it has been desired to collect information required for imaging in shorter time.

Summary of the Invention

The present invention has been made in light of the problems as described above, and has as its object to provide an object information analyzing apparatus, an endoscope system and an object information analyzing method, which are able to readily obtain characteristics information on the object, including scattering information on a region to be examined of the object, with good resolution.

The present invention concerns an apparatus for analyzing information indicative of a characteristic of an object to be diagnosed having the features of claim 1 and a method of analyzing information indicative of a characteristic of an object to be diagnosed having the features of claim 23.

Brief Description of the Drawings

In the appended drawings:

Fig. 1 is a block diagram illustrating a basic configuration of an object information analyzing apparatus, according to first to fourth embodiments of the present invention;

Fig. 2 is a block diagram illustrating a general arrangement of an optical imaging apparatus serving as object information analyzing apparatus, according to the first embodiment of the present invention;

Fig. 3 is an enlarged view of an ultrasonic convergence point including its neighboring area;

Fig. 4 is a flow diagram illustrating contents of an operation performed by the optical imaging apparatus, according to the first embodiment;

Fig. 5 shows timing charts explaining the operation of the optical imaging apparatus, according to the first embodiment of the present invention;

Fig. 6 is a block diagram illustrating a general arrangement of an optical imaging apparatus serving as object information analyzing apparatus, according to the second embodiment of the present invention;

Fig. 7 is a flow diagram illustrating contents of an operation performed by the optical imaging apparatus, according to the second embodiment;

Fig. 8 is a block diagram illustrating a general arrangement of an optical imaging apparatus, according to a first modification of the second embodiment;

Fig. 9 is a block diagram illustrating a general arrangement of an optical imaging apparatus, according to a second modification of the second embodiment;

Fig. 10 shows timing charts explaining the operation of the optical Imaging apparatus, according to the second modification;

Fig. 11 illustrates structures of a lens holder and a transducer holder, according to a third modification of the second embodiment;

Fig. 12 illustrates structures of a lens holder and a transducer holder, according to a fourth modification of the second embodiment;

Fig. 13 illustrates a structure of one portion, according to a fifth modification of the second embodiment;

Fig. 14 is a block diagram illustrating a general arrangement of an optical imaging apparatus serving as object information analyzing apparatus, according to the third embodiment of the present invention;

Fig. 15 illustrates a structure at an end face of an optical fiber;

Fig. 16 illustrates an example of an arrangement of an optical coupler;

Fig. 17 is a block diagram illustrating an arrangement of an endoscope apparatus provided with the optical imaging apparatus, according to a first modification of the third embodiment;

Fig. 18 illustrates an arrangement of a tip portion of an endoscope including its neighboring area, according to a second modification of the third embodiment;

Fig. 19 is a perspective view illustrating an arrangement of an optical fiber according to a third modification of the third embodiment;

Fig. 20 is a block diagram illustrating an arrangement of an optical imaging apparatus serving as object information analyzing apparatus, according to a fourth embodiment of the present invention;

Fig. 21 is an illustration explaining an operation for obtaining two-dimensional optical imaging information through one-dimensional scanning, according to the fourth embodiment;

Fig. 22 is a schematic diagram illustrating an ultrasonic emitter/radiator/detector array, according to a modification of the fourth embodiment

Fig. 23 is an illustration explaining an operation of the ultrasonic emitter/radiator/detector array illustrated in Fig. 22;

Fig. 24 is a block diagram illustrating an example of a typical arrangement of an object information analyzing apparatus, according to fifth to eleventh examples not forming part of the present invention;

Fig. 25 is a block diagram illustrating a general arrangement of an optical imaging apparatus serving as object information analyzing apparatus, according to the fifth example;

Fig. 26 is an illustration explaining Doppler shifting in an ultrasonic convergence region including its neighboring area;

Fig. 27 is a flow diagram explaining an operation of the optical imaging apparatus, according to the fifth example;

Fig. 28 illustrates an example of a waveform for an angular frequency of real-part components in a Fourier transformed signal;

Fig. 29 illustrates structures of a lens holder and a transducer holder, according to a first modification of the fifth example;

Fig. 30 is an illustration explaining Doppler shifting in an ultrasonic convergence region including its neighboring area, according to a second modification of the fifth example;

Fig. 31 is a block diagram illustrating a general arrangement of an optical imaging apparatus serving as object information analyzing apparatus, according to the sixth example;

Fig. 32 illustrates structures of a lens holder and a transducer holder, according to a modification of the sixth example;

Fig. 33 is a block diagram illustrating a general arrangement of an optical imaging apparatus serving as object information analyzing apparatus, according to the seventh example;

Fig. 34 is a blow diagram explaining an operation of the optical imaging apparatus, according to the seventh example;

Fig. 35 illustrates an example of a waveform for an angular frequency of real-part components in a Fourier-transformed signal;

Fig. 36 is a block diagram illustrating a general arrangement of an optical imaging apparatus, according to a first modification of the seventh example;

Fig. 37 is a block diagram illustrating a general arrangement of an optical imaging apparatus, according to a second modification of the seventh example;

Fig. 38 is a block diagram illustrating a general arrangement of an optical imaging apparatus serving as object information analyzing apparatus, according to the eighth example;

Fig. 39 is a flow diagram explaining an operation of the optical imaging apparatus, according to the eighth example;

Fig. 40 is a block diagram illustrating a general arrangement of an optical imaging apparatus, according to a modification of the eighth example;

Fig. 41 is a block diagram Illustrating a general arrangement of an optical imaging apparatus serving as object information analyzing apparatus, according to a ninth example;

Fig. 42 shows timing charts explaining the operation of the ninth example;

Fig. 43 is a block diagram illustrating a general arrangement of an optical imaging apparatus, according to a first modification of the ninth example;

Fig. 44 is a block diagram illustrating a general arrangement of an optical imaging apparatus, according to a second modification of the ninth example;

Fig. 45 is a block diagram illustrating a general arrangement of an optical imaging apparatus, according to a third modification of the ninth example;

Fig. 46 is a block diagram illustrating an arrangement of one portion of an optical Imaging apparatus, according to a fourth modification of the ninth example;

Fig. 47 is a block diagram illustrating a general arrangement of an optical imaging apparatus serving as object information analyzing apparatus, according to the tenth example; ;

Fig. 48 illustrates a structure of an end face of an optical fiber;

Fig. 49 illustrates an example of an arrangement of an optical coupler;

Fig. 50 is a block diagram illustrating a general arrangement of an optical imaging apparatus, according to a first modification of the tenth example;

Fig. 51 is a block diagram illustrating an arrangement of an endoscope apparatus provided with an optical imaging apparatus, according to a second modification of the tenth example;

Fig. 52 illustrates a tip side of an endoscope provided with an optical imaging apparatus, according to a third modification of the tenth example;

Fig. 53 is a perspective view illustrating an arrangement of an optical fiber, according to a fourth modification of the tenth example;

Fig. 54 is a block diagram illustrating an arrangement of an optical imaging apparatus serving as object information analyzing apparatus, according to the eleventh example;

Fig. 55 is a schematic diagram illustrating an ultrasonic emitter/radiator/detector array according to a first modification of the eleventh example;

Fig. 56 is a block diagram illustrating a basic arrangement of an object information analyzing apparatus, according to twelfth example and thirteenth to fifteenth embodiments of the present invention;

Fig. 57 is a block diagram illustrating a general arrangement of an optical imaging apparatus serving as object information analyzing apparatus, according to the twelfth example;

Fig. 58 is an illustration explaining Doppler shifting in an ultrasonic convergence region including its neighboring area according to the twelfth example;

Fig. 59 is a flow diagram illustrating contents of an operation of the optical imaging apparatus, according to the twelfth example;

Fig. 60 shows timing charts explaining the operation of the optical imaging apparatus, according to the twelfth example;

Fig. 61 is a block diagram illustrating an arrangement of a Fourier transform circuit including its neighboring portion, which can be incorporated into an optical imaging apparatus, according to a first modification of the twelfth example;

Fig. 62 is a block diagram illustrating a general arrangement of an optical imaging apparatus, according to a second modification of the twelfth example;

Fig. 63 is a block diagram illustrating a general arrangement of an optical imaging apparatus, according to a third modification of the twelfth example;

Fig. 64 is a block diagram illustrating a general arrangement of an optical imaging apparatus serving as object information analyzing apparatus, according to the thirteenth embodiment of the present invention;

Fig. 65 is a flow diagram illustrating the contents of an operation of an optical imaging apparatus, according to the thirteenth embodiment;

Fig. 66 is a block diagram illustrating a general arrangement of an optical imaging apparatus, according to a modification of the thirteenth embodiment;

Fig. 67 schematically illustrates an example of a pulse waveform of a light beam reflected from a tissue of a living organism;

Fig. 68 is a block diagram illustrating a general arrangement of an optical imaging apparatus serving as object information analyzing apparatus, according to the fourteenth embodiment of the present invention;

Fig. 69 illustrates a structure of an end face of an optical fiber;

Fig. 70 illustrates an example of an arrangement of an optical coupler;

Fig. 71 is a block diagram illustrating a general arrangement of an optical imaging apparatus, according to a first modification of the fourteenth embodiment;

Fig. 72 is a block diagram illustrating a general arrangement of an optical imaging apparatus, according to a second modification of the fourteenth embodiment;

Fig. 73 is a block diagram illustrating an arrangement of an endoscope apparatus serving as object information analyzing apparatus, according to the fifteenth embodiment of the present invention;

Fig. 74 is a block diagram illustrating an arrangement of an endoscope apparatus, according to a first modification of the fifteenth embodiment;

Fig. 75 illustrates an arrangement of a tip side of an endoscope illustrated in Fig. 74; and

Fig. 76 is a perspective view illustrating an arrangement of an optical fiber, according to a second modification of the fifteenth embodiment.

Detailed Description of Preferred Embodiments

Hereinafter, various preferred embodiments of according to a method and apparatus for analyzing (, obtaining, observing or imaging) information indicative of an internal structural characteristic of an object (, specimen or sample) being diagnosed, which are based on the present invention, will now be descried. In the following, the "information indicative of an internal structural characteristic of an object" is simply referred to as "object information." Some of the following embodiments also include endoscope systems in which the analyzing technique realized by the analyzing apparatus and method is functionally reduced into practice. The term "object information" means information indicative of a characteristic of a region to be examined (target region) of an object to be diagnosed.

The object information analyzing apparatuses described in the following embodiments (i.e., first to fifteenth embodiments and modifications thereof) are based on the structure that comprises "an ultrasound generator generating an ultrasound wave toward an object along a direction given by a desired spatial axis; a light generator generating light toward a region to be examined within the object, the ultrasound sound generated being transmitted to the region; a light receiver receiving light coming from the region and outputting an electric signal corresponding to the received light; an information acquiring unit acquiring information indicative of scattering of the light at the region by using either the signal outputted from the light receiver or the light coming from the region; and an information producer producing information indicative of a characteristic of the region based on the scattering information acquired by the information acquiring unit."

Of the embodiments, in particular, the first to fourth embodiments are characteristic of having "the information obtaining unit including a phase-information extractor extracting information indicative of a phase from either the signal outputted from the light receiver or the light coming from the region to be examined and a scattering information calculator calculating the Information indicative of the scattering of the light at the region to be examined, on the basis of the information indicative of the phase extracted."

The fifth to eleventh examples not forming part of the claimed invention are characteristic of having "the information obtaining unit including a frequency-information extractor extracting information indicative of a Doppler-shift frequency of the light from either the signal outputted from the light receiver or the light coming from the region to be examined and a scattering information calculator calculating the information indicative of the scattering of the light at the region to be examined, on the basis of the information indicative of the frequency extracted."

Further, the twelfth example and thirteenth to fifteenth embodiments are characteristic of having "the ultrasound generator composed of a pulsed ultrasound generator generating a pulsed ultrasound wave serving as the ultrasound wave and the light generator composed of a pulsed light generator generating a pulsed light along an axis which is set at a spatially different angle from the axis along which the pulsed ultrasound wave is transmitted toward the object, the angle being equal to or less than a predetermined amount. In this case, it is preferred to have a synchronizer allowing the pulsed ultrasound wave and the pulsed light to be generated in synchronism at a predetermined timing.

Since there are many embodiments, it is assumed that the following description should attach importance to how smooth the respective embodiments are developed, with less labor in reading back previous embodiments. Hence some technical matters are to be included repeatedly in some of the embodiments.

**(First embodiment)**

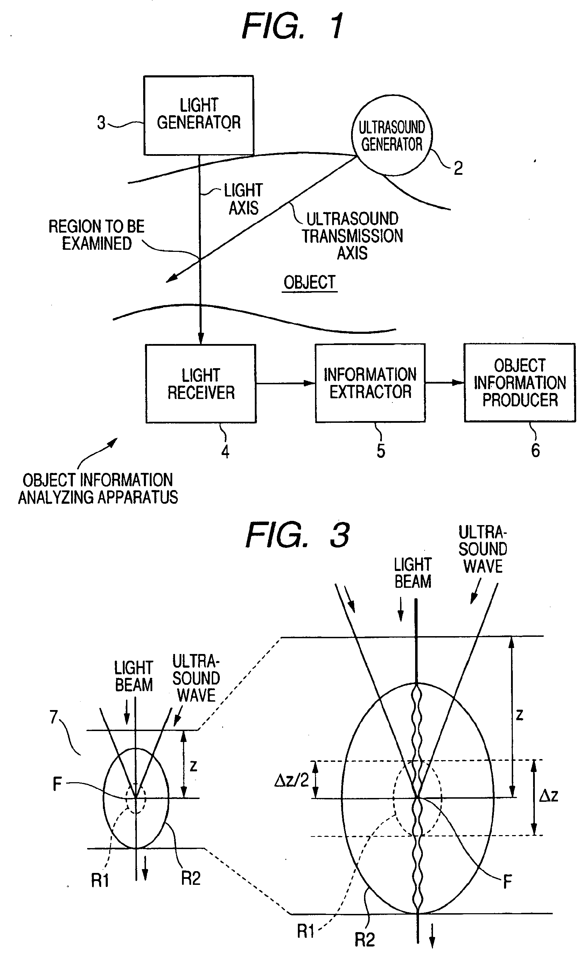

Referring to Figs. 1 - 5, a first embodiment of the present invention will now be described.

Fig. 1 is a block diagram illustrating a basic configuration of an object information analyzing apparatus of the present invention. As shown in Fig. 1, the object information analyzing apparatus of the present invention is provided with an ultrasound generator 2 capable of generating ultrasound waves so as to be transmitted into an object along a predetermined ultrasound transmission axis, and a light generator 3 capable of generating a light beam that reaches a region to be examined in an object to which the ultrasound waves generated by the ultrasound generator 2 are transmitted.

The object information analyzing apparatus is also provided with a light receiver 4 which is arranged being oriented to the region to be examined so as to receive the light beam that has been generated by the light generator 3 and passed through the region to be examined, and an information extractor 5 for extracting information corresponding at least to a real part of a complex refractive index of the region to be examined where the generated light beam has reached, based on phase information which is based on received-light signals that have been received by the light receiver 4.

As will be described later in this first embodiment, for example, the information extractor 5 includes a phase information extractor for extracting phase information corresponding at least to the real part of the complex refractive index, from transmitted or scattered light (or reflected light) received by the light receiver 4, and a scattering light information extractor (hereinafter referred to as "scattering information extractor") for extracting scattering information from the phase information.

In Fig. 1, the light receiver 4 receives a light beam transmitted through an object as an observation light beam, the light beam being received on an optical axis to which a light beam generated by the light generator 3 is radiated. Although the light receiver 4 is shown in an arrangement (configuration) example as being corresponding to a transmitted light receiver, the arrangement is not limited to this. For example, as in a second embodiment, the light receiver 4 may be arranged as a reflected light receiver which receives reflected light as observation light.

The object information analyzing apparatus is further provided with an object information producer 6 for producing object characteristics information corresponding to the region to be examined to which the light beam has reached, based on the information extracted by the information extractor 5.

The object information producer 6 has an image former which forms and displays image based, for example, on scattering information at individual positions of two-dimensional or three-dimensional scanning of the region to be examined of an object.

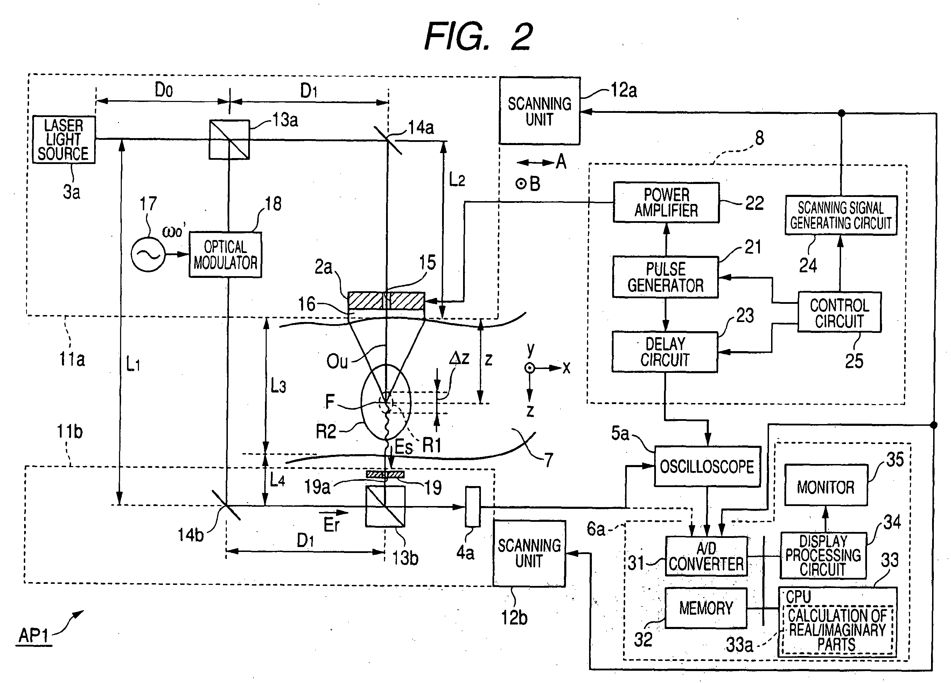

The arrangement of the object information analyzing apparatus is now described in detail with reference to Fig. 2.

An optical imaging apparatus AP1 shown in Fig. 2 according to the first embodiment has a laser device (light source) 3a for generating coherent light, for example, serving as the light generator 3 and disposed in a first unit 11a. The light source for generating light is not intended to be limited to the laser device 3a that generates coherent light, but instead a heat/light source such as a xenon light source or a halogen light source, or an LED (light emitting diode) or an SLD (Super Luminescent Diode) may be used.

The first unit 11a is incorporated with an ultrasound transducer 2a serving as the ultrasound generator 2.

A second unit 11b incorporating a photo detector 4a, for example, serving as the light receiver 4 is arranged opposed to the first unit 11a with a tissue 7 of living organism (hereinafter just referred to as "tissue 7") being Interposed therebetween.

Scanning units 12a, 12b are attached to the first and second units 11a, 11b, respectively. The scanning units 12a, 12b serve as scanning motion providers for providing the units 11a, 11b, respectively, with two-dimensional or three-dimensional scanning motion in a synchronized manner. The scanning units 12a, 12b operate in response to a scanning signal from a scanning signal generating circuit 24 provided, for example, in a control unit 8.

The present embodiment is so arranged that a position for radiating ultrasound waves coincide with the position for radiating light beams. Therefore, the scanning motion providers not only give scanning motions to radiated positions of laser beams or light beams, but also give scanning motions to radiated positions of ultrasound waves.

Further, as will be described hereunder, an arrangement is so made that, with the timing when the ultrasound waves that have converged on a convergence point F, which is a region to be examined, are present on the convergence point F, transmitted light based on the laser beam that has reached the convergence point F (and further transmits the tissue 7) is received by the photo detector 4a serving as the light receiver 4.

Specifically, in the present embodiment, a control circuit 25 is provided for controlling, for example, the scanning signal generating circuit 24, so that the first unit 11a can move (scan) and change its position, and that, when the position has been changed, a light beam from the position can be received by the photo detector 4a serving as the light receiver 4. In this way, scattering information on different regions to be examined is ensured to be obtained to form an image.

As shown in Fig. 2, when a direction in which the ultrasound waves advance in the tissue 7 is set as a z-axis, the scanning units 12a, 12b provide two-dimensional scanning motion to the units 11a, 11b, respectively, in x- and y-directions, for example (indicated by references A and B in Fig. 2). It will be appreciated that the scanning units 12a, 12b may provide scanning motion in x- and z-directions or y-and z-directions instead of x- and y-directions. Alternatively, three-dimensional scanning motion in x-, y- and z-directions may be provided.

An electrical signal subjected to photoelectric conversion by the photo detector 4a serving as the light receiver 4 and provided in the second unit lib, is inputted to an oscilloscope 5a serving as the phase information extractor in the information extractor 5. The oscilloscope 5a extracts phase components of a complex refractive index from the electrical signal and outputs them to a personal computer (PC) 6a serving as (an image former of) the object information producer 6.

A laser beam from the laser device 3a in the first unit 11a is split into a transmission beam and a reflection beam by a half mirror 13a having a function of a beam splitter. The transmission laser bearn is reflected by a mirror 14a and then radiated on the tissue 7 through an aperture 15 of the ultrasound transducer 2a serving as the ultrasound generator 2.

The ultrasound transducer 2a generates pulsed ultrasound waves with the application of a pulsed ultrasound driving signal, which has been generated by the a pulse generator 21 provided in the control unit 8 and amplified by a power amplifier 22, onto an electrode, for example, provided in an ultrasound transducer body made up of a piezo-electric element and having a function of electroacoustic conversion.

In the present embodiment, the ultrasound transducer 2a constitutes a pulsed ultrasound generator which generates pulsed ultrasound waves with the aid of the pulse generator 21 and the power amplifier 22.

The ultrasound driving signals from the pulse generator 21, which have been amplified by the power amplifier 22, may have continuous waveforms instead of pulsed waveforms, so that continuous ultrasound waves may be generated from the ultrasound transducer 2a.

The same effects may be obtained as in the case where pulsed ultrasound waves are radiated, by separately inputting pulsed signals as reference signals into the oscilloscope 5a from the pulse generator 21 according to crude density of the continuous ultrasound waves, i.e. according to the wavelength of the ultrasound waves, to perform synchronous detection.

An acoustic lens 16 serving as an ultrasound convergence portion is attached to an ultrasound transmission side of the ultrasound transducer 2a.

In the present embodiment, the aperture 15 is provided along an ultrasound transmission axis (also referred to as a sound axis) Ou of the acoustic lens 16. The laser beam that has transmitted through the half mirror 13a and reflected by the mirror 14a travels through the aperture 15 along the ultrasound transmission axis Ou of the ultrasound waves enters into the tissue 7.

In the present embodiment, although the acoustic lens 16 is in contact with a surface of the tissue 7, the ultrasound waves may be transmitted to the surface of the tissue 7 through a transmission medium for the ultrasound waves.

The ultrasound waves transmitted from the ultrasound transducer 2a to the side of the tissue 7 are converged in a vicinity region R1 centering the convergence point F and corresponding to a focal point of the acoustic lens 16.

In Fig. 2 or Fig. 3, the vicinity region R1 is shown by a broken line.

The ultrasound waves localized in the vicinity region R1 of the convergence point F causes density change $\Delta p(z)$ in molecules composing the tissue 7 of living organism in the vicinity region R1 (as will be described later).

A modulated beam based on this will eventually be detected as an observation beam by the photo detector 4a.

On the other hand, the laser beam reflected by the half mirror 13a passes through an optical modulator 18 which is driven by oscillation output of an oscillator 17, and after being reflected by a reference mirror 14b arranged upstream, is made incident on a mixing (interference) half mirror 13b as a reference beam.

The optical modulator 18 arranged between the half mirror 13a and the reference mirror 14b is made up of ferroelectric crystal, such as LiNbO3, having electro-optic effect. With the application of an AC electric field at oscillatory frequency to the ferroelectric crystal by the oscillator 17, refractive index of the ferroelectric crystal changes in proportion to the applied electric field.

As a result, the laser beam that has passed through the ferroelectric crystal is optically modulated. Specifically, when an angular frequency of the oscillator 17 is $\omega'_0$ which is used for optical modulation by the optical modulator 18, the laser beam reflected by the half mirror 13a is optically modulated at the angular frequency $\omega'_0$.

The angular frequency ($\omega'_0$ is much lower than the angular frequency of the laser beam so as to facilitate signal processing.

An aperture 19a of a light shield member 19 is arranged opposed to the aperture 15, i.e. on the other side of the tissue 7, so as to align with the ultrasound transmission axis Ou. The laser beam that has been reflected by the mirror 14a and has passed through the vicinity region R1 in the tissue 7 passes through the aperture 19a and made incident on the half mirror 13b arranged upstream of the traveling direction of the laser beam. The aperture 19a allows the passage of the beam that has passed through the vicinity region R1 of the convergence point F as the observation beam, and suppresses (or remove) stray light components around the aperture 19a.

Thus, the observation beam that has been scattered at the vicinity region R1 and the reference beam that has been modulated are made incident on the half mirror 13b. The interference half mirror 13b then mixes both of the beams or allows interference therebetween.

In particular, in the half mirror 13b, on which the reference beam modulated at the frequency $\omega'_0$ and the observation beam that has passed through the vicinity region of the convergence point F in the tissue 7 and has transmitted through the tissue 7 are incident and mixed with each other, an interference beam (or beat signal beam) modulated at the angular frequency $\omega'_0$ is produced. (That is, the beams turn to a heterodyne detected beam.)

Thus produced interference beam is received by the photo detector 4a and is detected as an interference signal (beat signal), which is an electrical signal. After photoelectric conversion of the interference signal at the photo detector 4a, the resultant can be filtered by using an LPF, for example, that allows passage of the beat components of the angular frequency $\omega'_0$ to extract interference signal components. Thus, the present embodiment employs an arrangement of a Mach-Zehnder interferometer.

The interference signal detected by the photo detector 4a is then inputted to the oscilloscope 5a having a function of a phase meter that extracts phase information to detect phase modulation components and amplitude modulation components of the laser beam at the vicinity region R1.

As will be described later, the phase modulation components and the amplitude modulation components correspond to a real part and an imaginary part, respectively, of the complex refractive index, and reflect scattering characteristics and absorption characteristics (hereinafter referred to as "absorption characteristics"), respectively.

The phase modulation components and the amplitude modulation components can be detected by using the oscilloscope 5a as a phase meter, however, these components may be adapted to be detected by the PC 6a using a Fourier transformation process.

In the present embodiment, an arrangement is so made that the phase modulation components and the amplitude modulation components corresponding to the real part and the imaginary part of the complex refractive index can also be calculated by the PC 6a. In this case, the PC 6a functions not only as the object information producer 6, but also as the phase information extractor and the scattering information extractor.

In this case, the interference signal is also inputted to the PC 6a as shown by the broken line in the figure. Further, in this case, a CPU 33 is provided with a real/imaginary part calculator 33a for calculating the real part and the imaginary part from the reference signal as shown by the broken line in the figure.

In order to detect the transmission beam of the laser beam radiated to the vicinity region R1 of interest in the tissue 7 for extraction of at least the phase modulation components (or components of the real part of the complex refractive index) in the vicinity region R1, the output of the pulse generator 21 is inputted to the oscilloscope 5a through a delay circuit 23, which is capable of delay amount control, as well as to the PC 6a.

In the present embodiment, in order to obtain two-dimensional optical imaging information (encompassing at least one dimension), the scanning signal generating circuit 24 is provided for generating a scanning signal in synchronization with the output, for example, of the pulse generator 21.

The pulse generator 21, the delay circuit 23 and the scanning signal generating circuit 24 are controlled by the control circuit 25 which is made up, for example, of a CPU (central processing unit), not shown.

The output signal for the phase modulation components and the amplitude modulation components detected by the oscilloscope 5a are inputted, together with the scanning signal, to an A/D converter circuit 31 in the PC 6a for digital conversion and then memorized in a memory 32.

As shown by a broken line in Fig. 2, in case the reference signal is memorized in the memory 32 without passing through the oscilloscope 5a, the CPU 33 carries out an imaging process for calculating the real part and the imaginary part of the complex refractive index corresponding to the phase (modulation) components and the amplitude (modulation) components, respectively, of the laser beam in the vicinity region R1.

The CPU 33 stocks signal data resulting from the imaging process in the memory 32 in correlation with position information, outputs the data to a monitor 35 through a display processing circuit 34, and displays the two-dimensional optical imaging information on the phase components and the amplitude components as the object characteristics information.

The memory 32 stocks the phase components, for example, as image forming information provided by the CPU 33 constituting the image former, correlating with the position information.

The oscilloscope 5a operates as the phase meter for extracting the amplitude modulation components or the phase modulation components (corresponding to phase components in the second term in formula (10) which will be explained later) according to the synchronization signal from the pulse generator 21.

The pulse generator 21 generates a pulsed ultrasound driving signal at the timing synchronizing with this modulation signal, for example, or at a period of integral multiple of the modulation signal, for example.

The A/D converter circuit 31 in the PC 6a performs A/D conversion by a clock synchronizing with the modulation signal. (More particularly, the A/D converter circuit 31 performs A/D conversion in synchronization with the modulation signal and by a clock of a clock oscillator, not shown, which is oscillated at an angular frequency of integral multiple of the modulation signal). The A/D converted data is then memorized in the memory 32.

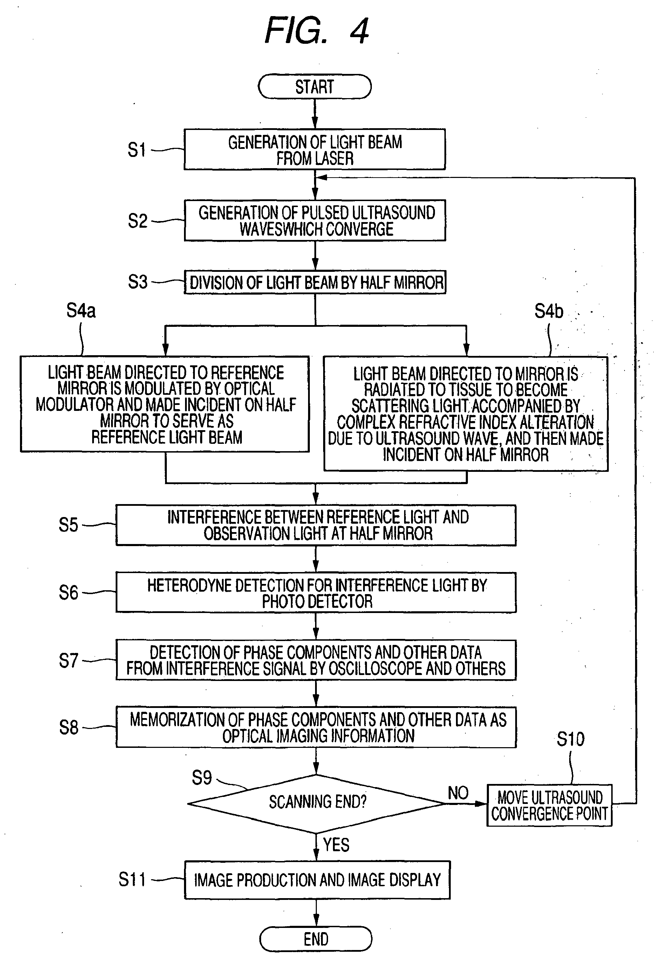

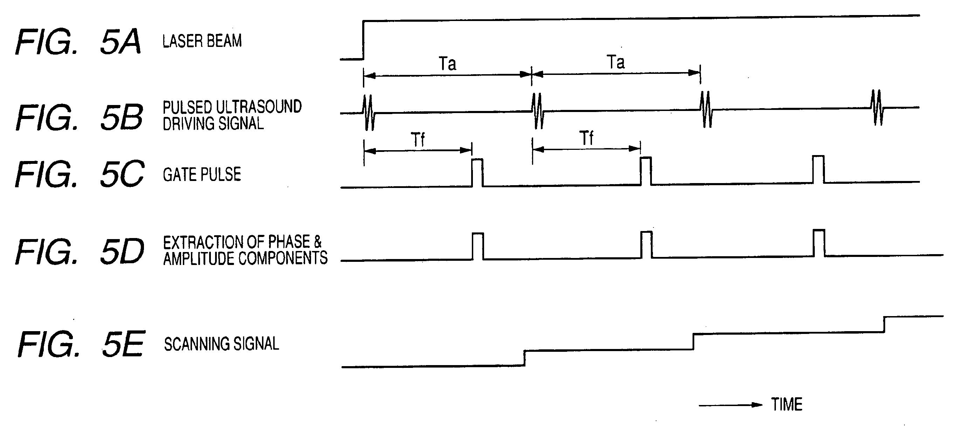

An operation of the present embodiment having the arrangement as described above is now explained with reference to Figs. 4 and 5.

When a power source of the optical imaging apparatus AP1 of the present embodiment is turned on, or when a switch for starting measurement is depressed, the laser device 3a generates a laser beam as indicated at step S1 of Fig. 4. The laser beam is successively generated as shown in Fig. 5(A).

As indicated at step S2, a pulsed ultrasound wave is generated at a constant period by the ultrasound transducer 2a. Particularly, the control circuit 25 transmits a control signal to the pulse generator 21, which then generates, as shown in Fig. 5(6), a pulsed ultrasound driving signal at a constant period T for driving the ultrasound transducer 2a.

The pulsed ultrasound waves produced by the ultrasound transducer 2a with the application of the ultrasound driving signal are propagated into the tissue 7, being converged by the acoustic lens 16. The pulsed ultrasound waves are then converged in the vicinity region R1 centering the convergence point F.

On the other hand, the laser beam generated by the laser device 3a is divided out, as indicated at step S3, by the half mirror 13a.

As indicated at step S4a, the laser beam directed to the reference mirror 14b is modulated by the optical modulator 18, then reflected by the reference mirror 14b and is made incident on the half mirror 13b.

As indicated at step S4b, the laser beam directed to the mirror 14a is reflected by the mirror 14a and then radiated to the inside of the tissue 7.

As described above, the ultrasound waves propagating through the tissue 7 are converged at the vicinity region R1 centering the convergence point F. Thus, the laser beams radiated into the tissue 7 are modulated with the alteration of the complex refractive index caused by the ultrasound waves. The modulated beams are then incident on the half mirror 13b through the aperture 19a.

Then, as indicated at step S5, the reference beam and the observation beam (object beam) interfere with each other at the half mirror 13b.

As indicated at step S6, the interference beam is received by the photo detector 4a for conversion into an electrical signal, which is then heterodyne detected being filtered by an LPF (low pass filter), for example, to serve as an Interference signal.

In the subsequent step S7, the phase components and the amplitude components (simplified as "phase components, etc." in the figure) are detected from the interference signal by the oscilloscope 5a. It will be appreciated that the phase components, etc. may be extracted using a lock-in amplifier instead of the oscilloscope 5a.

The control circuit 25 effects control so that the delay amount of the delay circuit 23 will be equivalent to the time from when the pulsed ultrasound driving signal is generated to when the pulsed ultrasound wave reaches the convergence point F (indicated by Tf in Fig. 5(C)).

The pulse delayed by the delay circuit 23 is inputted to the oscilloscope 5a, at which timing, the oscilloscope 5a generates therein a gate pulse as indicated in Fig. 5(C).

The oscilloscope 5a produces from the oscillator 17 a sweep signal synchronized with the $\omega'_0$-modulation signal within a period of the gate pulse, and indicates a value of the signal detected by the photo detector 4a as an ordinate with the sweep signal as a horizontal time base.

In other words, signal components at any phase angle in the interference signal detected by the photo detector 4a can be detected, with a period of the modulation signal at the angular frequency $\omega'_0$ as a reference. The amplitude components may also be detected.

Thus, the oscilloscope 5a detects, from the interference signal outputted from the photo detector 4a, its phase components and amplitude components in the period of the gate pulse, i.e. in a short period close to the time when the pulsed ultrasound wave reaches the convergence point F.

The phase components and the amplitude components detected by the oscilloscope 5a are inputted to the PC 6a and converted to digital signal data at the A/D converter circuit 31. As indicated at step S8, the digital signal data is memorized as optical imaging information in the memory 32, for example, which serves as information storing means.

The PC 6a also detects the amplitude components other than the phase components for storage in the memory 32 as the optical imaging information.

The optical imaging information is stored in the memory 32 being correlated to a scanning signal, i.e. being correlated to two-dimensional position information (x, y) and three-dimensional position information (x, y and z).

As described above, in the short time when the ultrasound wave is present in the vicinity region R1 of the convergence point F, the data of the phase components and the amplitude components detected from the interference signal through the oscilloscope 5a is stored in the PC 6a. This is schematically illustrated in Fig. 5(D) as phase and amplitude components extraction. In this case, a second term of formula (11), which will be explained later, is detected by the oscilloscope 5a and stored. In the oscilloscope 5a, with the $\omega'_0$-period as a time base (phase reference), an amount of shifting in a temporal axis direction and an amplitude alteration at the time when the ultrasound wave is converged are detected from the waveform of the interference signal in a state where the ultrasound wave is not converged, for example.

At the subsequent step S9, the control circuit 25 determines as to whether or not scanning has been performed to an end edge. If not yet performed to the end edge, the control circuit 25 controls the operation of the scanning signal generating circuit 24 to move the convergence point F for the ultrasound wave.

In this case, the control circuit 25 changes the amplitude of a scanning signal from the scanning signal generating circuit 24 by one step. The scanning signal generating circuit 24 generates a scanning signal of a stepped shape or of a saw-tooth shape, for example.

The scanning signal generating circuit 24 changes a value of the amplitude of a scanning signal, for example, by 1/256, for example. Fig. 5(E) illustrates an example of an x-direction scanning signal, for example.

The scanning units 12a, 12b slightly move the units 11a, 11b in the x-direction in response to the scanning signal.

Then, control returns to step S2 to repeat the same processes. After repeatedly performing the same processes from a start point to an end point of a scanning range in the x-direction, the amplitude of a y-direction scanning signal is changed by one step. Then, similarly, the units 11a, 11b are moved from the start point to the end point in the x-direction.

Image information is obtained by repeating such processes, i.e. by repeated movement of the scanning units 12a, 12b from the start points to the end points of the scanning ranges in the x- and y-directions according to the scanning signals and with the aid of the units 11a, 11b.

At step S9, when scanning is determined as having come to an end edge, control proceeds to step S11 where image formation for the optical image information is completed. The image on the optical imaging information is then displayed to terminate the operation.

In case of using the continuous ultrasound waves as well, processes can be carried out in the manner similar to the processes of Fig. 4. In this case, however, step 52 is required to be changed so as to generate continuous ultrasound waves for convergence.

A description is provided hereunder on image display of the optical imaging information by the PC 6a.

As described above, the memory 32 in the PC 6a stocks the phase components and the amplitude components corresponding to one frame of each signal data. The phase components and the amplitude components correspond to the real part and the imaginary part of the complex refractive index, respectively, and reflect the scattering characteristics and the absorption characteristics, respectively.

The CPU 33 reads out the phase and amplitude components stored in the memory 32 being correlated to position information and transfer the read-out components to a display processing circuit 34. The display processing circuit 34 converts a phase-component value or an amplitude-component value at each position into a luminance level for output to the monitor 35. Then, the optical imaging information on the scattering characteristics derived from the phase components and the absorption characteristics derived from the amplitude components in the vicinity region R1 of the convergence point F is displayed on the monitor 35 being formed into an image.

Hereinafter is described an operation for calculating scattering components (real part) and absorption components (imaginary part) from the interference signal.

In a narrow region in the tissue 7 as in the vicinity region R1 of the convergence point F of the pulsed ultrasound wave, where the ultrasound wave is localized, the molecular density change $\Delta\rho$ (z) is dynamically caused.

When the Lorentz-Lorenz relation is differentiated once with respect to a molecular density, a complex refractive index change $\Delta m$ (z) and a density change $\Delta\rho$ (z) of a complex refractive index "m" are expressed by the following formula:

$$\Delta m = (A/W)(m^2+2)^2 \Delta\rho/(6m) \quad (1),$$

wherein W represents a molecular weight of molecules structuring a medium, and A represents a total polarizability per mol.

Being effected by the change in the complex refractive index "m" as the above formula (1), the beam of light passing the vicinity region R1 of the ultrasound convergence point F interacts with a medium portion, in which the complex refractive index has been changed, and is scattered and absorbed in the medium portion.

The beam incident on the tissue 7, namely, an electric field $E_s$ (z, t) that passes through the tissue 7 is approximated by the following formula:

$$E_s(z,t) \equiv E_s = E_{s0}\exp\{i(\omega_s t - k_s z)\} \quad (2),$$

wherein $\omega_s$ is an angular frequency of the laser beam, $k_s$ is its number of waves, "z" is a depth of the tissue 7 (medium) from its surface to the ultrasound convergence point F (refer to Fig. 3 which is a partially enlarged view of Fig. 2), and ko is the number of waves of the beam in a vacuum state.

On the other hand, an optical path "z" is expressed by the following formula:

$$z = D_0 + D_1 + L_2 + m(L_3 - \Delta z) + L_4 + (m + \Delta m)$$
$$\Delta z = D_0 + D_1 + L_2 + L_4 + mL_3 + \Delta m \Delta z$$
$$(3),$$

wherein, as shown in Fig. 2, Do is a distance between the laser device 3a and the half mirror 13a, $D_1$ is a distance between the half mirror 13a and the mirror 14a (or between the half mirror 14b and the half mirror 13b), $L_1$ is a distance between the half mirror 13a and the mirror 14b, $L_2$ is a distance between the mirror 14a and the surface of the tissue 7 (at a position on which the laser beam is incident along the ultrasound transmission axis Ou), $L_3$ is a thickness of the tissue 7 on the ultrasound transmission axis Ou, and $L_4$ is a distance between a bottom surface of the tissue 7 and the half mirror 13b.

The complex refractive index m+$\Delta$m in the vicinity region R1 of the ultrasound convergence point F (hereinafter referred to as an "ultrasound convergence region") including its alteration is to be defined by the following formula:

$$m + \Delta m = (m_r + \Delta m_r) - i(m_l + \Delta m_l) = (m_r - im_l) + (\Delta m_r - i\Delta m_l) \quad (4)$$

When formula (3) is assigned to formula (2), the following formula is obtained:

$$E_s = E_{s0}\exp\{i(\omega_s t - k_s z)\}$$
$$= E_{s0}\exp[i\{(\omega_s t - k_0(D_0 + D_1 + L_2 + L_4 + mL_3 + \Delta m \Delta z)\}]$$
$$(5)$$

When the relation (4) is assigned to formula (5) for organization, the following formula is obtained:

$$E_s$$
$$= E_{s0} \exp$$
$$[i\{(\omega_s t - k_0(D_0 + D_1 + L_2 + L_4 + (m_r - im_l)L_3 + (\Delta m_r - i\Delta m_l)\Delta z)\}]$$
$$= E_{s0} \exp[i\omega_s t - ik_0(D_0 + D_1 + L_2 + L_4 + m_r L_3 + \Delta m_r \Delta z)]$$
$$\exp\{ik_0(im_l)L_3 + ik_0(i\Delta m_l)\Delta z\}$$
$$= E_{s0} \exp[-k_0(m_l L_3 + \Delta m_l \Delta z)]$$
$$\exp[i\{\omega_s t - k_0(D_0 + D_1 + L_2 + L_4 + m_r L_3 + \Delta m_r \Delta z)\}]$$
$$(6)$$

In formula (6), when the real part (i.e. $m_r + \Delta m_r$) and the imaginary part (i.e. $m_l + \Delta m_l$) of the complex refractive index $m + \Delta m$ of the medium at an ultrasound convergence region change together, a phase term and an amplitude term of the transmission beam or the reflection beam that has passed the ultrasound convergence region are locally modulated.

In the present embodiment, by extracting the local phase modulation components and amplitude modulation components such as from the transmission beam passing through the medium of the ultrasound convergence region, local scattering characteristics and absorption characteristics in the medium can be obtained.

In this case, the imaginary part ($m_l + \Delta m_l$) of the complex refractive index $m + \Delta m$ is an amount indicating the absorption of light, which is also referred to as an extinction coefficient, and has a relation with an absorption coefficient $\alpha$ expressed by the following formula:

$$\alpha = 4\pi m_l / \lambda \quad (7)$$

The absorption coefficient $\alpha$ corresponds to an inverse of a propagated distance in which incident beam intensity is reduced to $1/e$. By extracting the amplitude modulation components caused by the ultrasound waves from formulas (6) and (7), the absorption coefficient $\alpha$ of the ultrasound convergence region can be indirectly calculated.

In the present invention, the scattering characteristics and the absorption characteristics can be obtained as an image. As will be described hereinafter, enabling the acquisition of the scattering characteristics is a particularly important feature of the present invention.

Generally, structural alteration in the tissue, such as the alteration in the concentration of nuclear chromatin accompanying the canceration of a tumor and the alteration in the spatial distribution of nuclei induce alteration in the complex refractive index. On the other hand, distribution of intensity of scattering light (hereinafter referred to as "scattering intensity distribution") reflects a real part $m_r$ of a complex refractive index. (According to the Mie theory, a surrounding medium and a real part of the refractive index of a scatterer are parameters for determining a scattered waveform).

Accordingly, as can be understood from formula (6), since a refractive index change $\Delta m_r$ corresponds to the phase modulation components in the observation beam, the heterodyne detection of the phase change may enable observation of the change in the real part of the complex refractive index in the ultrasound convergence region, i.e. observation of the scattering characteristics correlated to the structural alteration of tissue accompanying the canceration of the tissue.

Formula (6) is of the case where the incident beam is made incident on the tissue 7 and passes through the ultrasound convergence region, and this transmission beam (i.e. observation beam) is directly detected. However, this is not sufficient for detecting the phase components with good S/N ratio.

Thus, in the present embodiment, the heterodyne detection is adapted to be performed, in which the reference beam that has been modulated by the optical modulator 18 and the observation beam that has passed through the ultrasound convergence region are mixed at the half mirror 13b for interference with each other to obtain an interference beam (or beat signal beam), and this interference beam is extracted, as shown in Fig. 2. The interference beam corresponding to the heterodyne detection is received by the photo detector 4a to obtain the interference signal.

In particular, a beam for electric field components expressed by formula (6) and serving as the observation beam from the ultrasound convergence region is incident on the photo detector 4a. In addition, the reference beam for electric field components expressed by the following formula (8), which is reflected by the reference mirror 14b, is also incident on the photo detector 4a.

$$E_r = E_{r0}\exp[i\{(\omega_s - \omega'_0)t - k_0(D_0 + L_1 + D_1)\}] \quad (8)$$

The photo detector 4a is capable of detecting a time quadrature of the square of the sum of formula (6) and formula (8). For clarity, by establishing the following formula:

$$E_s = E'_s\exp\{i(\omega_s t + \phi_1)\}$$

and

$$E_r = E'_r\exp\{i(\omega_s - \omega'_0)t + \phi_2\},$$

the following formula is established:

$$
\begin{aligned}
E &= E_s + E_r \\
&= \{E'_s\exp(i\phi_1) + E'_r\exp(-i\omega'_0 t + \phi_2)\}\exp(i\omega_s t)I(z,t) \\
&= |E^2| \\
&= |EE^*| \\
&= [E'_s\exp(i\phi_1) + E'_r\exp\{-i(\omega'_0 t - \phi_2)\}]\exp(i\omega_s t) \\
&\qquad \times \{E'_s\exp(-i\phi_1) + E'_r\exp\{i(\omega'_0 t - \phi_2)\}]\exp(-i\omega_s t) \\
&= E'^2_s + E'^2_r + E'_s E'_r\exp(i\phi_1) \\
&\qquad \exp\{i(\omega'_0 t - \phi_2)\} + E'_s E'_r\exp(-i\phi_1)\exp\{-i(\omega'_0 t - \phi_2)\} \\
&= E'^2_s + E'^2_r + E'_s E'_r\exp\{i(\omega'_0 t - \phi_2 + \phi_1)\} + E'_s E'_r\exp\{-i(\omega'_0 t - \phi_2 + \phi_1)\} \\
&= E'^2_s + E'^2_r + 2E'_s E'_r\cos(\omega'_0 t - \phi_2 + \phi_1)
\end{aligned}
$$

$$(9)$$

Since the following formula is established:

$$E'_s = E_{s0}\exp\{-k_0(m_i L_3 + \Delta m_i \Delta z)\}$$

and

$$E'_r = E_{r0}$$

and further, since the following formula is established:

$$\phi_1 = -k_0(D_0 + D_1 + L_2 + L_4 + m_r L_3 + \Delta m_r \Delta z)$$

and

$$\phi_2 = -k_0(D_0 + D_1 + L_1),$$

the following formula can be derived from formula (9):

$$I(z,t)$$
$$= |E_{s0}\exp\{-k_0(m_lL_3+\Delta m_l\Delta z)\}|^2 + |E_{r0}|^2 +$$
$$\quad 2E_{s0}E_{r0}\exp\{-k_0(m_lL_3+\Delta m_l\Delta z)\}$$
$$\quad \cos\{\omega'_0t+k_0(D_0+D_1+L_1)-k_0(D_0+D_1+L_2+L_4+m_rL_3+\Delta m_r\Delta z)\}$$
$$= |E_{s0}\exp\{-k_0(m_lL_3+\Delta m_l\Delta z)\}|^2 + |E_{r0}|^2 +$$
$$\quad 2E_{s0}E_{r0}\exp\{-k_0(m_lL_3+\Delta m_l\Delta z)\}$$
$$\quad \cos\{\omega'_0t-k_0(L_2+L_4-L_1+m_rL_3+\Delta m_r\Delta z)\}$$
$$= D.C. + 2E_{s0}E_{r0}\exp\{-k_0(m_lL_3+\Delta m_l\Delta z)\}$$
$$\quad \cos\{\omega'_0t-k_0(L_2+L_4-L_1+m_rL_3+\Delta m_r\Delta z)\}$$

$$(10)$$

$L_2+L_4-L_1\approx0$ may also be established by providing the delay circuit for elongating the optical path, between the mirror 14a and the tissue surface. In this case, formula (10) may be expressed as formula (11) provided below:

$$I(z,t)$$
$$= D.C. + 2E_{s0}E_{r0}\exp\{-k_0(m_lL_3+\Delta m_l\Delta z)\}\cos\{\omega'_0t-k_0(m_rL_3+\Delta m_r\Delta z)\}$$

$$(11)$$

In photocurrent components detected by the photo detector 4a, the first term of formula (11) corresponds to DC components, and the second term corresponds to AC components which sinusoidally change at the beat angular frequency $\omega'_0$. Specifically, each piece of information associated with the absorption characteristics and the scattering characteristics in a region proximate to a position "z" can be detected from the amplitude components and the phase components (more particularly, phase difference) in the AC components.

The signal of formula (11) is inputted to the oscilloscope 5a having a function of a phase meter, so that the oscilloscope 5a can detect the phase difference in the AC components. In other words, it is so arranged that the amount of scattering can also be measured. Also, the absorption characteristics can be detected from the amplitude components in the AC components.

In the present embodiment, an arrangement is so made that the output signal of the oscilloscope 5a or the interference signal inputted to the oscilloscope 5a is inputted to the PC 6a, and that, in the PC 6a as well, the phase difference of formula (11) is calculated so that the scattering characteristics can be calculated.

Hereinafter is described how the PC 6a calculates the phase difference, for example, from the output signal of the photo detector 4a (without using the oscilloscope 5a).

The data of formula (11) is A/D converted and memorized in the memory 32 in the PC 6a. Then, the CPU 33 in the PC 6a processes the data memorized in the memory 32 to compute the phase difference (real part of the complex refractive index) and the amplitude (imaginary part of the complex refractive index) of the AC components of formula (11).

In order to compute a value of phase difference, which greatly contributes to the scattering characteristics, the CPU 33 carries out Fourier transformation process for the data memorized in the memory 32 so as to achieve detection.

For detecting the phase difference, Fourier transformation F of I(z, t) with respect to a time variable "t" establishes the following formula (in this case, $\omega'_0=2\pi f_0$);

$$F\{I(z,t)\}$$
$$=a\delta(f)+(b/2)exp(ik_0(m_rL_3+\Delta m_r\Delta z))\delta(f-f_0)+$$
$$(b/2)exp(-ik_0(m_rL_3+\Delta m_r\Delta z))\delta(f+f_0)$$

$$(12)$$

wherein,

$$a=D.C.$$

and

$$b=2E_{s0}E_{r0}exp\{-k_0(m_iL_3+\Delta m_i\Delta z)\}.$$

Further, (f) indicates a delta function which is "1" only when f=0.

It is possible to retrieve a complex amplitude of a frequency spectrum $F_0$ from the second term of formula (12), and obtain a ratio between a real part and an imaginary part of the complex amplitude, so that a term $\{k_0(m_rL_3+\Delta m_r\Delta z)\}$ of the phase difference can be determined from the following formula (13). In particular, the $\{k_0(m_rL_3+\Delta m_r\Delta z)\}$ is:

$$\{k_0(m_rL_3+\Delta m_r\Delta z)\}$$
$$=tan^{-1}[Im\{(b/2)exp(ik_0(m_rL_3+\Delta m_r\Delta z))\}/$$
$$Re\{(b/2)exp(ik_0(m_rL_3+\Delta m_r\Delta z))\}]$$

$$(13)$$

The CPU 33 performs FFT processing for the data memorized in the memory 32 to calculate the phase difference value of formula (13) from the result of the FFT processing.

The individual values corresponding to the respective phase differences are memorized in the memory 32 being correlated to position information. Each value for one frame is then outputted as a luminance value, for example, to the monitor 35 through the display processing circuit 34, and displayed on its display surface as an optical tomographic image (optical imaging information). Alternatively, each value corresponding to the phase difference may be allocated to a different color signal depending on the level of the value and displayed with a false color.

Thus, the present embodiment has the following advantages.

In the present embodiment, at least the real part of the complex refractive index, i.e. two-dimensional information deeply related to two- or three-dimensional light scattering, is ensured to be extracted. Therefore, it is possible to obtain optical imaging information correlated to factors that may induce alteration in the light scattering characteristics, which alteration is caused by the structural alteration in the tissue accompanying the canceration, for example, of a tumor in the tissue 7.

More specifically, alteration in the light scattering characteristics is particularly induced by the structural alteration of tissue, such as the alteration in the concentration of nuclear chromatin accompanying the canceration of a tumor and the alteration in the spatial distribution of nuclei. Therefore, enabling optical imaging, which is deeply related to the scattering characteristics, is equivalent to enabling observation of information, which is correlated to the structural alteration In tissue accompanying the canceration of the tissue. Thus, it is highly likely that the optical imaging information can be effectively utilized In diagnosing a mucous membrane in a deep part of a living organism.

According to the present embodiment, a deeper part of a living organism is expected to be observed in comparison with the optical diagnostics techniques, such as a confocal technique or an OCT. Further, according to the present embodiment, imaging information of higher spatial resolution can be obtained in comparison with the optical CT. Accordingly, there is a good chance of obtaining readily identifiable optical imaging information at an initial stage of canceration of tissue.

The present invention can be practiced using the single laser device 3a as a light source for providing radiation to an object, and using the single photo detector 4a as light receiving means. Thus, the optical imaging apparatus AP1 of a compact size can be realized for obtaining image information.

It will be appreciated that the photo detector 4a may employ a linear one-dimensional sensor or a CCD that is a two-dimensional detector, for example.

In the present embodiment, the scanning units 12a, 12b are provided for serving as scanning motion providers. Therefore, two- or three-dimensional optical imaging information can be obtained by moving the vicinity region R1 of the convergence point F, which is the region to be examined, with the scanning motion of the units 11a, 11b involving the laser device 3a and the photo detector 4a. In particular, the two- or three-dimensional optical imaging information can be readily obtained without the necessity, for example, of laboring for locating the photo detectors 4a, for example, in plural number around the tissue 7, whereby the operability can be remarkably improved.

Further, the present embodiment is arranged such that ultrasound waves and laser beams can be coaxially radiated to an object by allowing the laser beams to travel along the ultrasound transmission axis (or sound axis) Ou, so that, for example, the optical imaging apparatus AP1 can be readily disposed.

The present embodiment may be applied to the measurements of scattering and absorption characteristics of an object to be detected not only in the surgical and medical fields but also in other industrial fields.

Additionally, the extraction of the interference beam may strengthen the intensity of the interference beam (relative to the intensity of other beams), whereby the interference beam can be extracted with good S/N ratio. As a result, scattering information of good accuracy can be obtained.

**(Second Embodiment)**

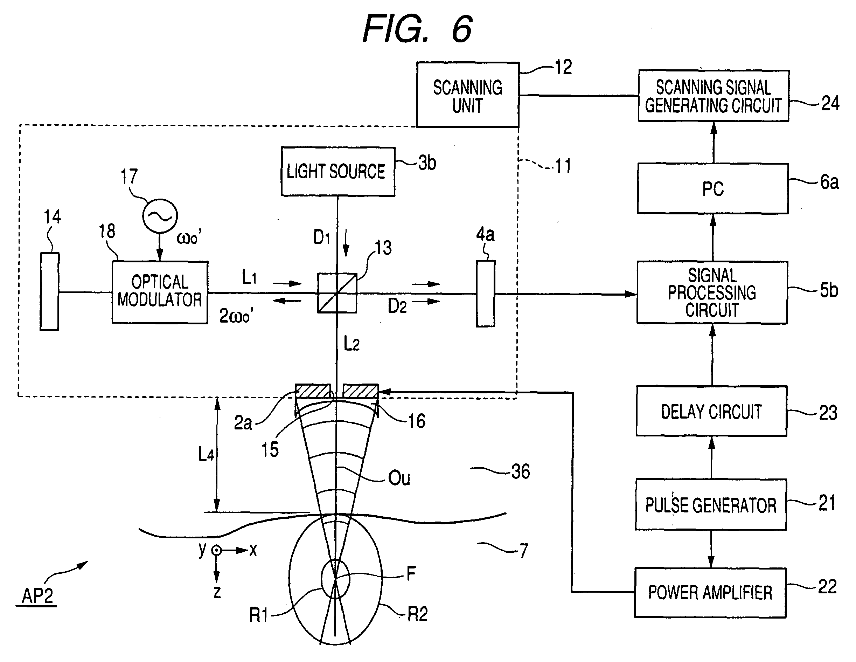

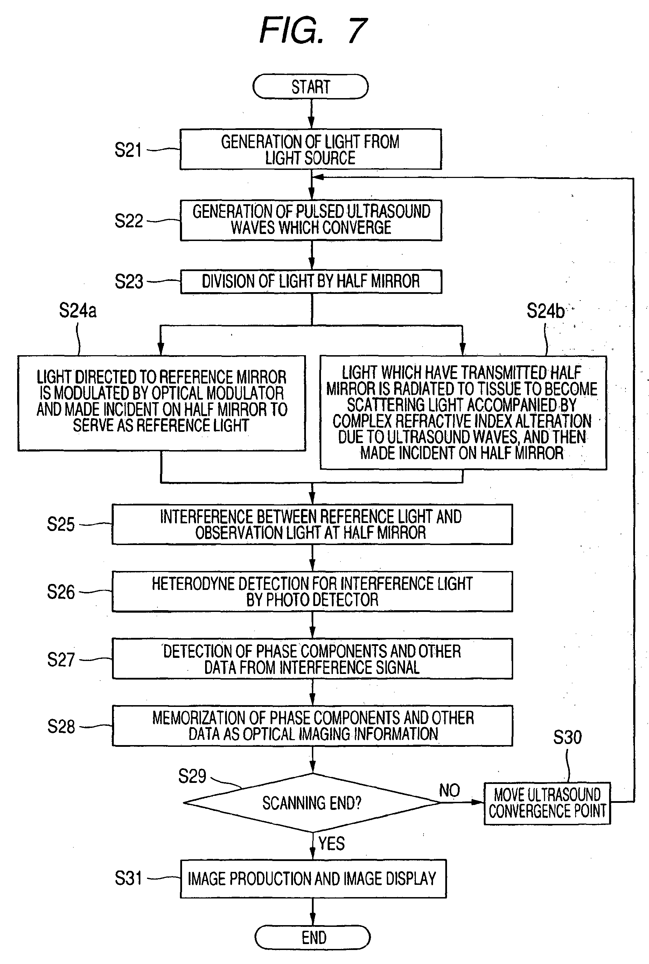

Referring to Figs. 6 and 7, a second embodiment of the present invention will now be described.

Fig. 6 illustrates an optical imaging apparatus AP2 according to the second embodiment.

The first embodiment has been arranged so as to detect the transmission beam that has transmitted through the tissue 7 and serves as the observation beam, whereas the present embodiment is so arranged that a light beam is radiated to a tissue 7 of a living organism and a beam returning to the side of radiation is detected as the observation beam. In particular, a photo detector 4a according to the present embodiment forms a reflected-light receiver. In the present embodiment, the identical or similar components or processes are given the same references as in the first embodiment for the sake of simplification or omission of explanation.

In Fig. 6, some references are different from those in Fig. 2, such as a distance "$D_1$" between a light source 3b and a half mirror 13.

The optical imaging apparatus AP2 shown in Fig. 6 is provided with a unit 11 in which the light source 3b and the photo detector 4a, for example, are arranged. The unit 11 is imparted with two- or three-dimensional scanning motion by a scanning unit 12.

The ultrasound transducer 2a attached to an end face of the unit 11 radiates ultrasound waves to the tissue 7 through water 36, for example, which serves as an ultrasound transmission medium for transmitting the ultrasound waves.

The ultrasound transducer 2a is provided, as in the first embodiment, with the aperture 15 through which a beam produced in the light source 3b is radiated to the tissue 7.

Particularly, a laser beam, for example, produced in the light source 3b is made incident on the half mirror 13, and the beam transmitted through the half mirror 13 is radiated to (incident on) the tissue 7 through the aperture 15.

This ultrasound transducer 2a also provides radiation of ultrasound waves to the tissue 7, which waves are converged by the acoustic lens 16 with a beam-traveling axis (optical axis) that passes through the aperture 15 serving as the ultrasound transmission axis Ou.

A portion of a light beam scattered in the vicinity region R1 of the convergence point F travels in a direction opposite to the direction through which the beam has been incident on the tissue 7, and is made incident on the half mirror 13. As is explained below, the beam, being reflected by the half mirror 13, turns to an interference beam that interferes with a reference beam from a reference mirror 14, and is received by the photo detector 4a.

On the other hand, the light beam from the light source 3b, which has been reflected by the half mirror 13, travels toward the reference mirror 14. In this case, the optical modulator 18 for performing optical modulation with the aid of the oscillation output power of the oscillator 17 is provided in an optical path between the half mirror 13 and the reference mirror 14, as in the first embodiment.

In the present embodiment, the reflection beam from the half mirror 13 is modulated by the optical modulator 18, and then is made incident on the reference mirror 14. The beam, after being reflected by the reference mirror 14, is again modulated by the optical modulator 18 and made incident on the half mirror 13.

In this way, the optical modulator 18 modulates the reference beam twice and returns the beam to the side of the half mirror 13. Thus, the reference beam is incident on the half mirror 13 being modulated by the optical modulator 18 at an angular frequency $2\omega'_0$.

The reference beam then interferes with the observation beam in the half mirror 13 to become an interference beam which is to be received by the photo detector 4a. In this way, the half mirror 13 of the present embodiment serves both as the half mirror 13a having a function of a beam splitter and as the half mirror 13b for performing mixing in the first embodiment.

An interference signal, which is an output signal of the photo detector 4a, is inputted to a signal processing circuit 5b serving as the information extractor 5 where signals for the phase and amplitude components in the observation beam are extracted. Alternative to the signal processing circuit 5b, the oscilloscope 5a of the first embodiment or a lock-in amplifier, for example, may be used.

An output signal of the signal processing circuit 5b is inputted to the PC 6a for conversion into digital data, and memorized in a memory in the PC 6a.

The PC 6a of the present invention also functions as the control circuit 25 in the first embodiment.

As in the first embodiment, the ultrasound transducer 2a is applied with a pulsed ultrasound driving signal that has been generated by the pulse generator 21 and has been amplified by the power amplifier 22. The pulse generated by the pulse generator 21 is delayed by the delay circuit 23 and inputted to the signal processing circuit 5b.

It will be appreciated that the ultrasound driving signals generated by the pulse generator 21 and amplified by the power amplifier 22 may have continuous waveforms instead of the pulsed waveforms, so that the ultrasound transducer 2a may generate continuous ultrasound waves.

In this regard, by allowing the pulse generator 21 to input pulsed reference signals to the signal processing circuit 5b at a time interval corresponding to a wavelength of the ultrasound wave, the same effects as in the case of radiating the pulsed ultrasound waves may be obtained.

As in the first embodiment and as will be explained hereunder, the signal processing circuit 5b carries out signal processing for extracting the phase and amplitude components in the observation beam.

In particular, the signal processing circuit 5b of the present embodiment is adapted to calculate the phase and amplitude components corresponding to the real and imaginary parts of the complex refractive index, respectively, which are closely associated with the scattering and absorption characteristics, respectively. Other parts of arrangement are the same as in the first embodiment.

A description is now provided on an operation of the present embodiment with reference to the flow diagram illustrated in Fig. 7. At an initial step S21, the light source 3b generates a light beam.

As indicated at step S22, the ultrasound transducer 2a is applied with pulsed ultrasound driving signals through the power amplifier 22, and allows the converged ultrasound waves to be generated as pulsed ultrasound waves.

As indicated at step S23, the light beam from the light source 3b is divided out at the half mirror 13.

As indicated at step S24a, after being modulated by the optical modulator 18, the beam directed to the reference mirror 14 is made incident on the half mirror 13 as a reference beam.

The beam that has transmitted the half mirror 13 Is made incident on the tissue 7, as indicated at step L24b, to turn to scattering light in the tissue 7 accompanied by refractive index alteration due to the ultrasound waves. A portion of the scattering light is made incident on the half mirror 13 to serve as the observation beam.

As indicated at step S25, the reference beam and the observation beam interfere with each other in the half mirror 13 to become an interference beam.

At step S26, the interference beam is subjected to heterodyne detection by the photo detector 4a to turn to an interference signal. In the present embodiment, the heterodyne detected interference signal becomes a signal having an angular frequency $2\omega'_0$.

At the subsequent step S27, the phase components and the amplitude components are detected from the interference signal, and memorized, as optical imaging information, in the memory in the PC 6a together with scanning position information at the subsequent step 528.

At the subsequent step S29, the CPU, for example, in the PC 6a determines whether or not an end edge of scanning has been reached. If not yet reached, the CPU drives the scanning signal generating circuit 24 through the scanning unit 12, as indicated at step S30, to move the unit 11 so that the convergence point F of the ultrasound waves is moved. Then, control returns to step S22 to repeat the processes of step S22 to step S30. In this way, scanning is performed to the end edge to proceed to steps S29 to S31.

Specifically, as indicated at step S31, when imaging of one frame has been completed, the PC 6a correlates the phase and amplitude components memorized in the memory with the scanning positions to form the optical imaging information into an image for display.

In this way, the optical imaging information reflecting the scattering and absorption characteristics can be obtained as in the first embodiment.

It will be appreciated that, in case of using continuous ultrasound waves as well, substantially the same procedure as in Fig. 7 may be executed. In this case, the process at step S22 may be changed to a process of generating the converged continuous ultrasound waves.

A description will be given hereunder as to the principle of extracting the phase components and the amplitude components of the scattering light.

An electric field Es (z', t) passing an object (the tissue 7) can be approximated to the following formula (14):

$$E_s(z',t) \equiv E_s = E_{s0}\exp\{i(\omega_s t - k_s z')\} \quad (14)$$

wherein an optical path z' can be expressed by the following formula:

$$z' = D_2 + D_1 + 2L_2 + 2n_0 L_4 + 2m(z - \Delta z/2) + (m + \Delta m)\,\Delta z$$
$$= D_2 + D_1 + 2L_2 + 2n_0 L_4 + 2(m_r - im_i)z - (m_r - im_i)\,\Delta z$$
$$+ \{(m_r + \Delta m_r) - i(m_i + \Delta m_i)\}\,\Delta z$$
$$= D_1 + D_2 + 2L_2 + 2n_0 L_4 + (2m_r z + \Delta m_r \Delta z) - i(2m_i z + \Delta m_i \Delta z)$$
$$(15),$$

wherein $n_0$ represents the refractive index of the water 36. By assigning formula (15) into formula (14) re-expressing z' with z, the following formula is reestablished:

$$E_s(z,t)$$
$$= E_{s0}\exp[i\{(\omega_s t - k_0\{(D_1 + D_2 + 2L_2 + 2n_0 L_4 + 2m_r z + \Delta m_r \Delta z) - i(2m_i z + \Delta m_i \Delta z)\}\}]$$
$$= E_{s0}\exp[i\{\omega_s t - k_0(D_1 + D_2 + 2L_2 + 2n_0 L_4 + 2m_r z + \Delta m_r \Delta z)\}]$$
$$\exp\{-k_0(2m_i z + \Delta m_i \Delta z)\}$$
$$= E_{s0}\exp\{-k_0(2m_i z + \Delta m_i \Delta z)\}$$
$$\exp[i\{\omega_s t - k_0(D_1 + D_2 + 2L_2 + 2n_0 L_4 + 2m_r z + \Delta m_r \Delta z)\}]$$
$$(16)$$

A reference beam $E_r(t)$ can be expressed by the following formula:

$$E_r(t) = E_{r0}\exp[i\{(\omega_s - 2\omega'_0)t - k_0\{D_1 + 2L_1 + D_2\}] \quad (17),$$

a formula of

$$I(z,\, t) = |E_s(z,\, t) + E_r(t)|^2$$

will now be calculated. When the following formula is established:

$$E_s(z,t) = E'_s\exp\{i(\omega_s t + \varphi_1)\}$$

and

$$E_r(t) = E_{r0}\exp[i\{(\omega_s - 2\omega'_0)t + \phi_2\}],$$

the following formula is obtained:

$$E = E_s(z,t) + E_r(t) = \{E'_{so}\exp(i\phi_1) + E_{ro}\exp(-i2\omega'_0 t + i\phi_2)\}\exp(i\omega_s t)$$

$$I(z,t) = |EE^*| = \{E_{so}\exp(i\phi_1) + E_{ro}\exp(-i2\omega'_0 t + i\phi_2)\}\exp(i\omega_s t)$$
$$\times \{E_{so}\exp(-i\phi_1) + E_{ro}\exp(i2\omega'_0 t - i\phi_2)\}\exp(-i\omega_s t)$$

$$I(z,t)$$
$$= E_{so}{}^2 + E_{ro}{}^2 + E_{so}E_{ro}\exp\{i2\omega'_0 t + i\phi_1 - i\phi_2\} + E_{so}E_{ro}\exp\{-i2\omega'_0 t - i\phi_1 + i\phi_2\}$$
$$= D.C. + E_{so}E_{ro}\exp\{-i(2\omega'_0 t - \phi_2 + \phi_1)\} + E_{so}E_{ro}\exp\{i(2\omega'_0 t - \phi_2 + \phi_1)\}$$
$$= D.C. + 2E_{so}E_{ro}\cos(2\omega'_0 t - \phi_2 + \phi_1) - \phi_2 + \phi_1$$
$$= k_0(D_1 + D_2 + 2L_1) - k_0(D_1 + D_2 + 2L_2 + n_0 L_4 + 2m_r z + \Delta m_r \Delta z)$$
$$= -k_0(2L_2 - 2L_1 + 2n_0 L_4 + 2m_r z + \Delta m_r \Delta z)$$

Accordingly, the following formula is obtained:

$$I(z,t) = |EE^*|$$
$$= D.C. + 2E_{so}E_{ro}\exp\{-k_0(2m_l z + \Delta m_l \Delta z)\}$$
$$\cos\{2\omega'_0 t - k_0(2L_2 - 2L_1 + 2n_0 L_4 + 2m_r z + \Delta m_r \Delta z)\}$$

When a length of a reference arm, and an optical path "$2L_2 + 2n_0 L_4$" of the half mirror 13 and the tissue 7 are adjusted so as to be $L_2 - L_1 + n_0 L_4 = 0$, the following formula (18) is established:

$$I(z,t)$$
$$= D.C. + 2E_{so}E_{ro}\exp\{-k_0(2m_l z + \Delta m_l \Delta z)\}$$
$$\cos\{2\omega'_0 t - k_0(2m_r z + \Delta m_r \Delta z)\}$$
$$(18)$$

This formula (18) indicates that when the real part (i.e., $m_r + \Delta m_r$) and the imaginary part (i.e., $m_l + \Delta m_l$) of the complex refractive index $m + \Delta m$ change together, the phase term and the amplitude term of the returning light beam or the scattering light beam that has passed through the ultrasound wave converging region are modulated, respectively.

In the present embodiment the phase and amplitude modulation components such as in the returning light beam from the medium in the ultrasound convergence region are extracted to obtain the local scattering and absorption characteristics, respectively, in the medium.

The imaginary part $m_l + \Delta m_l$ of the complex refractive index $m + \Delta m$, which is also referred as an extinction coefficient, indicates an amount of light absorption and has a relation with the absorption coefficient $\alpha$ as expressed by formula (7).

Formula (18) indicates the case where the incident beam is incident on the tissue 7 and the incident beam returning from the ultrasound convergence region (i.e. the observation beam) is directly detected. This, however, is not sufficient for detecting the phase components with good S/N ratio. Therefore, as shown in Fig. 6, the reference beam modulated by the optical modulator 18 is adapted to be mixed, at the half mirror 13, with the observation beam returning from the ultrasound convergence region for interference with each other to thereby obtain the interference beam, and to perform heterodyne detection of this interference beam. The interference beam corresponding to the heterodyne detection is received by the photo detector 4a and becomes the reference signal.

In photocurrent components detected by the photo detector 4a, the first term of formula (18) corresponds to DC components, and the second term corresponds to AC components which sinusoidally change at the beat angular frequency $2\omega'_0$. Specifically, each piece of information associated with the absorption characteristics and the scattering characteristics closely related to the position "z" can be detected from the amplitude components and the phase components in the AC components.

The signal of formula (18) is inputted to the oscilloscope 5a or a lock-in amplifier having a function of a phase meter to perform detection of a scattering amount and an absorption amount from the phase difference and the amplitude components, respectively, in the AC components.

Alternative to the use of the above phase meter, a phase difference, for example, may be calculated by the signal processing circuit 5b as follows.

The data of formula (18) is A/D converted and memorized in the memory in the PC 6a. The CPU 33 in the PC 6a processes the data memorized in the memory 32 to compute the phase difference (real part of the complex refractive index) of formula (18).

In order to compute a value of phase difference, which greatly contributes to the scattering characteristics, the CPU 33 carries out Fourier transformation process for the data memorized in the memory 32 so as to achieve detection.

For detecting the phase difference, Fourier transformation F of I(z, t) with respect to a time variable "t" establishes the following formula (8') (in this case, $2\omega'_0 = 2\pi f_0$):

$$F\{I(z,t)\}$$
$$= a\delta(f) + (b/2)\exp(ik_0(2m_r z + \Delta m_r \Delta z))\delta(f - f_0) +$$
$$(b/2)\exp(-ik_0(2m_r z + \Delta m_r \Delta z))\, \delta(f + f_0)$$
$$\tag{8'}$$

wherein "a" represents DC components and $b = 2E_{s0}E_{r0}\exp\{-k_0(2m_i z + \Delta m_i \Delta z)\}$.

It is possible to retrieve a complex amplitude of a frequency spectrum $F_0$ from the second term of formula (8'), and obtain a ratio between a real part and an imaginary part of the complex amplitude, so that a phase-difference term $\{k_0(2m_r z + \Delta m_r \Delta z)\}$ can be determined from the following formula (9'). In particular, the $\{k_0(2m_r z + \Delta m_r \Delta z)\}$ is:

$$\{k_0(2m_r z + \Delta m_r \Delta z)\}$$
$$= \tan^{-1}[\mathrm{Im}\{(b/2)\exp(ik_0(2m_r z + \Delta m_r \Delta z))\}/$$
$$\mathrm{Re}\{(b/2)\exp(ik_0(2m_r z + \Delta m_r \Delta z))\}]$$
$$\tag{9'}$$

According to the present embodiment, the optical imaging information on the scattering and absorption characteristics can be obtained as in the first embodiment, and the optical imaging information can also be displayed being formed into an image. Accordingly, diagnosis information which is useful for totally diagnosing a lesion site can be provided.

The optical imaging apparatus AP2 of the type of detecting reflection beams may achieve a more compact size arrangement for measuring light scattering or the like than the type of detecting transmission beams. Also, the latter type provides a merit of using only one scanning unit 12 that serves as a scanning motion provider.

Similar to the first embodiment, extraction of the interference beam may provide an advantage, for example, that scattering information of good S/N ratio can be obtained.

It will be appreciated that the photo detector 4a may be a linear one-dimensional detector or a two-dimensional detector, such as a CCD.

**(Modifications of the second embodiment)**

Hereinafter is described a modification of the second embodiment.

< First modification >

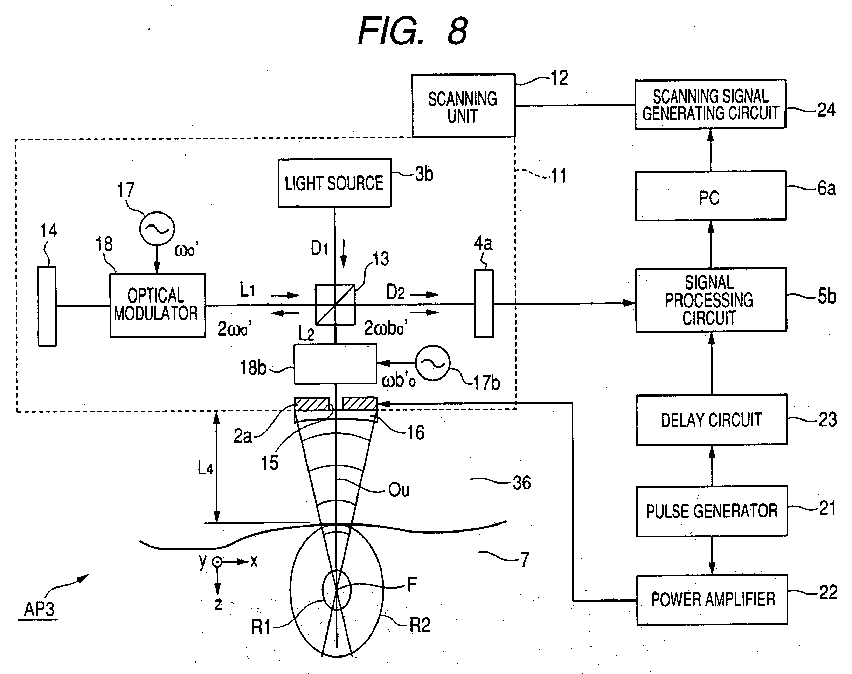

Fig. 8 illustrates an optical imaging apparatus AP3 according to a first modification. The optical imaging apparatus AP3 is provided with a second optical modulator 18b for performing optical modulation with the aid of the oscillation output power of an oscillator 17b, which is arranged in the optical path between the half mirror 13 and the ultrasound transducer 2a in Fig. 6

This oscillator 17b employs an angular frequency $\omega b'_0$ which is different from the angular frequency $\omega'_0$ of the oscillator 17. Thus, a reference beam modulated at $2\omega'_0$ and an observation beam modulated at $2\omega b'_0$ are allowed to interfere with each other in the half mirror 13 to produce an interference beam.

Then, an interference signal of $2(\omega'_0+\omega b'_0)$, for example, is extracted by heterodyne detection.

According to the present modification, an observation beam of better S/N ratio can be extracted (detected) by arranging an optical modulating portion in an optical path as well on the side of the observation beam.

In particular, in case the optical modulator 18b is not arranged on the side of the observation beam, the observation beam is not modulated when extracting the interference beam of $2\omega'_0$ caused by the interference in the half mirror 13. Therefore, as a matter of fact, components resulting from an interference with the beam from the light source 3b are unavoidably mingled into the interference beam, other than the observation beam returning from the side of the tissue 7.

Contrarily, according to the present modification, an interference signal of $2(\omega'_0+\omega b'_0)$ is extracted, so as to extract components in the interference beam alone, which have been obtained by the interference between the reference beam and the observation beam actually returning from the side of the tissue 7.

It will be appreciated that the continuous ultrasound waves may be used as in the embodiment shown in Fig. 6.

<Second Modification>

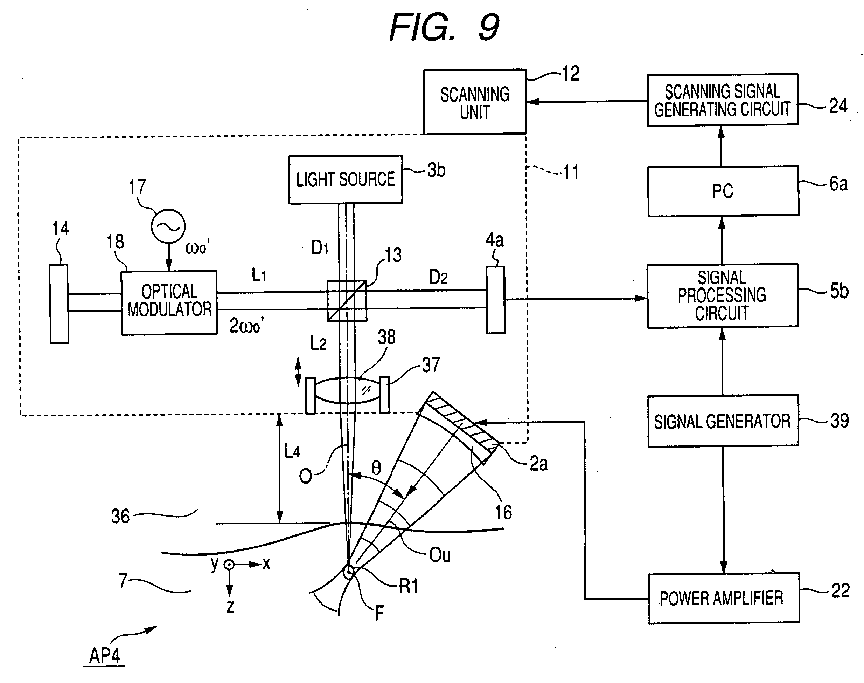

Fig. 9 illustrates an optical imaging apparatus AP4 according to a second modification. The second embodiment described above has provided an arrangement in which a beam of light is radiated to the tissue 7 along the ultrasound transmission axis Ou of the ultrasound waves radiated to the tissue 7. In the present modification, the ultrasound waves and the light beams are adapted to be radiated to the tissue 7 from different directions.

In the present modification, a radiation beam convergence portion is provided in the arrangement, so that the beams to be radiated to the tissue 7 can be converged to the portion. As shown in Fig. 9, beams produced by the light source 3b and passed through the half mirror 13 converge through a collimator lens (or a condenser lens) 38 attached to a lens frame 37 and radiated to the tissue 7.

The ultrasound transducer 2a is attached to the end face, for example, of the unit 11, so that a direction which makes an angle (>0), for example, with an optical axis O of the collimator lens 38, will be the ultrasound transmission axis Ou.

The ultrasound transducer 2a is provided with the acoustic lens 16 serving as the ultrasound convergence portion, however, no aperture 15 is provided therein.

As shown in Fig. 9, the light beams scattered in the direction of the optical axis O in the vicinity of the vicinity region R1 of the convergence point F return to the side of the collimator lens 38 being barely influenced, after the scattering, by the converged ultrasound waves. Thus, in the present modification, the arrangement is so made that the light beams scattered at the vicinity region R1 of the convergence point F can be detected being barely influenced by the ultrasound waves in a region other than the vicinity region R1, not only in the case of using pulsed ultrasound waves but also the case of using continuous ultrasound wave.

Accordingly, in the present modification, no pulse generator 21 is used, but a signal generator 39 is used for generating, for example, ultrasound driving signals for the continuous ultrasound waves. As a matter of course, the pulsed ultrasound waves may be used.

The ultrasound driving signal generated by the signal generator 39 is applied to the ultrasound transducer 2a after being amplified by the power amplifier 22.

The lens frame 37 attached with the collimator lens 38 may be fitted to a cylindrical body, not shown, which is provided on the side of the unit 11, so that the light beams can be controlled for focus on the position of the convergence point F of the acoustic lens 16 for converging the ultrasound waves, by allowing the lens frame 37 to move in the direction of the optical axis O. Particularly, the collimator lens 38 together with the lens frame 37 forms a control portion for an radiation position to which the radiation beams are radiated.

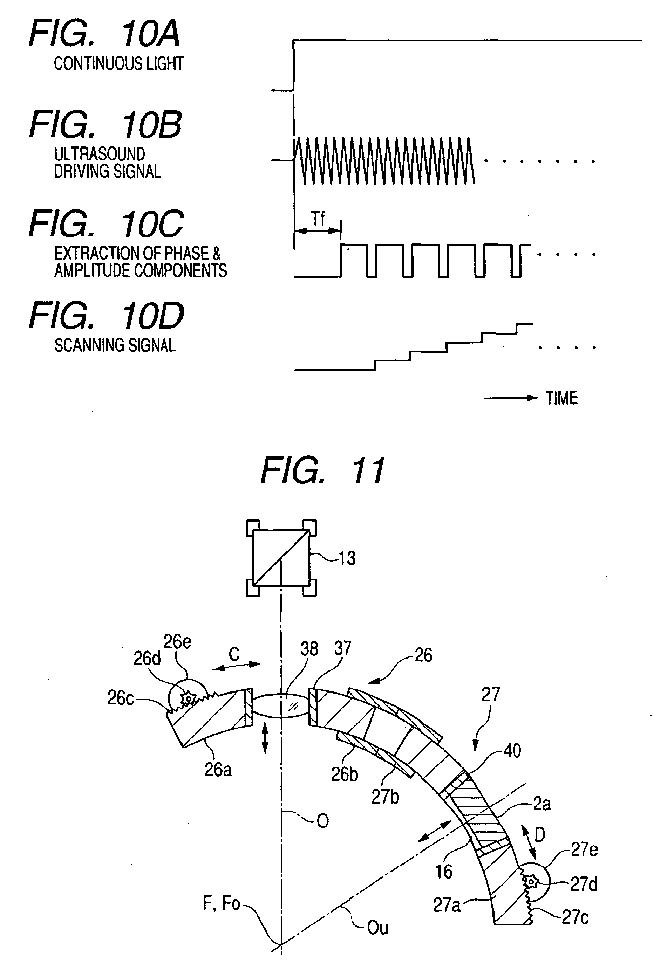

The acoustic lens 16 may also have an arrangement for enabling control of a focal position. Specifically, a focus controller for ultrasound waves may be formed, so that the ultrasound waves may focus on the position where the beams of light have focused through the collimator lens 38. In Fig. 11, which will be described later, an arrangement with the focus controller is shown.

It will be appreciated that in the modified embodiments shown in Figs. 8 and 9 as well, a linear one-dimensional detector or a two-dimensional detector, such as a CCD, may be used replacing the photo detector 4a.

Fig. 10 show timing charts for the present modification. As shown in Fig. 10(A), the light source 3b produces continuous beams of light. As shown in Fig. 10(B), the signal generator 39 generates an ultrasound driving signal of continuous waves.

In response to the signal from the signal generator 39, the signal processing circuit 5b extracts the phase and amplitude components from the interference signal in synchronization with a modulation signal with the gate being open, for example, in a short period up to the timing when the ultrasound waves reach the convergence point F, as shown in Fig. 10(C).

The control for the timing of retrieving the interference signal is carried out only at the beginning until when the ultrasound waves reach the convergence point F. However, as shown in Fig. 10(D), scanning signals are generated with a short period (by the control of the PC 6a, for example).

The phase and amplitude components extracted by the signal processing circuit 5b are memorized in the memory, for example, in the PC 6a, being correlated to the scanning signals.

In the present modification operating in this way, the angle θ (made between the optical axis O and the ultrasound transmission axis Ou is set at a value equal to or more than an appropriate value. Therefore, the beams of light which have scattered, with the complex refractive index being changed by the converged ultrasound waves in the vicinity of the convergence point F, can be detected thereafter as the observation beams being barely influenced by the ultrasound waves.

Thus, scanning can be performed substantially successively while continuously radiating the ultrasound waves. In addition one-frame optical imaging information can be obtained in a short period of time.

Matters other than those mentioned in the above exert similar effects as in the second embodiment. The first modification may be applied to the present modification to detect the interference signal with better S/N ratio.

<Third modification>

Fig. 11 illustrates a lens holder 26 serving as an radiation optical axis holder, and a transducer holder 27 serving as an ultrasound transmission axis holder, including the neighboring area.

The example shown in Fig. 11 is a case where the modification has been applied to the arrangement shown in Fig. 9.

The lens holder 26 includes a movable holder 26a which effects holding so as to enable slidable movement by a through hole to which the lens frame 37 attached with the collimator lens 38 is fitted, and a fixed-side holder 26b which effects holding so as to enable rotatably slidable movement with one end side of the movable holder 26a being fitted thereto.

Further, a rack portion 26c is provided at the other end side of the movable holder 26a. A gear 26d provided at an end of a knob 26e engages with the rack portion 26c.A user may rotate the knob 26e for operation so as to move the movable holder 26a as shown by a reference C in Fig. 11. Thus, the optical axis O of the collimator lens 38 may be moved to thereby control the optical axis O.

In the similar fashion, the transducer holder 27 includes a movable holder 27a which effects holding so as to enable slidable movement by a through hole to which a movable frame 40 attached with the ultrasound transducer 2a is fitted, and a fixed-side holder 27b which effects holding so as to enable rotatably slidable movement with one end side of the movable holder 27a being fitted thereto.

Further, a rack portion 27c is provided at the other end side of the movable holder 27a. A gear 27d provided at an end of a knob 27e engages with the rack portion 27c.

A user may rotate the knob 27e for operation so as to move the movable holder 27a as shown by a reference D in Fig. 11. Thus, the optical axis 0 of the ultrasound transducer 2a may be moved to thereby control the direction of the optical axis O.

In Fig. 11, by controlling the lens frame 37 in the direction of the optical axis O, a focal position Fo may be controlled. Similarly, by controlling the movable frame 40, the ultrasound convergence point F may be controlled.

Further, there is provided a radiation beam control portion which effects control so that, for example, the convergence point F coincides with the focal position Fo. Also, an ultrasound wave focus controller is provided which effects control so that the focal position Fo coincides with the convergence point F

In the present modification, the movable holders 26a and 27a are held in a rotatably movable manner by the fixed-side holders 26b and 27b, respectively, In a single plane (a plane including the optical axis O and the ultrasound transmission axis Ou), whereby the beams of light and the ultrasound waves are readily focused on a region to be examined even when the directions of the optical axis and the ultrasound transmission axis are different.

Fig. 11 illustrates focus control and the direction control mechanism in the case where the optical axis O and the ultrasound transmission axis Ou are set in different directions. However, in case the directions of the optical axis O and the ultrasound transmission axis Ou are allowed to coincide with each other, the lens holder 26 and the transducer holder 27 may be arranged coaxially or in stacked manner

<Fourth modification>

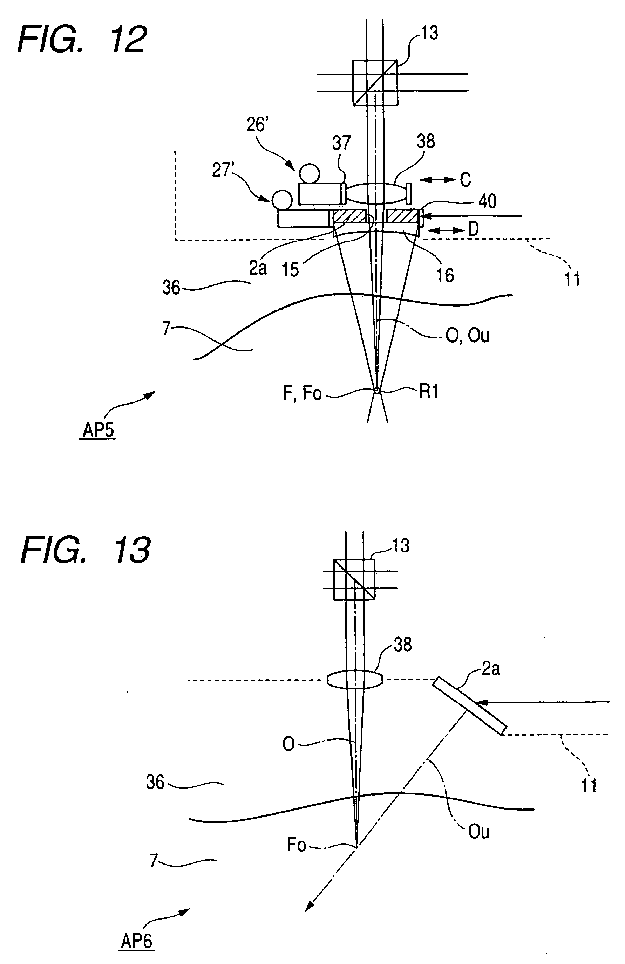

Fig. 12 illustrates an optical imaging apparatus AP5 according to a fourth modification, in which the lens holder 26 and the transducer holder 27 are coaxially arranged. Fig. 12 shows a lens holder 26' and a transducer holder 27', including the neighboring area.

In the present modification, the lens holder 26' and the transducer holder 27' analogous to the lens holder 26 and the transducer holder 27, respectively, explained referring to Fig. 11 are coaxially provided being juxtaposed with each other. The arrangement is so made that control can be effected so that the optical axis O of the collimator lens 38 and the ultrasound transmission axis Ou will coincide with each other.

The lens holder 26' and the transducer holder 27' are different from the lens holder 26 and the transducer holder 27 in Fig. 11 in that the former are adapted to be movable in a direction perpendicular to the optical axis O and the ultrasound transmission axis Ou, respectively.

An radiation optical axis controller is formed so that the optical axis O can be controlled to coincide with the ultrasound transmission axis Ou by controlling the side of the lens holder 26', for example.

Further, a transmission axis controller is formed so that the ultrasound transmission axis Ou can be controlled to coincide with the optical axis O by controlling the side of the transducer holder 27', for example.

According to the present modification, the convergence point F and the focal position Fo can be controlled to coincide with each other even when there is a variation in the characteristics of the lens holder 26' and the transducer holder 27'. Thus, the optical imaging information can be obtained at high S/N ratio and high resolution.

<Fifth modification>

Fig. 13 illustrates an optical imaging apparatus AP6 according to a fifth modification. The optical imaging apparatus AP6 has an arrangement, for example, of the system shown in Fig. 9 with the acoustic lens 16 being omitted from the ultrasound transducer 2a. As a result, the ultrasound transducer is to radiate non-converged ultrasound waves to the tissue 7.

In the present modification, a simplified arrangement is provided so that the optical imaging information can be obtained at low cost. It will be appreciated that the condenser lens 38 may also be omitted, which may further reduce the cost.

In the modified embodiments shown in Figs. 12 and 13 as well, the ultrasound waves may be pulsed or continuous, and a linear one-dimensional detector or a two-dimensional detector, such as a CCD, may be used as the photo detector, as described above.

(Third Embodiment)

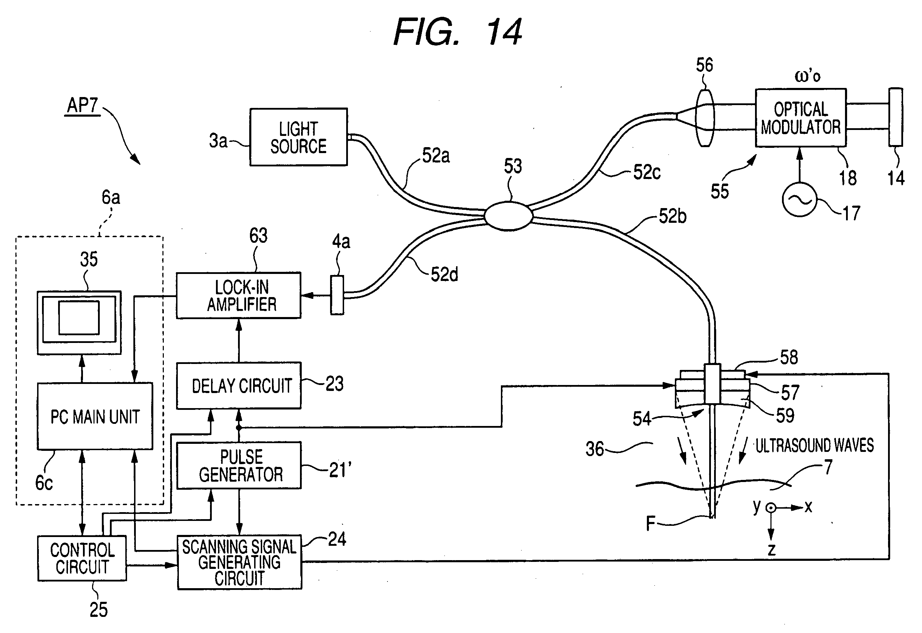

Referring to Figs. 14 - 16, a third embodiment of the present invention will now be described.

Fig. 14 illustrates an optical imaging apparatus AP7 according to the third embodiment of the present invention. In the present embodiment, an optical fiber is used to achieve more reduction in the size, in particular, reduction in the size of a two-dimensional scanning portion. In the present embodiment, the identical or similar components or processes are given the same references as in the first and second embodiments for the sake of simplification or omission of explanation.

The optical imaging apparatus AP7 is provided with the light source (or laser device) 3a for emitting a laser beam, for example, to be radiated to the tissue 7.

A light beam emitted from the light source 3a is incident on an end face of an optical fiber 52a for directing the light beam and divided into two beams in an optical coupler 53 provided midway through the optical fiber 52a. One beam is directed to a side of a beam radiator/receiver 54 for radiating and receiving beams through an optical fiber 52b, and the other beam is directed to a side of a reference-beam producer 55 through an optical fiber 52c.

The beam which is radiated to the tissue 7 through the water transmitting ultrasound waves and is returned from the tissue 7, enters the optical fiber 52b as an observation beam. This beam turns to an interference beam in the optical coupler 53, being interfered with the beam on the side of the reference beam producer 55. The interference beam is then received by the photo detector 4a through an optical fiber 52d which is arranged at an end face of the optical fiber 52d.

It will be appreciated that an acoustic lens 59 may be brought into contact with the tissue 7 without using the water 36.

In the reference beam producer 55, beams emitted from an end face of the optical fiber 52c are turned to parallel pencil beams by a collimator lens 56 so as to be incident on the reference mirror 14. The beams reflected by the reference mirror 14 are again allowed to enter the end face of the optical fiber 52c by the collimator lens 56.

In this case, the optical modulator 18, which is driven with the aid of the oscillation output power of the oscillator 17, is arranged between the collimator lens 56 and the reference mirror 14 as in the first embodiment. The reference beam is modulated by this optical modulator 18.

In the beam radiator/receiver 54, an ultrasound transducer 57 and a scanning device 58 are provided proximate to an end face of the optical fiber 52b for performing radiation (or emission) and reception of beams.

Being applied with pulsed ultrasound driving signals by a pulse generator 21', the ultrasound transducer 57 generates pulsed ultrasound waves, which are converged by an acoustic lens 59 and radiated to the side of the tissue 7. The pulse generator 21' also functions as the pulse generator 21 and the power amplifier 22 of Fig. 2.

The scanning device 58, when applied with scanning signals from the scanning signal generating circuit 24 in synchronization with the pulsed ultrasound driving signals, provides a two-dimensional scanning motion to the beam radiator/receiver 54. For example, the scanning motion is a given depthwise of the tissue 7, i.e. in a z-direction, and an x-direction, for example, which is perpendicular to the z-direction.

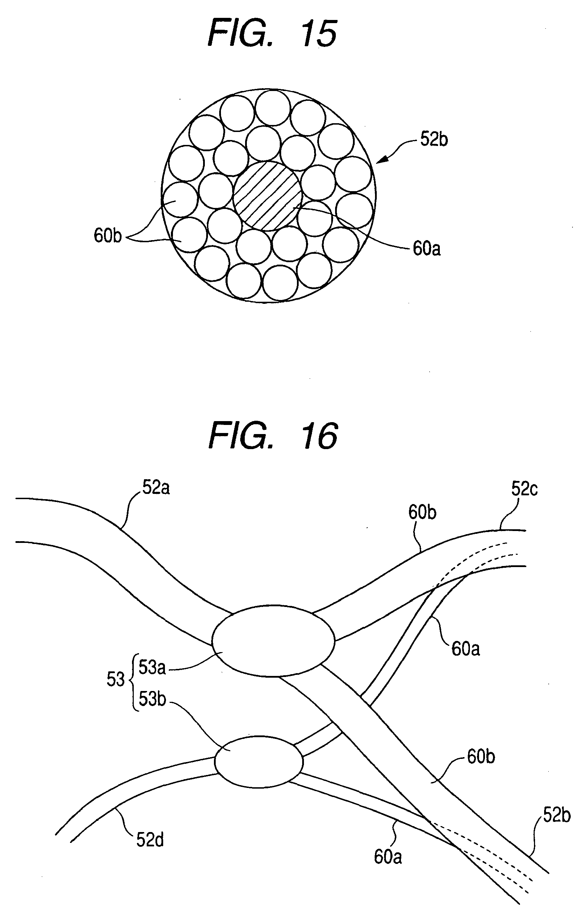

The end face of the optical fiber 52b is shown in Fig. 15, being enlarged. In the present embodiment, the optical fiber 52b is made up of a first fiber portion 61a arranged at a center portion and consists of a single or a plurality of optical fibers, and a second optical fiber portion 60b arranged around the first optical fiber portion 61a and consists of a plurality of fibers, i.e. a fiber bundle.

For example, as can be seen from an enlarged view of the optical coupler 53 shown in Fig. 16, it is so arranged that a laser beam generated by the laser device 3a is allowed to enter the optical fiber 52a and to be divided into two, for example, in a first coupler portion 53a constituting the optical coupler 53 so as to be directed to the second optical fiber portion 60b in the optical fiber 52b and the second optical fiber portion 60b in the optical fiber 52c.

Then, from an end face of the second optical fiber 60b, the directed laser beam is radiated to the tissue 7. The beam returned from the tissue 7 is adapted to be received by the first optical fiber portion 60a at the center. The beam received by the first optical fiber portion 60a is mixed, in a second coupler portion 53b, with the beam directed by the first optical fiber portion 60a from the reference beam producer 55 to produce an interference beam. The interference beam is then directed through the optical fiber 52d for reception by the photo detector 4a.

The signal detected by the photo detector 4a is inputted to a signal input terminal of a lock-in amplifier 63. A modulation signal at 2('0 from the oscillator 17 is applied to a reference signal input terminal in the lock-in amplifier 63. The lock-in amplifier 63 extracts, at high S/N ratio, only those signal components that are phase synchronized with the modulation signals (for the signals inputted from the signal input terminal).

The lock-in amplifier 63 performs signal extraction in synchronization with the timing delayed from the pulse generator 21' by the delay circuit 23.

The signal detected by the lock-in amplifier 63 is inputted to an A/D converter circuit in a PC main unit 6c of the PC 6a. The PC main unit 6c is substantially similar to the PC 6a of Fig. 2 with the omission of the monitor 35.

A scanning signal from the scanning signal generating circuit 24 is inputted to the A/D converter circuit in the PC main unit 6c. By capturing the scanning signal, scanning position information can be calculated.

The control circuit 25 controls the operations of the pulse generator 21', the scanning signal generating circuit 24 and the delay circuit 23. The control circuit 25 can perform transmission/reception of control signals to/from the CPU 33 in the PC main unit 6c. Thus, the pulse generator 21', for example, can be controlled by the control circuit 25, while the PC 6a can also control the pulse generator 21', for example, through the control circuit 25.

According to the present embodiment having the arrangement as described above, scanning motion at an end area of the optical fiber 52b may acquire the imaging information corresponding to the real and imaginary parts of the complex refractive index as in the first embodiment. Further, the scanning device 58 can be formed into a small size and thus may require only a small driving force.

**(Modifications of the third embodiment)**

<First modification>

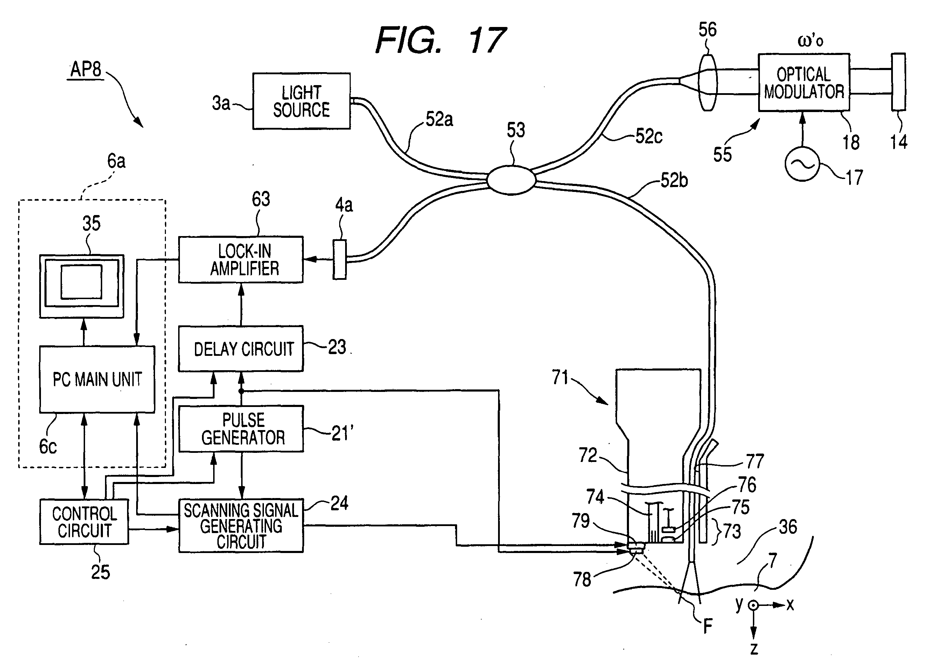

Fig. 17 illustrates an arrangement of an endoscope system AP8 having an optical imaging apparatus according to a first modification of the third embodiment. The present modification has an arrangement in which the beam radiator/receiver 54 is provided in an endoscope 71.

In the endoscope 71, a rigid end portion 73 provided at an end of an insert part 72 is furnished with an radiation window for radiating an radiation beam and an observation window for performing observation (imaging). An end side of a light guide 74 is attached to the radiation window and the radiation beam is emitted from an end face. The radiation beam Is made incident on an end face close at hand, not shown, of the light guide 74, from an endoscope light source, not shown.

An objective lens 75 is attached to the observation window, and at an imaging position of the objective lens 75, a CCD 76, for example, is arranged as an Imaging device. The CCD 76 is connected to a signal processor, such as a video processor, not shown. The signal processor performs processing for image signals picked up by the CCD 76 to produce video signals, and outputs the video signals to a monitor, not shown.

The insert part 72 is provided, in the longitudinal direction thereof, with a channel 77 through which manipulation tools can be inserted. The optical fiber 52b is inserted into the channel 77.

In the endoscope 71 according to the present modification, a scanning device 79 is attached to the end face of the end portion 73, for example, and an ultrasound transducer 78 is attached to a driving surface of the scanning device 79.

The ultrasound transducer 78 is adapted to perform two- or three-dimensional scanning motion with the application of the scanning signals from the scanning signal generating circuit 24 to the scanning device 79.

The ultrasound transducer 78 according to the present modification is made up, for example, of an electronic scanning ultrasound transducer. This type of ultrasound transducer is structured by a plurality of transducer elements disposed along the x-direction, for example, of Fig. 17, and is adapted to radiate ultrasound waves so as to make convergence by being driven with the control of delay time through delay elements or the like, not shown, and to achieve convergence at the convergence point F.

As shown in Fig. 17, the ultrasound convergence point F is set in an area in a radiation region made by the beam emitted from the end face of the optical fiber 52b. In this state, the scanning device 79 is driven by the scanning signals to impart the convergence point F with two-dimensional scanning motion.

Other parts of arrangement than explained above are analogous to the arrangement shown in Fig. 14 and description of these parts is omitted. According to the present modification, endoscopy can be carried out for the tissue 7 in a body cavity by using the endoscope 71. Then, for a site, such as a lesion site, requiring more detailed observation or diagnosis, the optical fiber 52b is inserted, as shown in Fig. 17, through the channel 77 to have the end face thereof be opposed to the region to be observed.

Then, the pulsed ultrasound driving signal is allowed to be generated by the pulse generator 21', while also allowing the scanning signal to be generated, so that the two-dimensional imaging information on the region to be observed and its neighboring area can be obtained and displayed on the display surface of the monitor 35.

In the present modification, the endoscope 71 is provided with the beam radiator for radiating beams, the ultrasound radiation means for radiating ultrasound waves so as to make convergence, and the scanning means for imparting the convergence region with two- or three-dimensional scanning motion. Therefore, the optical imaging information on the tissue 7 of a desired region in a body cavity can be obtained.

As described above, according to the present modification, it is possible to perform optical endoscopy of the tissue 7 such as of an affected area in a body cavity, obtain in doing so the optical imaging information on a region requiring more detailed diagnosis, such as a lesion site, and display the information on the monitor 35. In this way, useful information can be obtained for more accurately diagnosing the lesion site than in the case of only utilizing endoscopy.

As will be described hereunder as another modification of the third embodiment, the beam radiator/receiver 54 having a function of converging and radiating ultrasound waves and a function of scanning means in the third embodiment, may be provided at an end aperture portion of the channel 77, for example.

<Second modification>

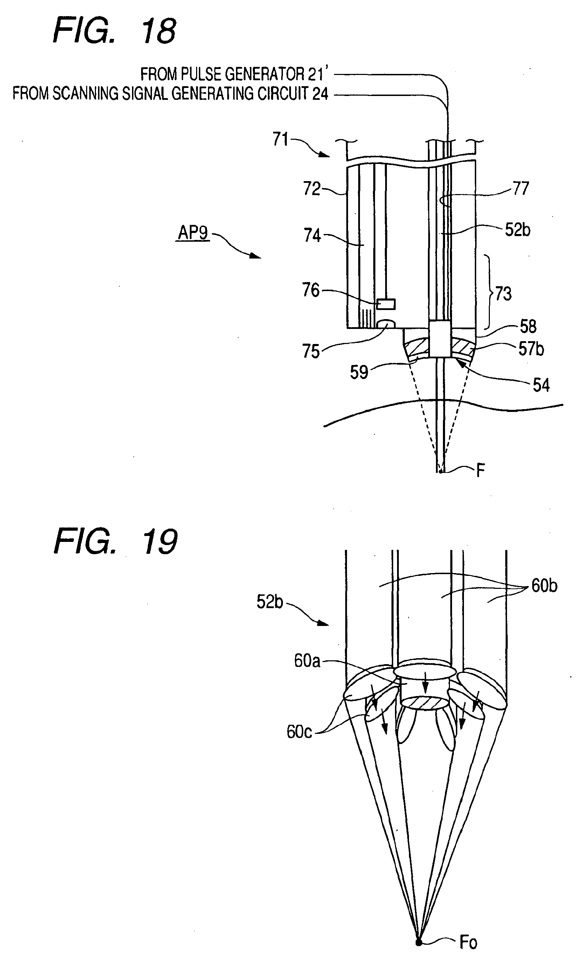

Fig. 18 illustrates an arrangement of an end portion of the endoscope 71 including its neighboring area in an endoscope system AP9 according to a second modification. In the arrangement of the present modification, the beam radiator/receiver 54 of Fig. 14 is detachably attached to an end aperture of the channel 77.

<Third modification>

Fig. 19 illustrates an arrangement of an end portion of the optical fiber 52b according to a third modification. As have been shown in Fig. 15, in the optical fiber 52b, the first optical fiber portion 60a is arranged at the center, and the second optical fiber portion 60b consisting of a plurality of fibers is arranged around the first optical fiber portion 60a. In the optical fiber 52b shown in Fig. 19, an end face of each of the fibers in the second optical fiber portion 60b is processed so that a concave surface, such as a paraboloidal surface, is formed, which is rotationally symmetrical with respect to an axis of the first optical fiber portion 60a positioned at the center. Further, the end face of each of the fibers in the second optical fiber portion 60b is furnished with collimator lenses (or condenser lenses) 60c for collecting light.

A beam of light emitted from the end face of each of the fibers in the second optical fiber portion 60b is ensured to focus on a focal position $F_0$. In this way, the optical imaging information can be obtained at high S/N ratio.

In the modified embodiments described above, the optical modulator and its driver may be provided between the coupler and the beam radiator (i.e. in the vicinity of the tissue a living organism) to improve the S/N ratio. Further, as described above, either of the pulsed or continuous ultrasound waves may be used, or a linear one-dimensional detector or a two-dimensional detector, such as a CCD, may be used as the photo detector.

**(Fourth Embodiment)**

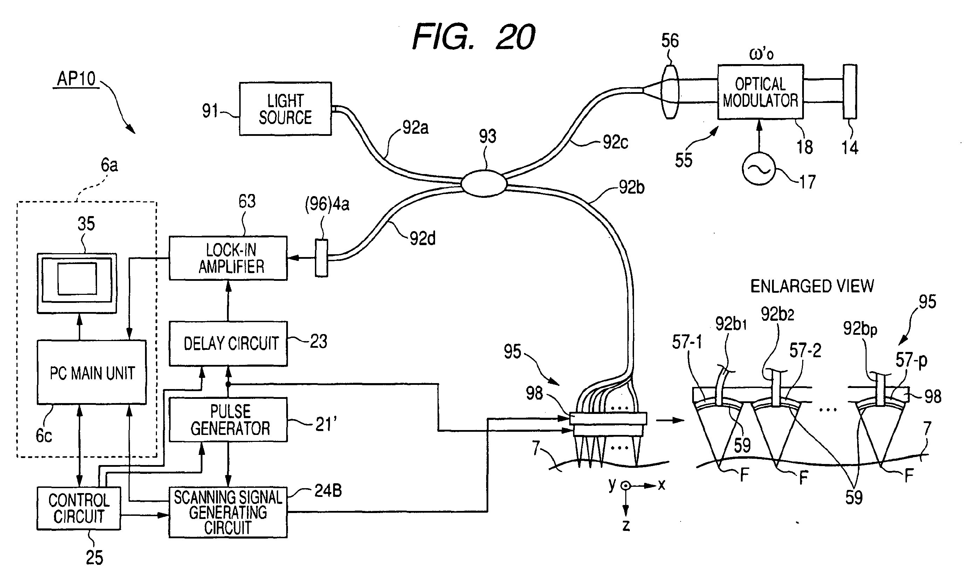

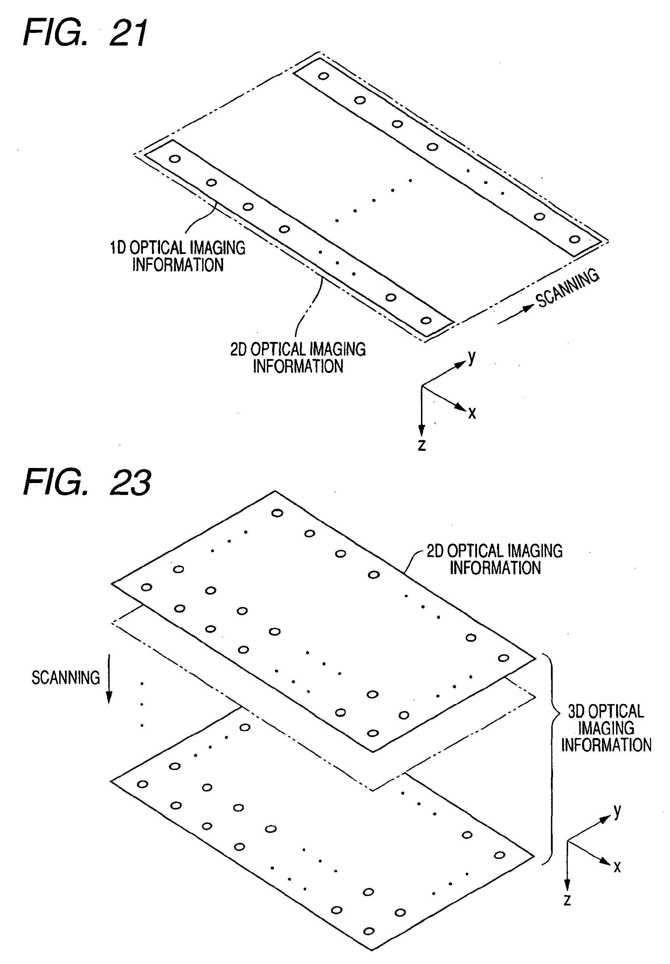

Referring to Figs. 20 and 21, a fourth embodiment of the present invention will now be described. In the present embodiment, the identical or similar components or processes are given the same references as in the first, second and third embodiments for the sake of simplification or omission of explanation.

Fig. 20 illustrates an arrangement of an optical imaging apparatus AP10 according to a fourth embodiment of the present invention. The arrangement of the present embodiment includes an ultrasound emitter/radiator/detector array 95 in which a plurality of ultrasound emitting/radiating/detecting portions each formed by integrating an ultrasound emitter with a beam radiator/detector are one-dimensionally arranged along a line. Use of the single ultrasound emitter/radiator/detector array 95 arranged in this way may allow one to obtain one-dimensional optical imaging information without providing any scanning motion. Accordingly, this arrangement has an advantage of providing one-dimensional optical imaging information in short time.

Further, two-dimensional optical information may be obtained by imparting the ultrasound emitter/radiator/detector array 95 with one-dimensional scanning motion.

As will be described later, use of an ultrasound emitter/radiator/detector array 95B in which a plurality of ultrasound emitting/radiating/detecting portions are two-dimensionally arranged may also allow one to obtain the two-dimensional optical imaging information without using any scanning motion such as of an ultrasound transducer.

It will be appreciated that a light source 91 for generating light is not limited to the laser device 3a for generating coherent light, but a heat/light source such as a xenon light source or a halogen light source, or an LED or an SLD may be used.

It will also be appreciated that the present embodiment is so arranged that the scattering information can be calculated by extracting phase information, for example.

A beam of light within a wavelength region of visible light or near infrared rays generated by the light source 91 is made incident on each of a plurality (e.g., "p" in number) of optical fibers structuring an optical fiber array 92a. The beam incident on each optical fiber is divided, by an optical coupler 93, into a beam for an optical fiber in an optical fiber array 92c and a beam for an optical fiber 92bi ("i" indicates one of 1 to "p" number) in an optical fiber array 92b.

The beam directed to the optical fiber in the optical fiber array 92c is produced into a reference beam in the reference beam producer 55 as in the case shown in Fig. 14, and returns to the optical coupler 93. The optical modulator 18 and the reference mirror 14 extend at some length to a direction perpendicular to the drawing surface of Fig. 20 to modulate and reflect, respectively, a plurality of beams.

On the other hand, the beam directed to the optical fiber 92bi in the optical fiber array 92b is radiated to the tissue 7 through an aperture provided in an ultrasound transducer 57-i of each of the ultrasound emitting/radiating/detecting portions (structuring the ultrasound emitter/radiator/detector array 95).

As can be seen from an enlarged view in the lower right portion of Fig. 20, the ultrasound emitter/radiator/detector array 95 is provided therein with a plurality of ultrasound emitting/radiating/detecting portions along the x-direction. Each of the ultrasound emitting/radiating/detecting portions converges the ultrasound waves at the convergence point F with the aid of the ultrasound transducer 57-i, the component of each portion.

Meanwhile, a beam of light is radiated to the convergence point F from an end face of each of the optical fibers 92bi structuring the optical fiber array 92b, the end face serving as a beam radiator/detector. The end face of each of the optical fibers 92bi also receives a returning beam of light passing through the vicinity region R1 of the convergence point F.

Each of the beams received by the optical fibers 92bi in the optical fiber array 92b and serving as an observation beam is mixed, in the optical coupler 93, with a reference beam from each of the optical fibers in the optical fiber array 92c, so that an interference beam is produced.

Each interference beam Is directed by each of optical fibers in the optical fiber array 92d, received, through an optical fiber's end face, by each of the photo detectors 4a structuring a photo detector array 96, and subjected to photoelectric conversion. An output signal photoelectrically converted by each of the photo detectors 4a structuring the photo detector array 96 is inputted to the lock-in amplifier 63. The lock-in amplifier 63 is provided, for example, with a plurality of signal channels and performs high-speed signal processing suitable for each of the signals inputted through the plurality of signal channels.

The plurality of signals that have been processed by the lock-in amplifier 63 are memorized in a memory, for example, in the PC 61 through a plurality of input channels in the PC 6a.

A scanning signal generating circuit 24B in the present embodiment drives a scanning device 98 attached with the ultrasound emitter/radiator/detector array 95, for example, so as to effect scanning in the y-direction, for example, and to obtain the two-dimensional optical imaging information.

Fig. 21 illustrates a state where the two-dimensional (2D) optical imaging information is obtained by allowing the ultrasound emitter/radiator/detector array 95 to scan in the y-direction, for example. One-dimensional (1D) optical imaging information in the x-direction is obtained by the linearly arranged ultrasound emitting/radiating/detecting portions, and the two-dimensional optical imaging information can be obtained by allowing the emitting/radiating/detecting portions to scan in the y-direction.

It will be appreciated that the ultrasound emitter/radiator/detector array 95 may be driven by the scanning signal generating circuit 24B in such a way that scanning in the y- and z-directions can be performed, so that the three-dimensional optical imaging information can be obtained.

According to the present embodiment, the two- or three-dimensional optical imaging information can be obtained at high speed.

The x-direction resolution of the optical imaging information to be obtained depends on the number of the optical fibers 92bi in the optical fiber array 92b. Therefore, the resolution can be improved by providing the x-direction scanning as well.

**(Modifications of the fourth Embodiment)**

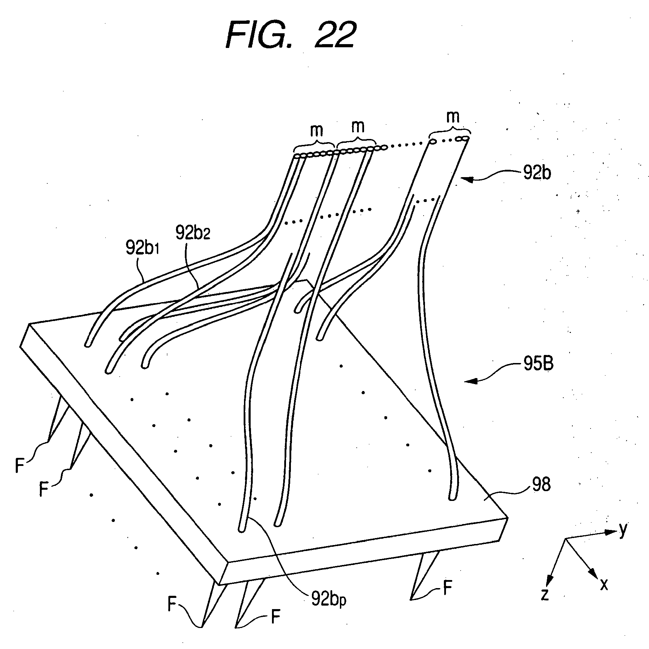

Fig. 22 is a schematic view of an ultrasound emitter/radiator/detector array 95B in an optical imaging apparatus according to a first modification. In the present modification, the more number of optical fibers are provided for structuring the optical fiber arrays 92a, 92b etc., in the optical imaging apparatus AP10 of Fig. 20 (e.g., equal to or more than twice of those shown in Fig. 20). For the increased number of optical fibers, the number of the emitting/radiating/detecting portions is also increased that much.

In the present modification, the emitting/radiating/detecting portions on the side of the end portion of the optical fiber array 92b are two-dimensionally arranged in x- and y-directions to structure the ultrasound emitter/radiator/detector array 95B.

As shown in Fig. 22, the optical fiber array 92b is formed by arranging, for example, "m" sets of "p" number of optical fibers, i.e. 92b1 to 92bp, in strips, for example. Each of the optical fibers 92bi is attached to each of apertures which are two-dimensionally provided in the scanning device 98.

An ultrasound transducer (not shown in Fig. 22) similar to the one shown in Fig. 20 is attached to a bottom surface of the scanning device 98. In a state where the ultrasound emitter/radiator/detector array 95B does not perform scanning, the two-dimensional optical imaging information (refer to Fig. 23) is ensured to be obtained.

In the present modification, the PC 6a stocks the output signal outputted from the lock-in amplifier 63 into a memory at each of addresses corresponding to the respective two-dimensionally arranged ultrasound emitting/radiating/detecting portions structuring the ultrasound emitter/radiator/detector array 95B.

Further, the present modification is so arranged that the three-dimensional optical imaging information can be obtained by driving the scanning device 98 in the z-direction using the scanning signal.

As described in the fourth embodiment, scanning motion in the x- and y-directions may also be provided in the present modification to obtain high-resolution optical imaging information. In the present modification as well, the two- or three-dimensional optical imaging information can be obtained at high speed.

As another modification of the fourth embodiment, an arrangement may be so made that the two-dimensional optical imaging information may be obtained without providing scanning motion.

In each of the modified embodiments provided above, an optical modulator may be provided in a path of the fiber array 92b to improve S/N ratio.

Further, in each of the modified embodiments provided above, it may be so arranged that beams of light may be converged. Further, both of ultrasound waves and beams of light in the forms of continuous waves may be radiated to the tissue of a living organism.

It will be appreciated that, alternative to the photo detector, any two-dimensional detector, such as a point detector, one-dimensional line sensor or CCD may be used.

As described above, according to the arrangements of the first to fourth embodiments and their modifications, an arrangement is so made that a beam of light is radiated to a region to be examined where the living organism tissue has been radiated with ultrasound waves, the beam is detected as an observation beam, and at least the scattering information corresponding to the imaginary part of the complex refractive index is detected to form an image. In this way, information which is highly correlated to the structural alteration of the lesion tissue can be obtained, which information can be effectively utilized in diagnosing, for example, the lesion tissue.

**(Fifth example)**

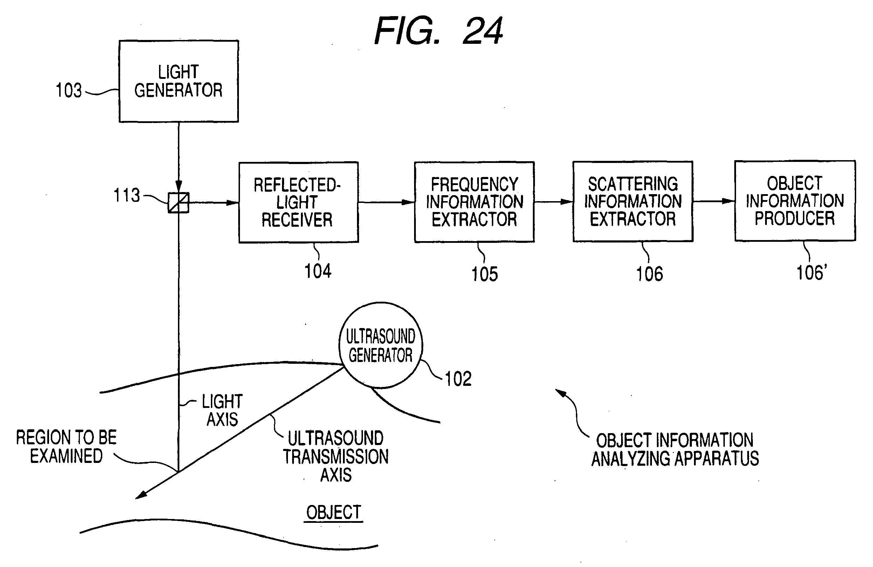

Referring to Figs. 24 - 28, a fifth example not forming part of the present invention will now be described.

Fig. 24 is a block diagram showing a typical configuration of an object information analyzing apparatus according to one aspect not forming part of the present invention.

As shown in Fig. 24, the object information analyzing apparatus is composed mainly of an ultrasound generator 102 and a light generator 103. The ultrasound generator 102 generates ultrasound waves transmitting through an object to be examined along a specified ultrasound transmission axis. The light generator 103 generates light to be radiated to a region to be examined (i.e., target region) of the object to be diagnosed to which the ultrasound waves generated by the ultrasound generator 102 also reaches.

The object information analyzing apparatus further has a reflected light receiver 104 and a frequency information extractor 105. For example, the reflected light receiver 104 is placed at the side of the light generator 103 (or the ultrasound generator 102) so as to receive the Doppler-shift frequency modulated light reflected at the target area in the object to be examined. The frequency information extractor 105 extracts the amount of the Doppler-shift light based on a received-light signal output from the reflection light receptor 104.

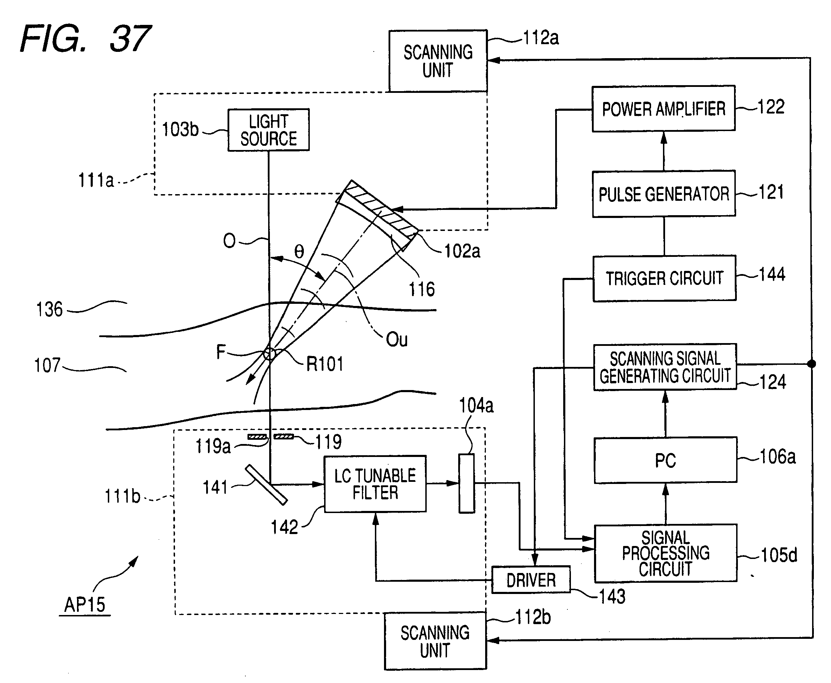

Although Fig. 24 shows the case of receiving the Doppler-shift reflected light by the reflected light receiver 104, it is acceptable to have a configuration in which the reflected light receiver 104 receives the penetrated light through the object, like a configuration (see Fig. 37) of a modification example of a seventh embodiment which will be explained later. In this case, the reflected light receiver 104 shown In Fig. 24 only acts as a receptor

Fig. 24 shows the configuration of electrically extracting the frequency information from the received light signal. The example is not limited by this configuration. It is acceptable to optically extract the frequency information from the Doppler-shift light by a liquid crystal tunable filter and the like as a frequency information extractor, similar to the configuration of the seventh example which will be explained later.

Such an optically frequency information extractor extracts the frequency information (as the Doppler-shift frequency component) from the Doppler-shift light before the reception by the reflection light receptor 104.

Fig. 24 shows the case of extracting the frequency information from the light signal, received by the reflected light receiver 104, by the frequency information extractor 105. It is possible to get the spectrum of the light received by the reflected light receiver 104 by a spectrometer (see the seventh embodiment described later, for example). In this configuration, the received light obtained by the reflected light receiver 104 is a signal including the frequency information, and is directly supplied to a scattering information extractor 106.

The object information analyzing apparatus of the fifth example further has the scattering information extractor 106 and an object information producer 106'. The scattering information extractor 106 extracts light scattering information corresponding to at least the real part of a complex refractive index of the target area of the object to be examined from the extracted frequency information. The object information producer 106' produces characteristic information corresponding to the target area of the object from the scattering information.

Fig. 24 shows the configuration of receiving the reflected light by the reflected light receiver 104 through a light separator 113.

The object information producer 106' has an image former configured to make and display the image of the scatted information and the like at each point, scanned in two or three dimension, of the target area in the object to be examined.

The object information analyzing apparatus of the fifth example is capable of obtaining, by extracting the Doppler-shift frequency information, the object information including the information regarding the real part of the complex refractive index to the target region of the object to be examined with relatively easy manner.

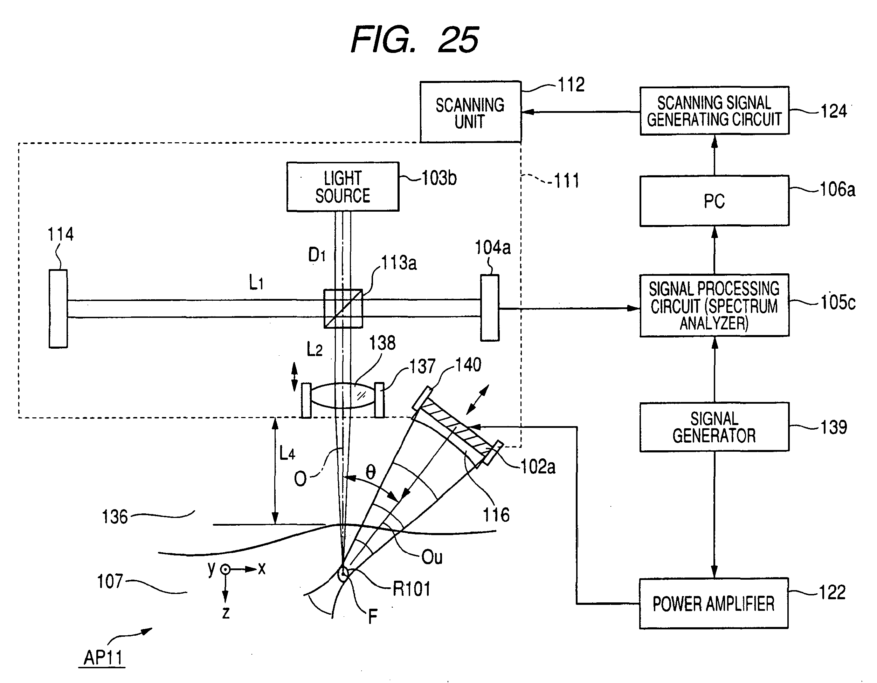

An optical imaging apparatus AP11 shown in Fig. 25 is equipped with a light source 103b as the light generator 103 in a unit 111 as a container. The light supplied from the light source 103b penetrates through a half mirror 113a as the light separator 113. The right separator 113 divides the received light into the penetrated light and the reflected light.

The light penetrated through the half mirror 113a is concentrated by a collimator lens (or a condensing lens) 138 mounted on a lens frame 137, and radiated to biological tissue 107 as the object.

An ultrasound transducer 102a is mounted on a side surface and the like of the unit 111 so that the direction of the angle $\theta$ (>0) to the optical axis O of the collimator lens 138 overlaps with an ultrasound transmission axis Ou.

The ultrasound transducer 102a is equipped with an acoustic lens 116 as an ultrasound wave focusing means.

As shown in Fig. 25, the light is scattered toward the optical axis O at a field vicinity region R101 surrounding the convergence point F and the scattering light undergoes the Doppler-shift (i.e., the scattering light is frequency-modulated). After the scattering, the Doppler-shift light is returned toward the side of the collimator lens 138 without being affected by the conversed ultrasound waves.

A part of the light returned to the side of the collimator lens 138 is reflected by the half mirror 113a and received by a photo detector 104a.

The light reflected by the half mirror 113a becomes a reference light, and is then reflected by a reference mirror 114 which is placed on a reference light path through which the reference light is passing so that the reference mirror 114 is faced to the reference light path, and the reflected light is then penetrated through the half mirror 113a. A part of the inputted light penetrates through the half mirror 113a. At this time, the photo detector 104a receives the mixed light composed of the Doppler-shift light and the reflected light.

The fifth embodiment described above shows the configuration in which the optical axis O Is different in direction from the ultrasound transmission axis Ou. This configuration enables to detect the scattering light obtained at the vicinity region R101 near the convergence point F without the effect of the ultrasound waves at the areas other than the vicinity region R101. The fifth example achieves the above feature by incorporating a signal generator 139 configured to generate a continuous ultrasound wave driving signal, for example. The ultrasound wave driving signal generated by the signal generator 139 is amplified by a power amplifier 122, and then applied to the ultrasound transducer 102a.

The lens frame 137 on which the collimator lens 138 is mounted is mated with a housing (omitted from the drawings) mounted on the unit 111. By shifting the lens frame 137 along the optical axis O, it is possible to focus the light into the convergence point F of the acoustic lens 116 capable of focusing the ultrasound wave. That is, the collimator lens 138 and the lens frame 137 form a radiation point adjustor to which the light is radiated.

The ultrasound transducer 102a is mounted on a movable frame 140. An ultrasound wave focusing adjuster is so formed that the convergence point F of the ultrasound wave can be adjusted by shifting the movable frame 140 toward the ultrasound transmission axis Ou. It is possible to perform the focus adjustment for the ultrasound wave so that the ultrasound wave is focused at the convergence point where the light is also focused by the collimator lens 138.

The unit 111 is equipped with a scanning unit 112 as a scanner capable of scanning the unit 111 in two dimensions, for example. The scanning unit 112 is driven based on a scanning signal transferred from a scanning signal generating circuit 124.

The scanning signal generating circuit 124 is controlled by PC106a that will be explained later.

The output signal from the photo detector 104a is supplied to a signal processing circuit 105c such as a spectrum analyzer as the frequency information extractor 105 which extracts the Doppler-shift component.

The frequency information extracted by the signal processor circuit 105c is transferred to a personal computer 106a (hereinafter, referred to as "PC 106a" for short) capable of acting as both of the scattering information extractor 106 and the object information producer 106'. The PC 106a extracts, from the frequency information, the information as the scattering information corresponding to the real part of the complex refractive index at the vicinity region R101 near the convergence point F of the biological tissue 107. The signal processing circuit 105c inputs the signal transferred from the signal generator 139. The signal processing circuit 105c outputs the extracted frequency information to the PC 106a in synchronization with the signal transferred from the signal generator 139.

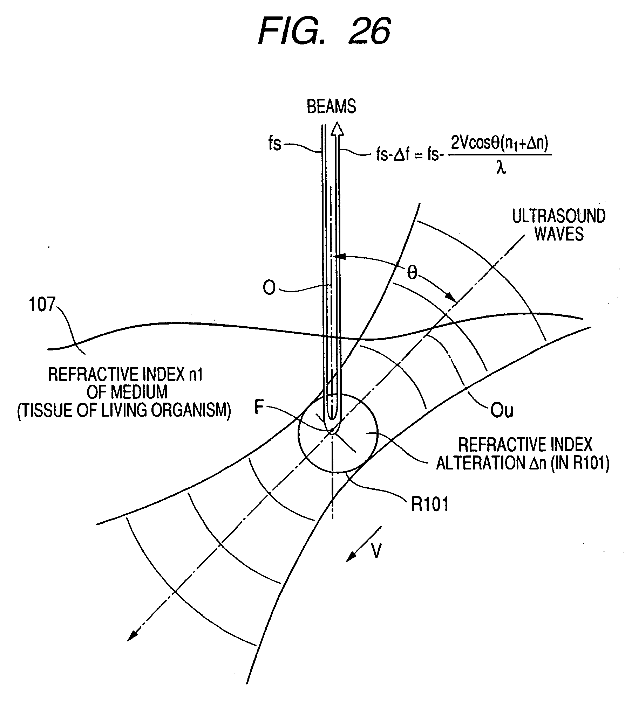

Fig. 26 shows the state of the Doppler-shift light by ultrasound wave at the vicinity region R101 surrounding the convergence point. In the relationship between the optical axis O and the ultrasound transmission axis Ou shown in Fig. 26, an angle made between both axes is $\theta$, the frequency of light is $f_s$, the wavelength of acoustic wave is $\lambda$, the transmission speed of the ultrasound wave in the biological tissue 107 is V, the refractive index of the biological tissue 107 is $n_1$, and the change of the refractive index at the vicinity region R1 by the ultrasound wave is $\Delta n$.

In a strict sense, although the real part and the imaginary part of the complex refractive index "m" of the biological tissue 107 are changed by the ultrasound wave radiation, the fifth example detects only the change of the real part when the primary change component (Doppler-shift frequency component) to the frequency of light, where the complex refractive index $m = m_r + im_l$, where $m_r$ is the real part and $m_l$ is the imaginary part of the complex refractive index. The change $\Delta n$ of the refractive index corresponds to the changed component $\Delta m_r$ of the real part $m_r$ in the complex refractive index.

The fifth example relating to the Doppler-shift uses a common manner using the reference character "n" which is widely used as the refractive index.

As shown in Fig. 26, the frequency of light returned to the input side after scattered at the vicinity region R1 surrounding the convergence point F becomes

$$f_s - \Delta f = f_s - 2V\cos\theta(n_1 + \Delta n)/\lambda \qquad (21).$$

The Doppler-shift amount $\Delta f$ as the secondary term of the formula (21) is detected in the fifth embodiment.

Next, a description will now be given of the operation of the object information analyzing apparatus according to the fifth. example.

At first, the signal detection operation by the photo detector 104a will be explained. The ultrasound wave output from the ultrasound transducer 102a transmits as a condensation and rarefaction wave through the inside of the biological tissue 107 as the object to be examined. In the ultrasound wave focusing area having a high sound pressure shown in Fig. 26, it can be considered that the refractive-index change $\Delta n$ is greatly induced at a high-density area of the sound pressure by a spatially density-change of body-component materials (as a scattering component and an absorption component) by the sound pressure. On the contrary, the rarefaction part of the material has a small density change.

On the other hand, when the light is radiated to the condensation area in the material, a strong Fresnel reflection occurs at the refractive-index change area. That is, the area in which the sound pressure is condensed acts as a mirror. In this case, because the refractive-index change area is shifted toward the transmission direction of the ultrasound wave according to the time elapse, the frequency of the reflected light is Doppler-shift by $\Delta f$.

In the case of the optical imaging apparatus AP11 forming Michelson interferometer as shown in Fig. 25, the electric field $E_r(t)$ at the reference mirror 114 and the electric field $E_o(t)$ at the measurement light to be inputted to the photo detector 104a can be expressed by the following formulae (22) and (23):

$$E_r(t) = E_r \exp i\{2\pi f_s t - k_o(D_1 + 2L_1 + D_2)\} \qquad (22)$$

$$E_o(t)=E_o \exp i\{2\pi(f_s-\Delta f)t-k_o(D_1+2L_2+2n_0L_4+2n_1z+D_2)\} \quad (23),$$

where $D_1$ designates the distance between the light source 103b and the half mirror 113a, $D_2$ indicates the distance between the half mirror 113a and the photo detector 104a, $L_1$ denotes the distance between the half mirror 113a and the reference mirror 114, $L_2+L_4$ indicates the distance on the surface of the biological tissue 107 into which the light from the half mirror 113a is radiated, $L_4$ means the distance of the surface of the biological tissue 107 measured from the collimator lens 138, and $n_0$ designates the refractive index of the of water 136.

The Doppler-shift amount $\Delta f$ can be expressed by the formula (21). According to the formula (21), it can be understood that the Doppler-shift amount $\Delta f$ includes the refractive index change $\Delta n$ induced by the ultrasound wave.

That is, because the refractive index is a main parameter of the light scattering phenomenon (for example, from Mie theory), there is a possibility of obtaining the local scattering characteristic by measuring the Doppler-shift frequency of the detected light.

The light intensity I(t) detected by the photo detector 104a can be expressed by the following formula (24):

$$
\begin{aligned}
I(t)&=<|E_r+E_o|^2>\\
&=<|E_r|^2+|E_o|^2>+\\
&\qquad 2<|E_r||E_o|>\cos\{2\pi(2V\cos\theta(n_1+\Delta n_1)/\lambda)t+2n_1k_o z\}
\end{aligned}
$$
$$(24),$$

where it is assumed that $(L_2+n_0L_4)\equiv L_1$.

Detecting AC component in the formula (24) as the frequency component by the spectrum analyzer enables to obtain the Doppler-shift frequency, namely, enables to perform the scattering measurement at a local area.

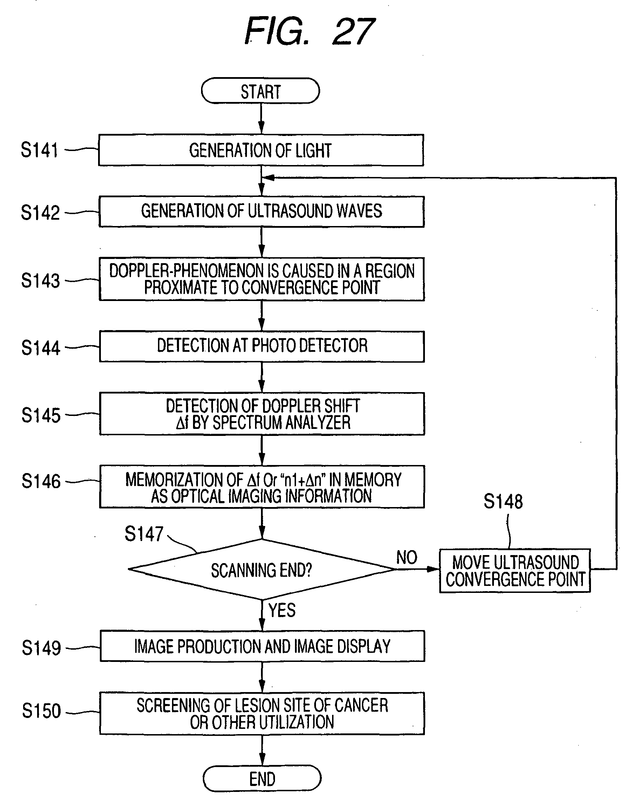

Next, a description will be given of the operation of the optical imaging apparatus AP11 according to the fifth example with reference to the flow chart shown in Fig. 27.

At initial step S141, the light source 103b generates light. At step S142, the ultrasound transducer 102a generates ultrasound waves. At step S143, the light from the light generator 103 causes Doppler frequency shift in the vicinity region R101 surrounding the convergence point F of the biological tissue 107. As shown by the formula (21), the light of the Doppler-shift amount $\Delta f$ interferes with the reference light at the half mirror 113a in order to generate the interference light. This interference light is detected by the photo detector 104a at step S144.

At step S145, the signal processing circuit 105c such as the spectrum analyzer detects the Doppler-shift amount $\Delta f$ based on the interference signal.

At step S146, the PC106a converts the output signal of the signal processing circuit 105c to an electric digital signal (electric signal in short). This electric signal is then stored in a memory and the like in the PC 106a as the light scattering information $\Delta f$ or "$n_1+\Delta n$" in connection with scanning position information. The transmission speed V, the wavelength $\lambda$, and the angle $\theta$ of the ultrasound wave are already-known values.

The PC106a judges whether or not the scanning is completed at step S147. When the judgment result indicates that the scanning operation is not completed, the scanning is performed by shifting the convergence point F of the ultrasound wave at step S148. The operation flows is then returned to step S142 or S143.

When the scanning in two or three dimensions is completed, the operation flow is forwarded to step S149 from step S147. The image generation is thereby completed. The obtained image is then displayed.

The operator observes the obtained optical image displayed at step S150 for the screening of a lesion such as a cancer.

In the above explanation, although the Doppler-shift amount $\Delta f$ is detected at step S145 by the spectrum analyzer, it is possible to detect this amount based on Fourier transform.

Hereinafter, the manner of obtaining the Doppler-shift amount $\Delta f$ based on Fourier transform will be explained.

As described above, the photo detector 104a detects the light intensity I(t) in the formula (24) that is expressed by the formula (25) using a parameter $I_{dc}$ for the primary and secondary terms, and parameters $I_{ac}$ and $\phi$ for the third term.

$$I(t)=I_{dc}+I_{ac}\cos(2\pi\Delta f t+\phi)=I_{dc}+I_{ac}\cos(\Delta\omega t+\phi) \quad (25),$$

where $\phi=2n_1k_0z$.

The signal processing circuit 105c performs Fourier transform for the formula (25). As a result, the formula (26) can be obtained:

$$F(\omega)$$
$$= \int \{I_{dc} + I_{ac}\cos(\Delta\omega t + \phi)\} e^{-j\omega t} dt$$
$$= \int \{I_{dc} + I_{ac}\cos(\Delta\omega t + \phi)\} e^{-j\omega t} dt \quad (-T \leq t \leq T)$$
$$= 2I_{dc}\sin\omega T/\omega + I_{ac}\{e^{j\phi}\sin(\Delta\omega - \omega)T/(\Delta\omega - \omega) + e^{-j\phi}\sin(\Delta\omega + \omega)T/(\Delta\omega + \omega)$$
$$= 2I_{dc}\sin\omega T/\omega + I_{ac}\cos\phi\{\sin(\Delta\omega - \omega)T/(\Delta\omega - \omega) + \sin(\Delta\omega + \omega)T/(\Delta\omega + \omega)\} + jI_{ac}\sin\varphi\{\sin(\Delta\omega - \omega)T/(\Delta\omega - \omega) + \sin(\Delta\omega + \omega)T/(\Delta\omega + \omega)\}$$
$$(26),$$

where the primary integration in the formula (26) has the range of $-\infty$ to $+\infty$, the secondary integration can be approximated in the range of the finite time -T to +T.

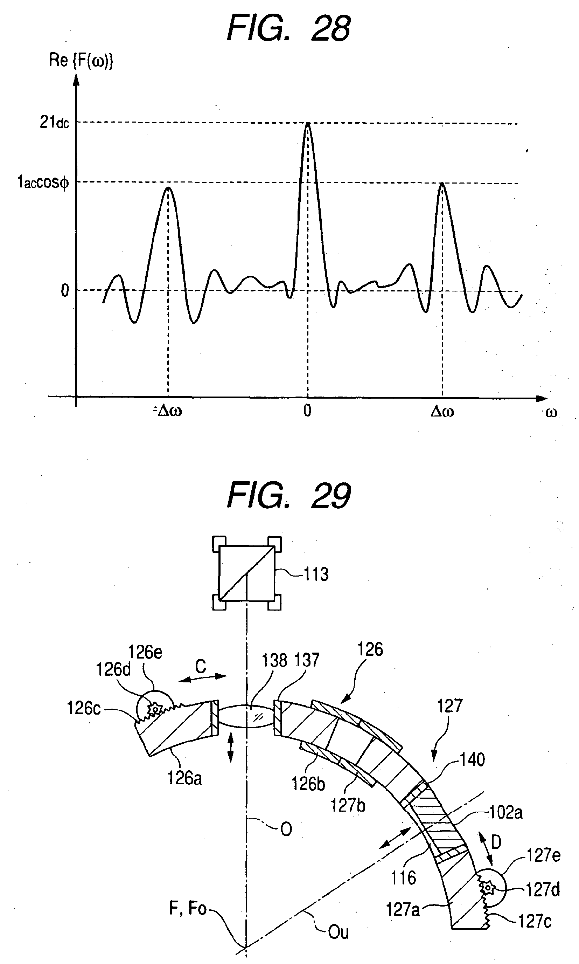

Fig. 28 is a graph showing the real part of the signal in the formula (26) after performing Fourier transform. As shown in Fig. 28, the Doppler-shift frequency $\Delta\omega$ taking the large peaks can be detected.

The fifth example enables to provide the analyzer capable of observing the scattering information of the biological tissue 107 as the object to be examined, that is, the morphological information of cells and components forming the cell in the biological tissue 107.

The extraction of the Doppler-shift amount $\Delta f$ can easily also extract the information regarding the real part of the complex refractive index when compared with conventional manners. Accordingly, it is possible to easily obtain the morphological characteristic information of the object to be examined.

It is also possible to extract the frequency component using Wavelet transform instead of extracting (or calculating) the frequency components based on Fourier transform.

**(Modifications of the fifth example)**

<First modification>

Fig. 29 shows the first modification of the optical imaging apparatus, in particular, mainly shows a lens supporter 126 as a light axis supporter and the periphery of a transducer supporter 127 as the ultrasound transmission axis supporter.

The lens supporter 126 has a movable supporter 126a and a fixed supporter 126b. The movable supporter 126a shiftably supports the lens frame 137 through a penetrate hole which engages the lens supporter 126. The lens frame 137 is equipped with the collimator lens 138. The fixed supporter 126b engages the movable supporter 126a so as to rotatably and shiftably support one end thereof.

A rack part 126c is mounted on the other end of the movable supporter 126a and engages a gear 126d mounted on the end of a knob 126e. The operator turns the knob 126e in order to shift the movable supporter 126a as designated by reference character "C" shown in Fig. 29. This configuration enables to adjust the direction of the optical axis O of the collimator lens 138 by shifting the optical axis O thereof.

Similarly, the transducer supporter 127 has a movable supporter 127a and a fixed supporter 127b. The movable supporter 127a is shiftably supported by the penetrate hole which engages the movable frame 140 on which the ultrasound transducer 102a is mounted. The fixed supporter 127b engages one end of the movable body 127 and rotatably and shiftably supports the movable body 127.

The other end of the movable supporter 127a is equipped with a rack part 127c which engages a gear 127d mounted on the end of a knob 127e. The operator or user turns the knob 127e in order to shift the movable supporter 127a as expressed by reference character "D" shown in Fig. 29. This configuration enables to adjust the direction of the ultrasound transmission axis Ou of the ultrasound transducer 102a by shifting the ultrasound transmission axis Ou thereof.

As shown in Fig. 29, the adjustment of the lens frame 137 toward the optical axis O direction enables to adjust the convergence point Fo of the light. Similarly, the adjustment of the movable frame 140 enables to adjust the convergence point F of the ultrasound wave.

For example, a light adjuster is mounted in order to coincide the convergence point Fo of the light to the convergence point F of the ultrasound wave. A focus adjuster for the ultrasound wave is further mounted in order to coincide the convergence point F of the ultrasound wave to the convergence point Fo of the light.

This modification example provides the configuration in which both the movable supporters 126a and 127a are rotatably and shiftably supported on a common surface (including the optical axis O and the ultrasound transmission axis Ou) by the fixed supporters 126b and 127b. This configuration enables to focus the light and the ultrasound wave into the area to be examined even if the light is different from the ultrasound transmission axis.

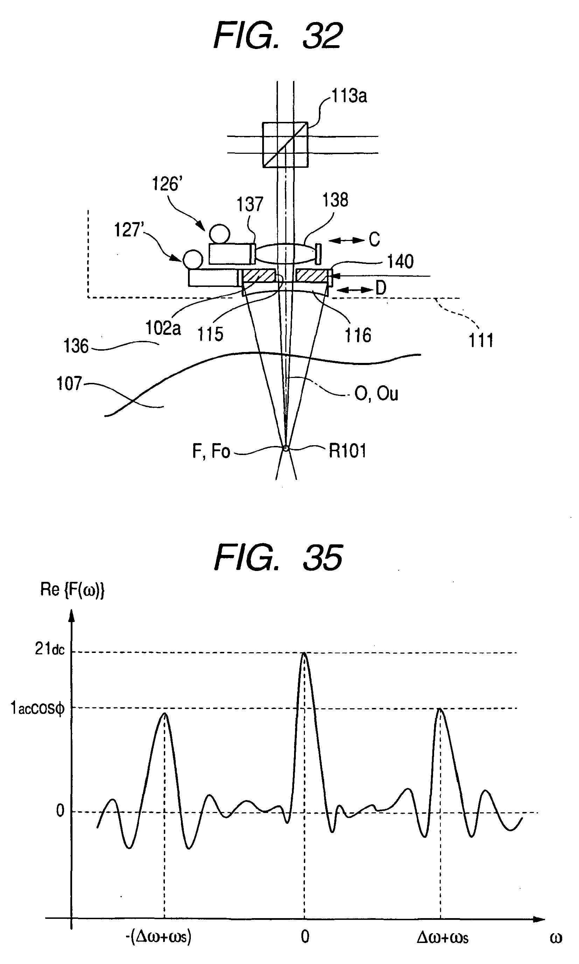

Fig. 29 shows the focusing adjustment and direction adjustment mechanism when the optical axis O is different in direction from the ultrasound transmission axis Ou. It is possible to take another manner of placing the lens supporter 126 and the transducer 127 in coaxial or lamination arrangement in order to coincide the optical axis O and the ultrasound transmission axis Ou. The configuration of such a manner will be explained later (see Fig. 32).

<Second modification>

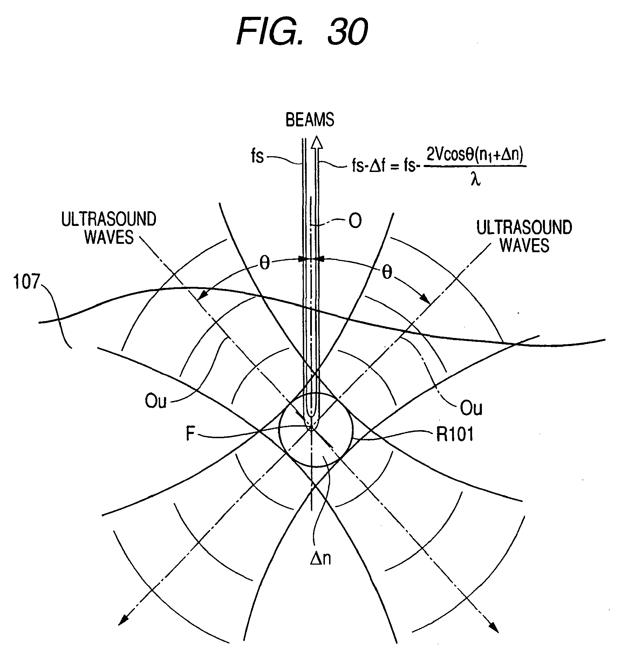

Fig. 30 shows the area near the convergence point F of the ultrasound wave according to the second modification example.

Although Figs. 25 and 26 show the configurations to provide the ultrasound transducer 102a only placed in the direction of the angle $\theta$ to the optical axis $\theta$, it is also acceptable to place the ultrasound transducer 102a in each of two directions of the angle $\theta$ (namely, two directions $\pm\theta$) to the optical axis $\theta$, as shown in Fig. 30. This configuration shown in Fig. 30 enables to increase the intensity of back-scattering light and also to increase S/N ratio thereof. It is further acceptable to place not less than two ultrasound transducers 102a in symmetry of rotation around the optical axis O.

**(Sixth example)**

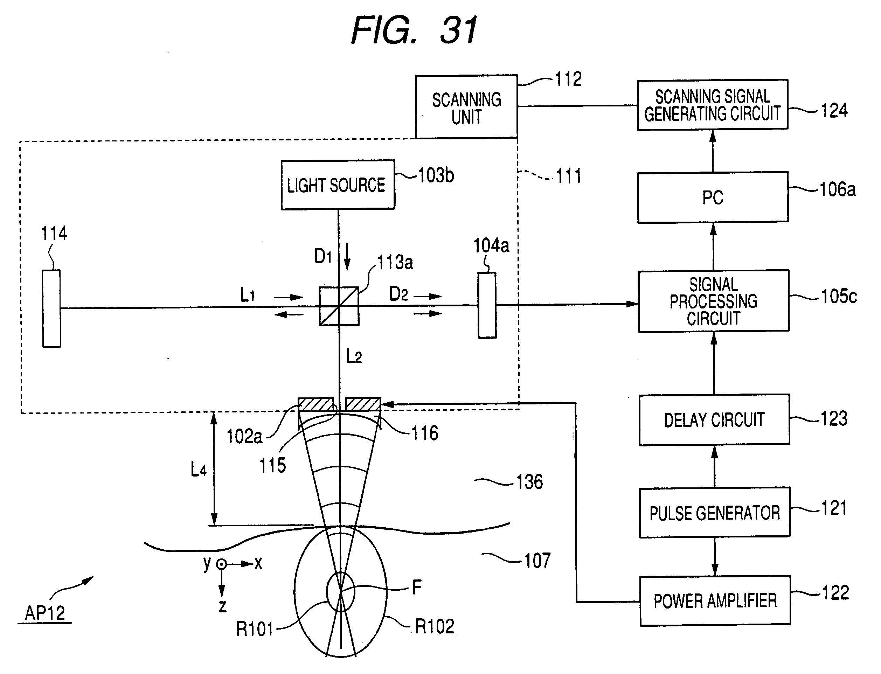

Referring to Fig. 31, a sixth example will now be described.

Fig. 31 shows the configuration of an optical imaging apparatus AP12 according to the sixth example.

The fifth embodiment previously explained shows the optical axis O that is different in direction from the ultrasound transmission axis Ou. On the contrary, the sixth embodiment provides the configuration in which the optical axis O coincides in direction with the ultrasound transmission axis Ou. The configuration of the sixth embodiment enables to reduce the entire size of the part that radiates the light and the ultrasound wave to the biological tissue 107 of the object. In the sixth example, the pulsed ultrasound wave is radiated in order to effectively detect only the Doppler-shift amount $\Delta f$ at the vicinity region R101 near the convergence point F of the ultrasound wave. In order to realize this function, a pulse generator 121 (see Fig. 31) is mounted instead of the signal generator 139 shown in Fig. 25.

The signal processing circuit 105c inputs the pulse delayed by a delay circuit 123, and then performs the frequency detection or frequency extraction of the Doppler-shift amount $\Delta f$ within the delayed short period of time.

Other same components in the configuration of the fifth example will be referred to with the same reference numbers, and the explanation thereof is omitted here. Although the configuration shown in Fig. 31 focuses the ultrasound wave and not focuses the light, it is acceptable to focus the light in addition to the ultrasound wave. This configuration further enables to increase the S/N ratio of the detected signal.

Because the angle $\theta$ between the optical axis $\theta$ and the ultrasound transmission axis Ou becomes zero ($\cos\theta=1$), namely, the both axes coincide, it is possible to detect the Doppler-shift amount $\Delta f$ at the maximum shifted frequency. As described above, the configuration of the sixth example enables to further reduce the size of the part which radiates the light and ultrasound wave to the biological tissue 107 in the object.

It is acceptable to supply the continuous electric signal, not the electric pulse signal, to the power amplifier 122 in order to radiate the continuous ultrasound wave to the biological tissue 107 by the ultrasound transducer 102a. It is acceptable to have another configuration in which the pulse generator 121 radiates the reference pulse signal to the signal processing circuit 105c at the time interval corresponding to the wavelength of the ultrasound wave in order to perform the synchronous detection. Such a case also enables to have the same effect of radiating the pulsed ultrasound wave.

**(Modifications of the sixth example)**

<First modification>

Fig. 32 shows a lens supporter 126' and the periphery of a transducer supporter 127' in the first modification adopted into the configuration shown in Fig. 31. The light of the light source 103b penetrates through the half mirror, passes through the collimator lens 138 in the lens supporter 126' mounted on the optical axis O, and further passes through an aperture 115 of a transducer supporter 127' mounted adjacent to the lens supporter 126' so as to approximately coincide with the optical axis O, and is then focused and radiated to the biological tissue 107.

The lens supporter 126' and the transducer supporter 127' can be shifted toward the direction perpendicular to the optical axis O and the ultrasound transmission axis Ou, instead of the configuration of the lens supporter 126 and the transducer supporter 127 enabling to rotatably move, as shown in Fig. 29.

A light axis adjuster is so formed that the optical axis O coincides with the ultrasound transmission axis Ou by shifting the optical axis O toward the direction expressed by reference character C by adjusting the lens supporter 126', for example.

A transmission axis adjuster is so formed that the ultrasound transmission axis Ou coincides with the optical axis O by shifting the ultrasound transmission axis Ou toward the direction designated by reference character D by adjusting the transducer supporter 127'.

Further an adjustment may be made such that the convergence point F and the convergence point Fo are made to agree with each other by shifting the lens frame 137 toward the direction of the optical axis O.

According to this modification example, it is possible to perform the adjustment of coincidence of the convergence point F and the convergence point Fo even if the lens supporter 126'and the transducer supporter 127' have characteristic variations. The configuration of the first modification of the sixth embodiment enables to obtain the optical imaging information with a high S/N ratio and high resolution. Because of focusing the light, it is possible to extract the frequency information with a high S/N ratio and high resolution.

In the examples described above, it is acceptable to use either a pulsed ultrasound wave or a continuous ultrasound wave, and also to use, as the photo detector 104a, a two dimensional detector such as a CCD, a one-dimensional line sensor, and a point detector such as a photo-detector and a photomultiplier. It is still further possible to detect the Doppler-shift amount by Fourier transform in the configurations of the fifth and sixth embodiments.

**(Seventh example)**

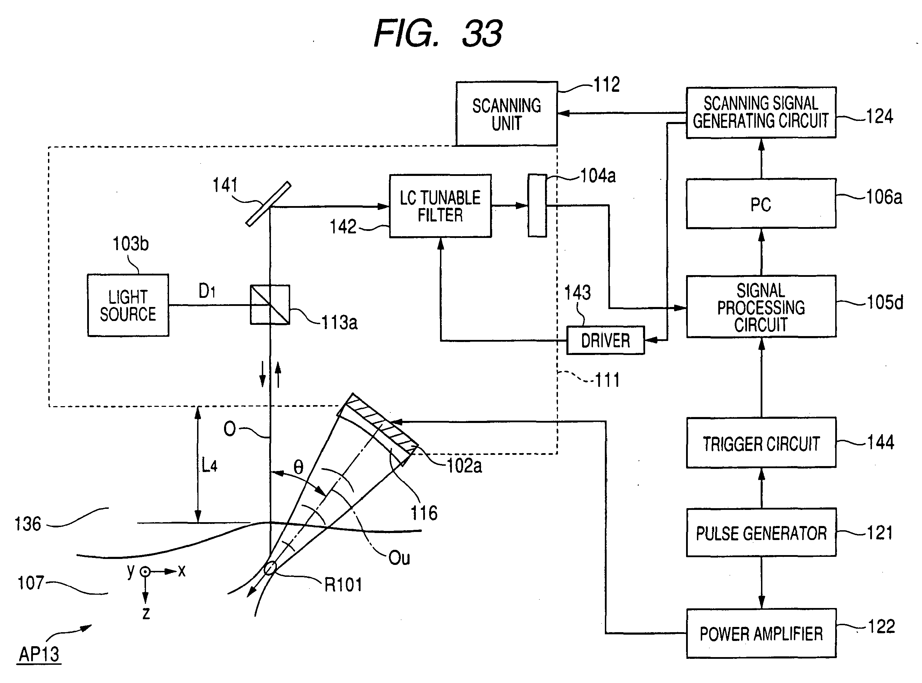

Referring to Figs. 33 - 34, a seventh example will now be described.

The fifth and sixth examples have explained the configuration equipped with Michelson interferometer. On the contrary, the configuration of the seventh example is equipped with a spectrum means such as a liquid crystal tunable filter instead of the interferometer.

Fig. 33 shows an optical imaging apparatus AP13 according to the seventh example. The light of the light source 103b mounted on the unit 111 is inputted into a half mirror 113a. The half mirror 113a then reflects the light from the light source 103b and the reflected light is radiated to the biological tissue 107 as the object immersed in water 136. The unit 111 is shifted in two or three dimensions by the scanning unit 112. Although the seventh example is applied to the biological tissue 107 immersed in water 136, it is possible to apply the seventh example to a balloon involving water or to directly contact the acoustic lens 116.

The ultrasound wave output from the ultrasound transducer 102a mounted on the end of the unit 111 is focused by the acoustic lens 116 and then radiated onto the inside of the biological tissue 107, along the ultrasound transmission axis Ou having the angle θ to the optical axis O, through the water 136 capable of propagating the ultrasound wave.

The light is Doppler-shifted at the vicinity region R101 near the convergence point F of the ultrasound wave. The back-scattering light is radiated into the half mirror 113a and a part thereof passes through the half mirror 113a. The light passing through the half mirror 113a is reflected by the mirror 141 and inputted into the liquid crystal tunable filter 142 as the spectrum means. The liquid crystal tunable filter 142 changes its penetrating wavelength band according to a driving signal supplied from a driver 143. The driver 143 drives the liquid crystal tunable filter 142 in synchronization with a scanning signal transferred from the scanning signal generating circuit 124, for example.

The photo detector 104a receives the light passing through the liquid crystal tunable filter 142, and outputs the output signal to the PC 106a through the signal processing circuit 105d.

The signal processing circuit 105d and the PC 106a perform the extraction of the scattering information, the generation of the object information, and more concretely, performs the image formation based on the output signal from the photo detector 104a. In this case, it is acceptable that the signal processing circuit 105d performs the extraction for the scattering information, and the PC 106a executes the image formation.

Together with the driving signal for sweeping the penetrating wavelength, the electric signal converted from the light during the photoelectric conversion by the photo detector 104a is temporarily stored into the memory and the like in the PC 106a.

The penetrating wavelength (or the Doppler-shift amount $\Delta f$ at the detection of the Doppler-shift is calculated by the following manner.

On detecting the Doppler-shift signal based on the output signal transferred from the photo detector 104a, the PC 106a (or the CPU therein) extracts the peak signal as the Doppler-shlft signal detected over a threshold value by a comparator (not shown), for example.

The penetrating wavelength is calculated from the information such as the driving signal at the time of detecting the peak signal. The calculated wavelength is stored into the memory together with the information regarding the penetrating wavelength calculated.

It is acceptable to detect the Doppler-shift signal based on the output signal of the photo detector 104a by Fourier transform by the PC 106a, that will be explained later.

The pulse generated by the pulse generator 121 and the pulsed ultrasound wave driving signal amplified by the power amplifier 122 are provided to the ultrasound transducer 102a. The pulse generated by the pulse generator 121 is inputted into the signal processing circuit 105d through a delay circuit or a trigger circuit 144, for example. The signal processing circuit 105d outputs the scattering information extracted according to the pulse to the PC 106a.

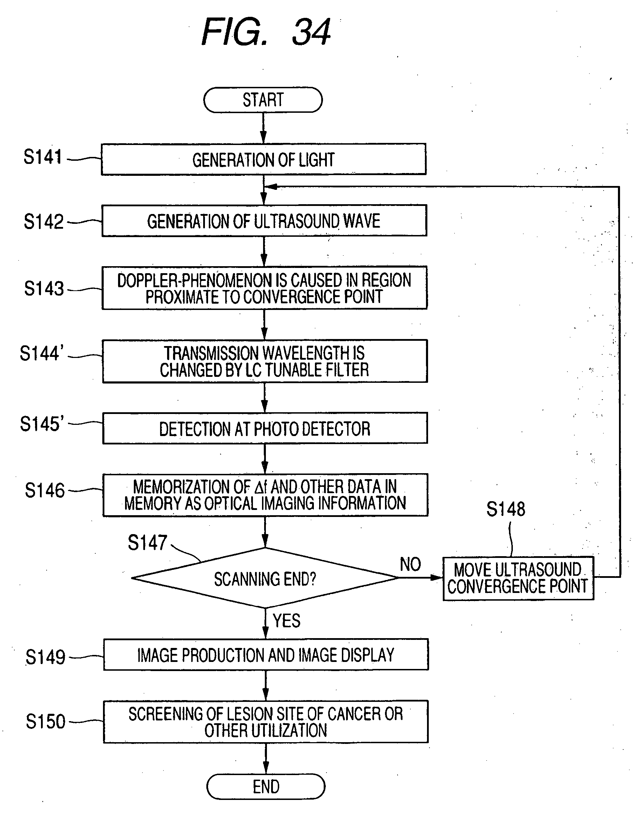

Although the ultrasound transducer 102a is driven by pulse signals (intermittently) in the seventh example, it is acceptable to drive it using continuous waves. The operation of the seventh example will be explained with reference to the flow chart shown in Fig. 34 and will be explained for short while referring the flow chart shown in Fig. 27 according to the fifth example.

Like the case shown in Fig. 27, the light and the ultrasound wave are generated at steps S141 and S142. As described above, the continuous ultrasound wave can be used instead of the pulsed ultrasound wave. As shown in step S143, the Doppler-shifting phenomenon occurs at the vicinity region R101 near the convergence point F. In the occurrence of the Doppler-shifting phenomenon, the light intensity I(t) takes the value obtained by multiplying the values of the formulae (22) and (23). The following formula (27) expresses the light intensity I(t),

$$I(t) = I_{dc} + I_{ac}\cos\{2n(f_s + \Delta f)t + \phi\} = I_{dc} + I_{ac}\cos\{(\omega_s + \Delta\omega)t + \phi\} \tag{27}.$$

As shown in step S144', the penetrating wavelength is changed by the liquid crystal tunable filter 142. At step S145', the photo detector 104a detects the light passing through the liquid crystal tunable filter 142.

The output signal of the photo detector 104a is inputted into the PC 106a through the signal processing circuit 105d. The signal processing circuit 105d or the PC 106a actually extracts the Doppler-shift signal from the inputted signal. The PC 106a stores the information such as the value $\Delta f$, shown in step S146, as the optical imaging information, into the memory.

Because the following process of step S147 to step S150 are same of those in the flow chart shown in Fig. 27, the explanation thereof is omitted here.

Because of not requiring any interferometer, the configuration of the seventh example has the advantage of reducing the size of the apparatus.

Although the above explanation shows the detection of the Doppler-shift signal based on the output signal from the photo detector 4a using the comparator and the like, it is possible to detect the Doppler-shift signal based on Fourier transform as follows.

PC 106a (or the signal processing circuit 105d) performs Fourier transform for the formula (27) and provides the transformation result expressed by the formula (28).

$$\begin{aligned}
F(\omega) &= \int\{I_{dc} + I_{ac}\cos(\Delta\omega t + \phi)\}e^{-j\omega t}dt \\
&= \int\{I_{dc} + I_{ac}\cos(\omega_s t + \Delta\omega t + \phi)\}e^{-j\omega t}dt \quad (-T \leq t \leq T) \\
&= 2I_{dc}\sin\omega T/\omega + I_{ac}\cos\varphi\{\sin(\Delta\omega + \omega_s - \omega)T/(\Delta\omega + \omega_s - \omega) + \\
&\quad \sin(\Delta\omega + \omega_s + \omega)T/(\Delta\omega + \omega_s + \omega)\} + \\
&\quad jI_{ac}\sin\varphi\{\sin(\Delta\omega + \omega_s - \omega)T/(\Delta\omega + \omega_s - \omega) + \\
&\quad \sin(\Delta\omega + \omega_s + \omega)T/(\Delta\omega + \omega_s + \omega)\}
\end{aligned} \tag{28},$$

where the primary integration in the formula (28) has the range of $-\infty$ to $+\infty$, and the secondary integration has the range $-T \leq t \leq T$.

The real part of the signal after executing Fourier transform shown in the formula (28) takes the values in the graph shown in Fig. 35. The angle frequency $\omega_s + \Delta\omega$ at the large peak-value is detected and the angle frequency $\Delta\omega$ of the Doppler-shift signal is detected by subtracting a known angle frequency $\omega_s$, as shown in Fig. 35.

**(Modifications of the seventh example)**

<First modification>

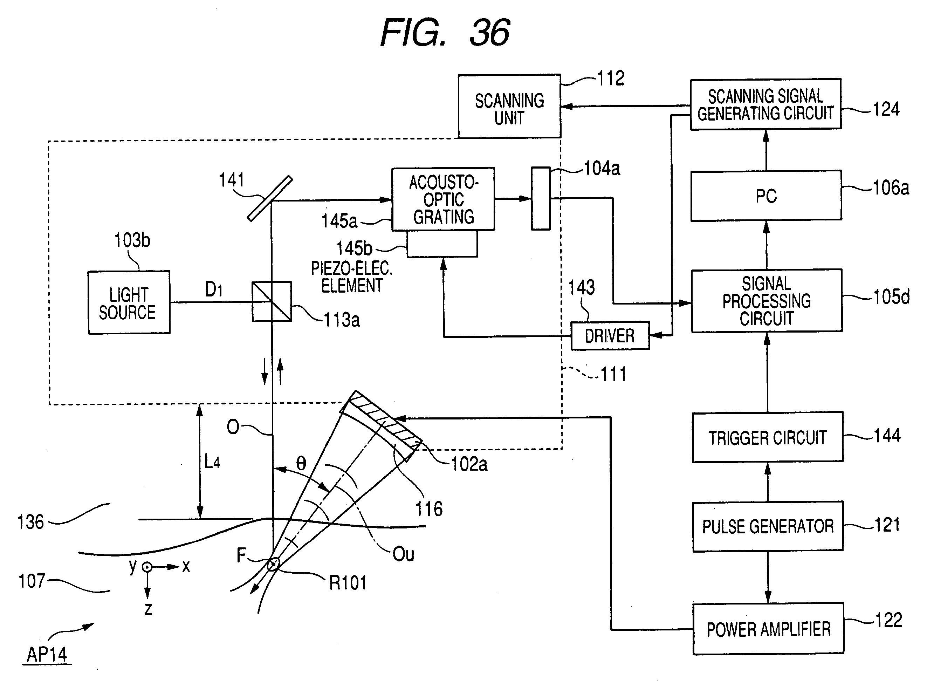

Fig. 36 shows the configuration of an optical imaging apparatus AP14 according to the first modification example of the seventh example. The configuration shown in Fig. 33 uses an acoustic-optical diffraction grating and a piezoelectric element 145b instead of the liquid crystal tunable filter 142. The acoustic-optical diffraction grating and the piezoelectric element 145b change the primary angle of diffraction to the wavelength of the inputted light by changing the pitch of the grating when the driving signal transferred from the driver 143 is provided to the piezoelectric element 145b. That is, the acoustic-optical diffraction grating 145a outputs (in different directions) the spectral diffracted (wavelength spectrum) light, like a spectrometer.
The photo detector 104a detects the light after the wavelength spectrum process performed by the acoustic-optical diffraction grating 145a.
Other components are the same of those of the configuration shown in Fig. 33. The action and effect of the first modification of the seventh example are the same of those of the device shown in Fig. 33.

<Second modification>

Fig. 37 shows an optical imaging apparatus AP15 according to the second modification of the seventh example. The optical imaging apparatus AP15 is obtained by using the device shown in Fig. 33 to the penetration-type device.
The light from the light source 103b placed at the side of the unit 111a passes along the optical axis O, and then through the water 136, and is radiated into the inside of the biological tissue 107.
This light is Doppler-shifted in the ultrasound wave focusing area and a part of the light passes along the optical axis O, and penetrates the biological tissue 107. The light is then radiated into the mirror 141 through the aperture 119a of a shielding plate 119 placed on the optical axis O at the unit 111b. The light is reflected by the mirror 141 and the reflected light is then radiated to the liquid crystal tunable filter 142. The light passing through the liquid crystal tunable filter 142 is detected by the photo detector 104a. Each of the units 111a and 111b is shifted in two or three dimensions by the scanning unit 112a or 112b, respectively. Other same components in the configuration shown in Fig. 33 and another figure will be referred to with the same reference numbers, and the explanation of those same components is omitted here.
The second modification of the seventh example detects the penetrated light instead of detecting the reflected light (returned light) in the device shown in Fig. 33. Other action and effect of this second modification are the same of those of the device shown in Fig. 33.
It is acceptable to use a spectrometer such as a spectroscope and an acoustic optical element instead of the liquid crystal tunable filter 142.
It is also possible to have a configuration of detecting the penetrated light instead of the device shown in Fig. 36.
It is further possible to use a point detector such as a photodiode and a photo multiplier or a two dimension detector such as a one dimensional line sensor and a CCD as the photo detector 104a shown in each example described above.
Still further, it is acceptable to use one of the continuous ultrasound wave and a pulsed ultrasound wave by adjusting the shape of driving the pulse generator 121 instead of the configurations of the modifications shown in Fig. 36 and Fig. 37. It is possible to detect the Doppler-shift amount based on Fourier transform without using the spectrometer means in the each example and modification described above.

**(Eighth example)**

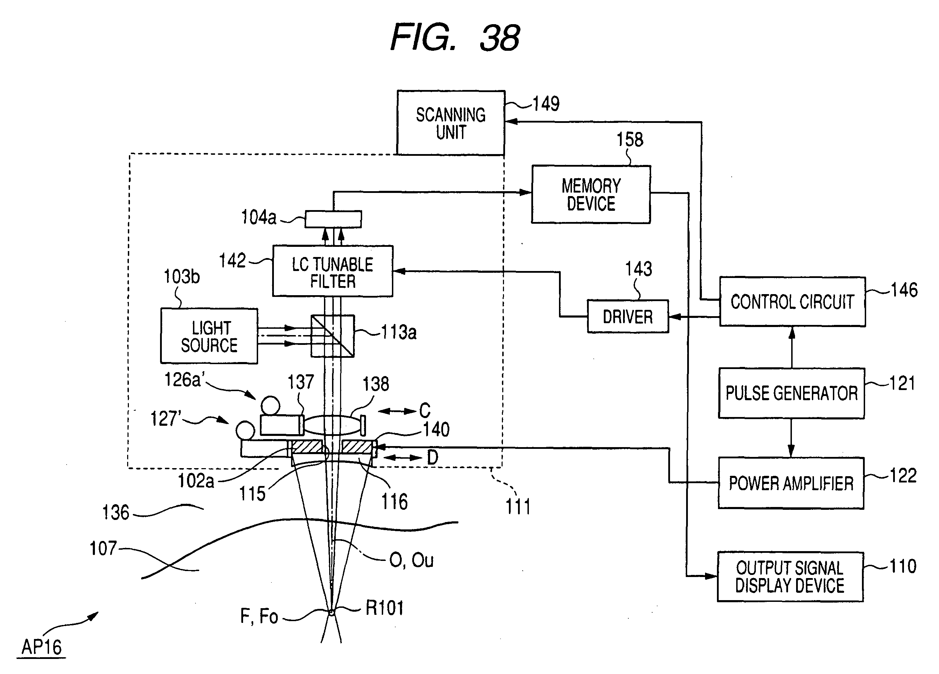

Referring to Figs. 38 - 39, an eighth example will now be described.
The eighth embodiment provides the configuration with a reduced size in which the ultrasound wavelength transmission axis Ou approximately equal or overlaps with the optical axis O, just like the configuration of the sixth example shown in Fig. 31. The configuration of the eight example detects the Doppler-shift amount without executing interference with the reference light. This feature of the eighth example is different from the feature of the sixth example.
An optical imaging apparatus AP 16 shown In Fig. 38 has the configuration similar to that of the optical imaging apparatus AP12 shown in Fig. 31. Although the configuration shown in Fig. 31 requires the interference with the reference light, the configuration of the eighth example can detect the Doppler-shift amount, without interference with the reference light, by performing the optical spectrum just like the seventh example, for example.
A part of the continuous light from the light source 103b capable of generating the continuous light is reflected by the half mirror 113, focused by the collimator lens 138, and then radiated onto the biological tissue 107. The Doppler-shift light, reflected by the vicinity region R101 of the biological tissue 107 to which the ultrasound wave is focused, comes into the half mirror 113a. A part of the reflected Doppler-shift light passes through the half mirror 113a and then comes into the optical spectrometer means such as the liquid crystal tunable filter 142, for example.

The transmission wavelength of the liquid crystal tunable filter 142 is changed according to the driving signal transferred from the driver 143.

The photo detector 104a Inputs the light which has penetrated through the liquid crystal tunable filter 142 and converted to the electric signal by the photoelectric conversion. The output signal from the photo detector 104a is inputted into a memory device 158m, and converted to a digital signal by the A/D converter in the memory device 158m, and stored into the memory together with the scanning information. The information stored in the memory device 158m is output to the output device, and displayed on an output signal display device 110 when one-frame information is generated, for example.

The pulse generated by the pulse generator 121 becomes the pulsed ultrasound wave driving signal through the power amplifier 122 and is provided to the ultrasound transducer 102a. The ultrasound transducer 102a generates the pulsed ultrasound wave on receiving the pulsed ultrasound wave driving signal.

The control circuit 146 receives the pulse transferred from the pulse generator 121 and controls the scanning performed by a scanning unit 149 in synchronization with the pulsed ultrasound wave. The control circuit 146 further controls the driving signal generated by the driver 143 in synchronization with the pulsed ultrasound wave. The memory device 158m stores the output signal from the photo detector 104a In synchronization with the pulsed ultrasound wave.

The configuration of the eighth example is equipped with the lens supporter 126' and the transducer supporter 127' shown in Fig. 32 previously explained, for example.

The light from the light source 103b is focused by the collimator lens 138, and such a focused light is propagated on the optical axis O of the collimator lens 138. The light then passes through the aperture 115 of the ultrasound transducer 102a and is finally radiated, through the water 136, to the inside of the biological tissue 107 as the object to be examined.

A part of the Doppler-shift light reflected in the vicinity region R101 near the convergence point passes through the collimator lens 138 and the half mirror 113a, and is then inputted to the liquid crystal tunable filter 142. The Doppler-shift light is extracted from the liquid crystal tunable filter 142 as the signal light. The pulsed ultrasound wave output from the ultrasound transducer 102a is focused by the acoustic lens 116, and is then radiated into the side of the biological tissue 107. The configuration of the eighth embodiment is capable of adjusting the optical axis O of the collimator lens 138 and the ultrasound transmission axis Ou in order to coincide them to each other.

A light axis adjuster is so formed that the optical axis O coincides with the ultrasound transmission axis Ou by adjusting the lens supporter 126', for example.

A transmission axis adjuster is so formed that the ultrasound transmission axis Ou agrees with the optical axis O by adjusting the transducer supporter 127'.

The unit 111 is driven in two or three dimensions by the scanning unit 149 under the control of the control circuit 146. This scanning unit 149 has both functions of the scanning signal generating circuit 124 and the scanning unit 112 shown in Fig. 34.

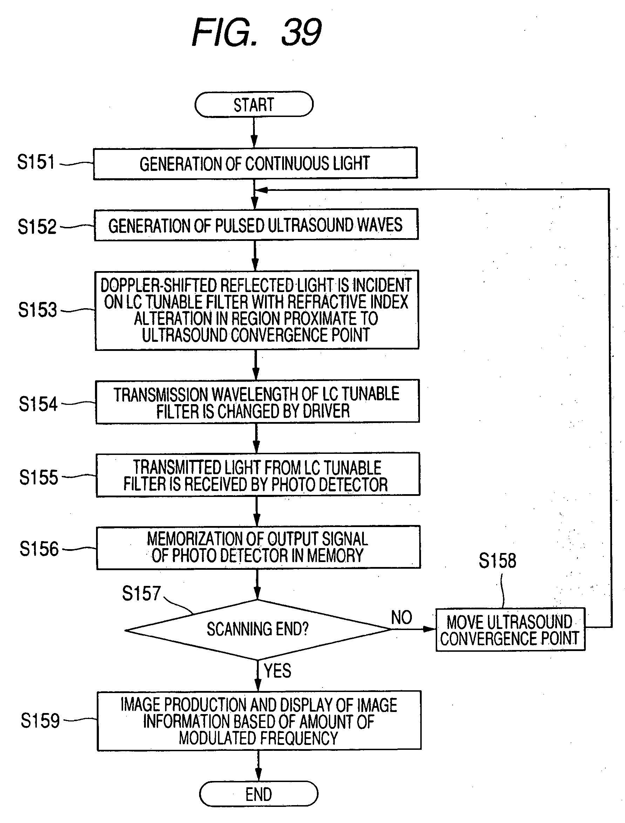

A description will now be given of the operation of the eighth embodiment with reference to Fig. 39.

At initial step S151, the light source 103b generates the continuous light. The generated light is radiated to the biological tissue 107 to be examined. As shown in following step S152, the pulse generator 121 generates the pulse, and the pulsed ultrasound wave driving signal generated by the power amplifier 122 is supplied to the ultrasound transducer 102a. The ultrasound transducer 102a generates the pulsed ultrasound wave on receiving the pulsed ultrasound wave driving signal.

The pulsed ultrasound wave is focused by the acoustic lens 116 and then radiated to the biological tissue 107.

In step S153, the light that has reached the region proximal to the convergence point of the ultrasound wave is Doppler-shifted due to changes in the refractive index responding to arrival of the pulsed ultrasound wave at the region. The Doppler-shifted reflected light is inputted into the liquid crystal tunable filter 142.

In step S154, on the time of inputting the reflected light after the Doppler-shift into the liquid crystal tunable filter 142, the driver 143 changes the penetrating wavelength of the liquid crystal tunable filter 142. The driver 143 is controlled by the control circuit 146 so that the penetrating wavelength in the liquid crystal tunable filter 142 is changed during the period of reaching the pulsed ultrasound wave to the convergence point F.

In step 5155, the penetrated light through the liquid crystal tunable filter 142 goes to the photo detector 104a.

In the following step S156, after receiving the light, the photo detector 104a converts it into the electric signal of the Doppler-shift. The electric signal is then stored into the memory device 158m as optical imaging information together with its scanning position information. In this case, the information regarding the penetrating wavelength of the liquid crystal tunable filter 142 is also stored into the memory device 158m. It is thereby possible to calculate the Doppler-shift amount $\Delta f$ and the change $\Delta n$ of the refraction index.

Next, in step S157, the control circuit 146 judges whether or not the above scanning process is completed. When the judgment result indicates not completion, the control circuit 146 shifts the convergence point of the ultrasound wave as shown in step S158. That is, the control circuit 146 controls the operation of the scanning unit 149 and shifts the unit 111. The operation flow is then returned to step S152 in order to repeat a series of the above steps.

When the scanning for a specified range is completed, the operation flow is forwarded from step S157 to step S159. In step S159, because the image processing for a one frame is completed, the image information of the one frame is transmitted to the output signal display device 110. The image information in relation to the change Δn In the refractive index is displayed as the characteristic information of the object on the output signal display device 110.

According to the eighth example, because both the ultrasound wave and the light are coaxially radiated and the Doppler-shift light is then received in the same coaxial light path, this configuration enables to reduce the entire size of the apparatus.

Although the eighth example, uses the optical spectrum means, it is allowed to use a spectrum analyzer and the like so as to calculate the Doppler-shift amount Δf and the change Δn of the refractive index (as the change amount of the real part of the refractive index) based on the interference signal provided from the photo detector 104a after performing the interference with the reference light.

**(Modification of the eighth example)**

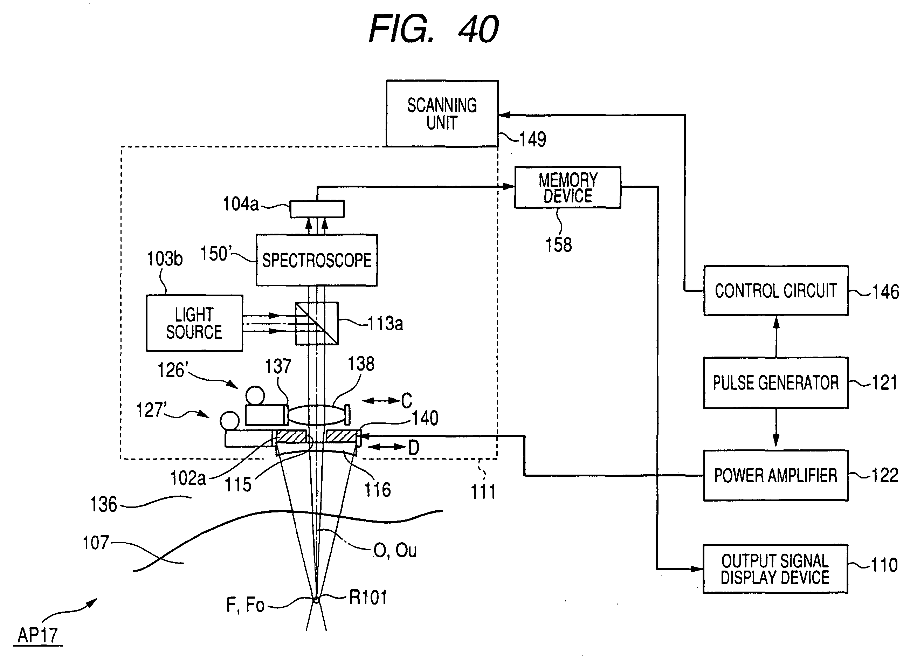

Fig. 40 shows an optical imaging apparatus AP17 according to the modification example of the eighth example. The modification example uses a spectrometer 150' instead of the liquid crystal tunable filter 142 shown in Fig. 38. The photo detector 104a inputs the light output from the spectrometer 150' after performing the wavelength spectrum, and then converted it into the electric signals.

The output signal from the photo detector 104a is stored into the memory device 158m together with its wavelength spectrum information by the spectrometer 150' and the information regarding the scanning position.

Other components are the same of those of the eighth example shown in Fig. 38. This modification example has the feature of reducing the entire size of the apparatus like the eighth example.

**(Ninth example)**

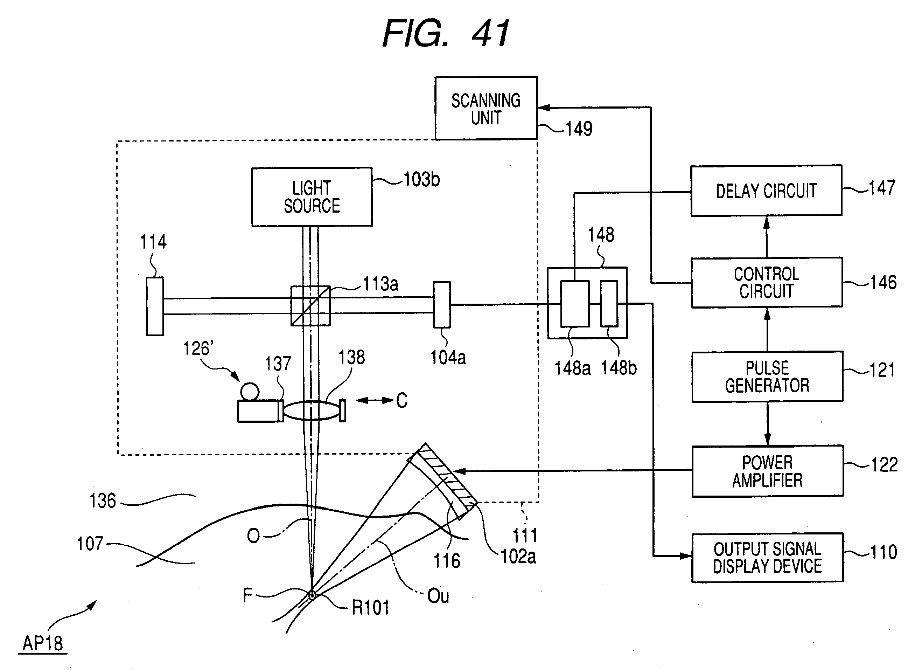

Referring to Figs. 41 - 42, a ninth example will now be described.

Fig. 41 shows an optical imaging apparatus AP18 according to the ninth example. In the optical imaging apparatus AP18, the ultrasound transmission axis Ou of the ultrasound transducer 102a is set to a different direction (for example, the ultrasound transmission axis Ou is set to the direction of the angle 6 to the optical axis O) from the direction of the optical axis O in the optical imaging apparatus AP16 shown in Fig. 38.

The ninth example has the configuration in which the light provided from the light source 103b is divided into the reference light to be transmitted to the reference mirror 114 and the observation light to be transmitted to the biological tissue 107 as the object. Then, the Doppler-shift light returned or reflected from the biological tissue 107 interferes with the reference light. The ninth example also uses the continuous light.

The control circuit 146 in the ninth example controls the detection time by a signal processing device 148 through a delay circuit 147. This signal processing device 148 is composed of a spectrum analyzer 148a and a component block 148b which is a part of a PC such as an A/D converter, a CPU, and a memory. The spectrum analyzer 148a inputs the output signal transferred from the photo detector 104a. The information stored in the component block 148b is output to the output signal display device 110.

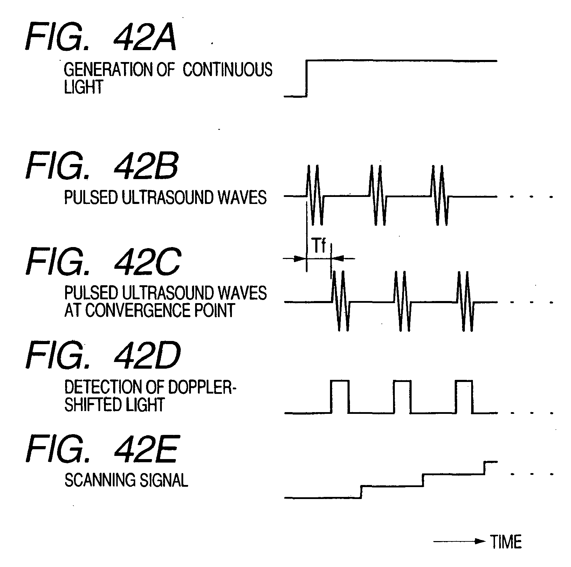

The configuration of the ninth example is equipped with only the lens supporter 126' (not with the transducer supporter 127' shown in Fig. 32). Fig. 42 shows a timing chart according to the ninth. example. As shown in Fig. 42(A), the light source 103b generates the continuous light.

As shown in Fig. 42(B), the ultrasound transducer 102a generates the pulsed ultrasound wave at specified intervals. As shown in Fig. 42(C), this pulsed ultrasound wave reaches the convergence point F after the time elapse Tf, for example, counted from the generation time of the pulsed ultrasound wave by the ultrasound transducer 102a.

The control circuit 146 supplies a gate pulse to the photo detector 104a in order that the photo detector 104a detects the Doppler-shift light at the timing delayed by the time TF counted from the generation of the pulsed ultrasound wave, for example. Fig. 42(D) shows this control operation designated by the peaks at which the Doppler-shift light is detected.

In case of continuously outputting the detected light signal, the optical imaging apparatus AP18 controls the component block 148b composed of the A/D converter, the CPU and the memory so that the photo detector 104a can input the output signal from the photo detector 104a at the above timing.

After the detection of the Doppler-shift light, the focus point F Is shifted according to the scanning signal. As described above, it is possible to detect the Doppler-shift amount Δf for the target region to be examined and to obtain the image information of the change Δn of the refractive index.

**(Modifications of the ninth example)**

<First modification>

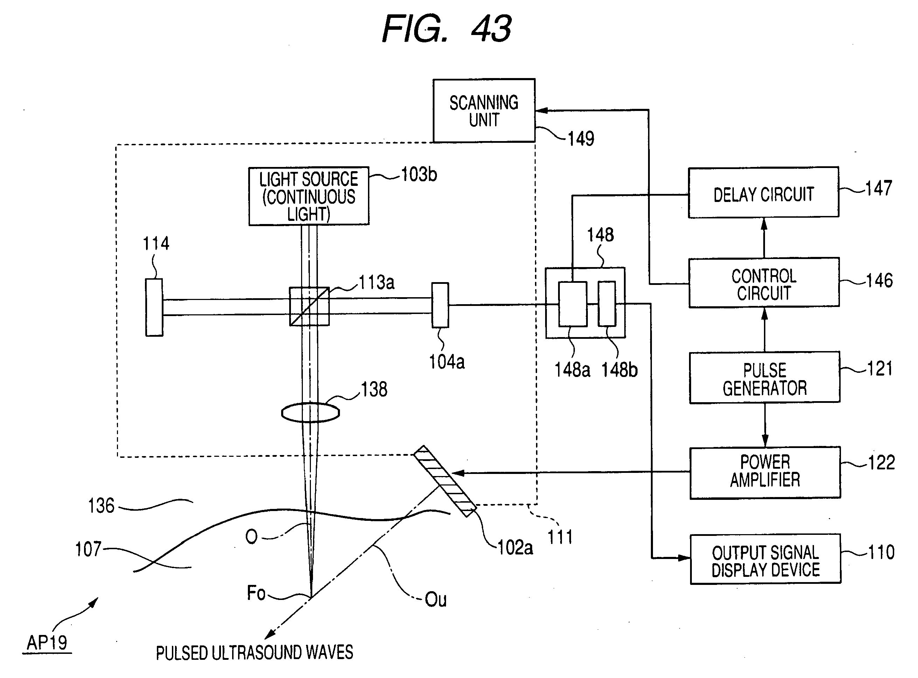

Fig. 43 shows an optical imaging apparatus AP19 according to the first modification example of the ninth example. The optical imaging apparatus AP18 has the configuration of not focusing the ultrasound wave in the optical imaging apparatus AP18 shown in Fig. 41. That is, the optical imaging apparatus AP19 is not equipped with the acoustic lens 116 capable of focusing the ultrasound wave in the configuration of the optical imaging apparatus AP18 shown in Fig. 41. The optical imaging apparatus AP19 is not equipped with the lens supporter 126'.

Because of not focusing the ultrasound wave in this modification example, it is possible to make the optical imaging apparatus AP19 with a simple configuration. In case of not focusing the ultrasound wave, it is acceptable to fix the ultrasound transducer 102a. That is, because of spreading the radiated ultrasound wave, it is possible to take a configuration of scanning the light to be focused by the collimator lens 138.

<Second modification>

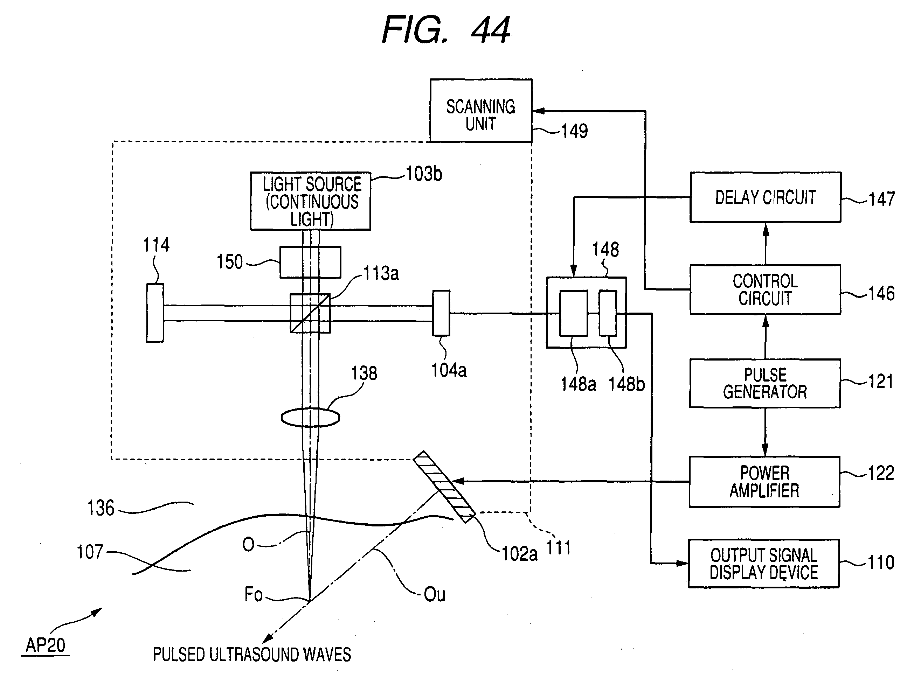

Fig. 44 shows an optical imaging apparatus AP20 according to the second modification example of the ninth example. The optical imaging apparatus AP20 is equipped with a beam expander 150 placed between the light source 103b and the half mirror 113a in addition to the configuration of the optical imaging apparatus AP19 shown in Fig. 43. This configuration of the second modification example enables to increase the detection intensity of the modulated light because of increasing the radius of the beam of light.

<Third modification>

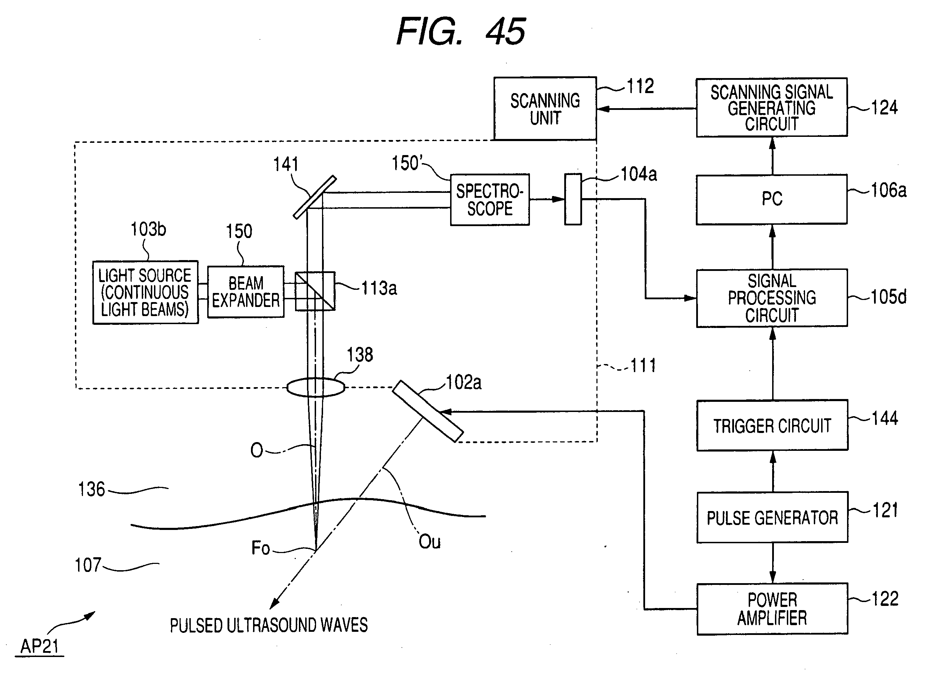

Fig. 45 shows an optical imaging apparatus AP21 according to the second modification example of the ninth example. The optical imaging apparatus AP21 is not equipped with the interferometer which is mounted on the optical imaging apparatus AP19 shown in Fig. 43.

The third modification example uses the configuration similar to the configuration shown in Fig. 33, for example.

In the optical imaging apparatus AP21, the reflected light by the half mirror 113a in the apparatus shown in Fig. 33 is focused by the collimator lens 138, and then radiated to the biological tissue 107 as the object.

The light reflected by the biological tissue 107 and penetrated through the half mirror 113a is reflected by the mirror 141, and then radiated to the spectrometer 150'. The photo detector 104a detects the light from the spectrometer 150'.

In the third modification example, the pulsed ultrasound wave driving signal is applied to the ultrasound transducer 102a. In this case, because the acoustic lens 116 is not mounted and the ultrasound transducer 102a radiates the pulsed ultrasound wave of not focused to the biological tissue 107.

In the configuration of the third modification example, the diameter of beam of the light provided from the light source 103b is increased by the beam expander 150, and the expanded light is then radiated to the half-mirror 113a. However, it is acceptable to eliminate the beam expander 150 from the configuration of the third modification example shown in Fig. 45.

This third modification example has the effect of reducing the size of the apparatus. In order to further reduce the entire size of the apparatus, it is possible to remove the collimator lens 138 from the configuration shown in Fig. 45.

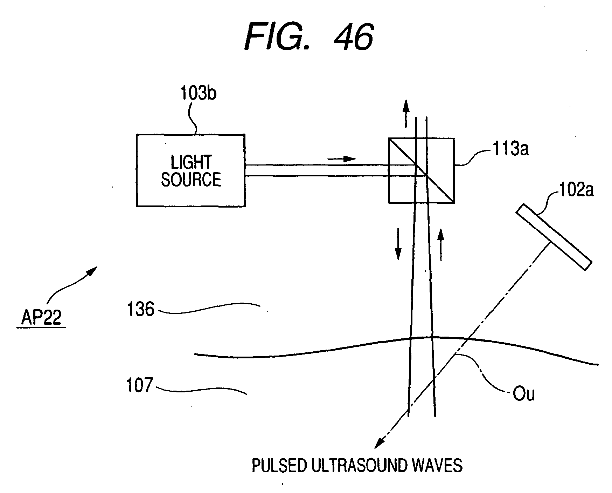

Fig. 46 shows a configuration of being not equipped with the collimator lens 138, namely, shows a part of an optical imaging apparatus AP22 configured to radiate the light, which has not been focused, to the biological tissue 107.

It is possible to use either the pulsed ultrasound wave or the continuous ultrasound wave in the configuration described above.

As the photo detector 104a mounted on each example described above, it is acceptable to use a point detector, a one-dimensional line sensor, or a two dimensional detector such as a CCD, and also acceptable to detect the Doppler-shift amount based on Fourier transform.

**(Tenth example)**

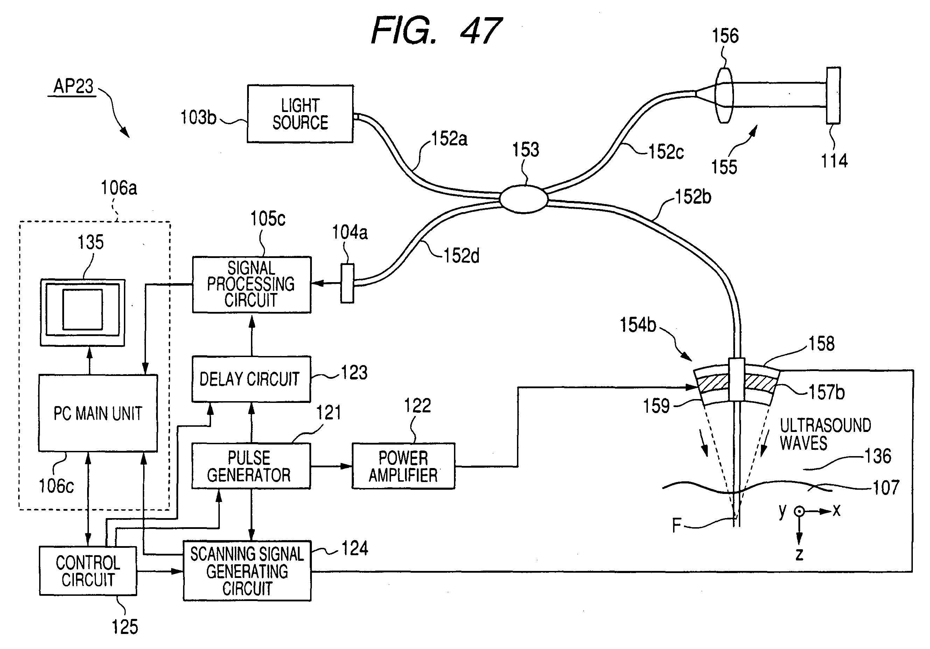

Referring to Figs. 47 - 49, a tenth example will now be described.

Fig. 47 shows an optical imaging apparatus AP23 capable of detecting the Doppler-shift amount according to the tenth example.

This optical imaging apparatus AP23 is equipped with the light source 103b which generates a laser light to be radiated to the biological tissue 107.

The laser light from the light source 103b is radiated to one end of an optical fiber 152a through which the laser light is transmitted and divided into two lights by a photo coupler 153 mounted on the way of the optical fiber 152a. One divided light is transmitted to a light radiation and receptor 154b through an optical fiber 152b, and the other divided light is transmitted to a reference light generator 155 through an optical fiber 152c. The light through the optical fiber 152b is radiated to the biological tissue 107 through water 136 capable of transmitting ultrasound waves. The Doppler-shift light reflected by the biological tissue 107 is radiated as the observation light into the optical fiber 152b, and then interferes with the reference light. The photo detector 104a placed at the end surface of the optical fiber 152d receives this interfered light, and converts the received light into electric signals.

The reference light generator 155 generates a parallel light flux from the light output from the end surface of the optical fiber 152c by the collimator lens 56, and radiates the parallel light flux to the fixed reference mirror 114. The light reflected by the reference mirror 114 is radiated again Into the end surface of the optical fiber 152c through the collimator lens 156.

The light radiation and receptor 154b is composed of an ultrasound transducer 157b, a scanning device 158, and an acoustic lens 159 placed near the end surface of the optical fiber 152b capable of radiating (or emitting) and receiving the light.

For example, the ultrasound transducer 157b has an ultrasound wave transmission surface of a concave shape. This ultrasound transducer 157b generates the pulsed ultrasound wave on receiving the pulsed ultrasound wave driving signal which is amplified by the power amplifier 122 and transferred from the pulse generator 121. The pulsed ultrasound wave generated by the ultrasound transducer 157b is focused and then radiated to the biological tissue 107.

On receiving the scanning signal provided from the scanning signal generating circuit 124, the scanning device 158 scans in a two- or three-dimensional form the light radiation and receptor 154b in synchronization with the received scanning signal. For example, the scanning device 158 scans the biological tissue 107 in its depth direction, namely, z-axis direction and x-axis direction which is perpendicular to the z-axis direction, for example.

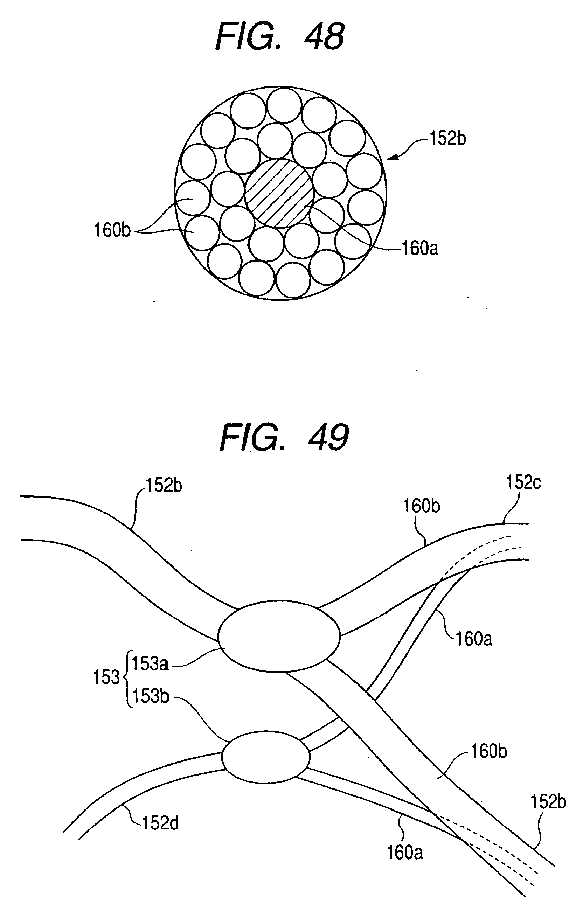

Fig. 48 shows an enlarged view of the end surface of the optical fiber 152b. The optical fiber 152b in the tenth example is composed of a first optical fiber part 160a and a second optical fiber part 160b. The first optical fiber part 160a is placed at the center area of the optical fiber 152b composed of one or more optical fibers. The second optical fiber part 160b is composed of plural optical fibers, namely, an optical fiber bundle placed around the first optical fiber part 160a.

As shown in the enlarged view of the optical coupler 153 shown in Fig. 49, the light from the light source 103b is radiated to the optical fiber 152b, and divided into two lights by the first coupler which forms the photo coupler 153. The divided lights are transmitted through the second optical fiber part 160b in the optical fiber 152b and the second optical fiber part 160b in the optical fiber 152c, respectively.

The light transmitted from the end surface of the second optical fiber part 160b is radiated to the biological tissue 107. The first optical fiber part 160a receives the Doppler-shift light from the biological tissue 107.

The Doppler-shift light received by the first optical fiber part 160a is mixed with the reference light transmitted through the first optical fiber part 160a in order to generate the Doppler-shift interfered light. This interference light is transmitted through the optical fiber 152b, and then received by the photo detector 104a.

The light signal detected by the photo detector 104a is inputted to the signal processing circuit 105c such as a spectrum analyzer in order to detect the Doppler-shift amount $\Delta f$.

The signal processing circuit 105c extracts the signal in synchronization with the timing when the delay circuit 123 delays the pulse signal from the pulse generator 121. The signal detected by the signal processing circuit 105c is inputted to the A/D converter in a main unit 106c of the PC 106a. This PC main unit 106c is equipped with a CPU, a memory, an A/D converter therein. The image display signal output from the PC main unit 106c of the PC 106a is transferred to a monitor 135. The image regarding the optical imaging information is displayed on the monitor 135.

The A/D converter in the PC main unit 106c receives the scanning signal from the scanning signal generating circuit 124. On receiving the scanning signal by the A/D converter, the PC 106a calculates the scanning position information.

The controller 125 controls each operation of the pulse generator 121, the scanning signal generating circuit 124 and the delay circuit 123. The controller 125 controls the data transmission of control signals from/to the CPU In the PC main unit 106c. The controller 125 controls the operation of the pulse generator 121 and the like. The PC 106a further controls the pulse generator 121 and the like through the controller 125 or without using the controller 125.

This configuration of the tenth example enables to obtain the optical imaging information corresponding to, at least, the real part of the complex refractive index by scanning the area near the front end part of the optical fiber 152b, like the case of the fifth example. The configuration of the tenth example can be equipped with a small-sized device of a small driving ability as the scanning device 158. The optical imaging apparatus AP23 according to the tenth example has another same effect of the fifth example.

**(Modifications of the tenth example)**

<First modification>

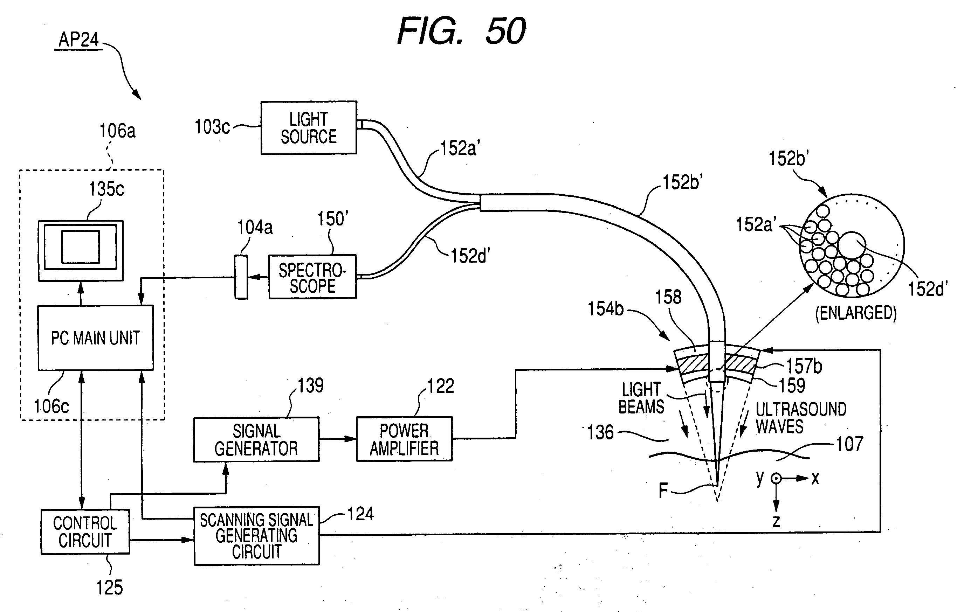

Fig. 50 shows the configuration of an optical imaging apparatus AP24 according to the first modification example of the tenth. example. This optical imaging apparatus AP24 is not equipped with the interferometer. That is, the optical imaging apparatus AP24 has the configuration in which the optical coupler 153 capable of obtaining the light spectrum and of performing the light coupling is eliminated from the configuration of the optical imaging apparatus AP23 shown in Fig. 47.

The configuration of the first modification example uses the light source 103c capable of generating the continuous light and the continuous ultrasound wave. Like the case shown in Fig. 47, it is acceptable to use the continuous light and the pulsed ultrasound wave.

The light from the light source 103c is transmitted to the front end surface of the optical fiber bundle 152a', and the transmitted light from the front end surface of the light radiation and preceptor 154b is radiated to the biological tissue 107 as the object.

An optical fiber bundle part 152b' integrated with the optical fiber 152d' is placed on the way of the optical fiber bundle 152a'.

The optical fiber bundle part 152b' has a configuration shown in Fig. 50. In a concrete example, as shown in an enlarged end-surface view of Fig. 50, an optical fiber 152d' for reception is placed at the center position of the optical fiber bundle part 152b', and the optical fibers forming the optical fiber bundle 152a' are placed in a concentric configuration around the optical fiber 152d'.

An ultrasound transducer 157b of a concave shape, the scanning device 158, and the acoustic lens 159 are placed at the outside of the area near the front end of the optical fiber bundle 152a'.

The Doppler-shift light from the biological tissue 107 is received by the optical fiber 152d' placed at the center position of the optical fiber bundle part 152b', and then transmitted through it to the base end surface of the optical fiber bundle part 152b'. For example, the spectrometer 150' is placed at the base end surface of the optical fiber bundle part 152b'. The spectrometer 150' optically extracts light having the Doppler-shift frequency components. The extracted light is inputted to the photo detector 104a so as to convert it to the electric signals. The electric signal is inputted to the main unit 106c of the PC 106a, and then stored into the memory therein together with the scanning information and the frequency spectrum information obtained by the spectrometer 150'.

Because this modification example does not perform the electric signal spectrum and extraction of the Doppler-shift frequency component, which is performed in the configuration shown in Fig. 47, the output signal from the photo detector 104a shown in Fig. 47 is directly inputted to the PC main unit 106c of the PC 106a without passing through the signal processing circuit 105c.

The ultrasound transducer 157b is driven by the ultrasound wave driving signal transferred through the signal generator 139 and the power amplifier 122, like the configuration shown in Fig. 25.

Other components of the first modification example have the same of those of the configuration shown in Fig. 47. The first modification example has the same effect of the configuration shown in Fig. 47. Because of eliminating the photo coupler 153 and the reference light generator 155 and the like from the configuration shown in Fig. 47, it is possible to make the apparatus of the first modification example of a compact size.

It is also possible to use another optical spectrometer (or a wavelength spectrum and extraction means) such as a liquid crystal tunable filter, an acoustic optical element, a diffraction grating, as a concrete example, instead of the spectrometer 150' mounted on the first modification example.

<Second modification>

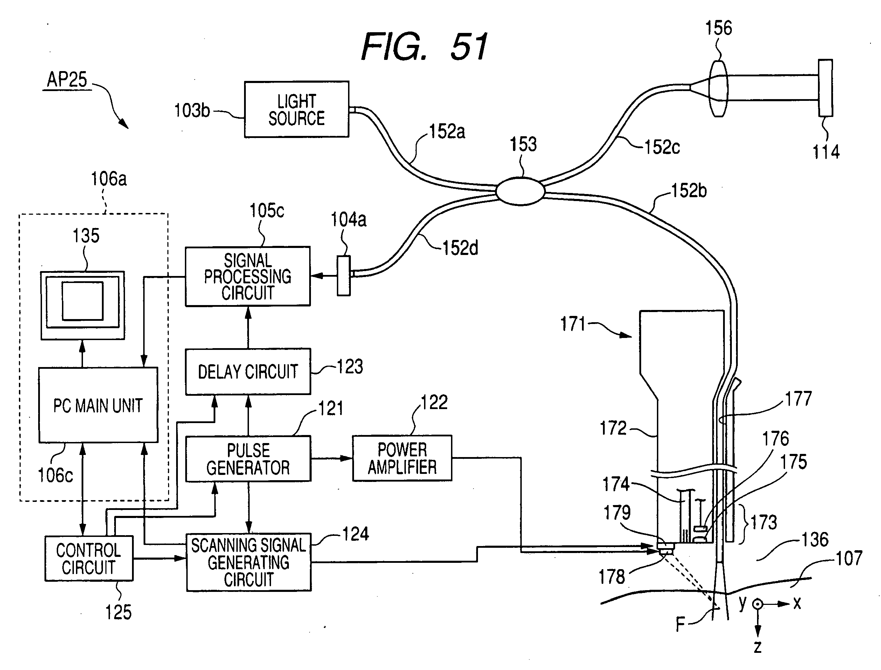

Fig. 51 shows the configuration of an endoscope apparatus AP25 equipped with the optical imaging apparatus according to the second modification example of the tenth example. An endoscope 171 in the second modification example is equipped with the configuration corresponding to the light radiation and receptor 154b shown in the tenth example.

The endoscope 171 has a light window and an observation window placed at a hard front end part 173 formed at the front end of an insertion part 172. The observation light is output through the light window and the operator observes the biological tissue 107 through the observation window.

A front end of a light guide 174 is mounted on the light window, and the light is output through the front end surface of the light guide 174. The observation light from an endoscope light source (not shown) is inputted to the other end surface (not shown) of the light guide.

An object lens 175 is attached to the observation window. A CCD 176 as an image pickup device is placed at the position of image formation.

The CCD 176 is electrically connected to a signal processor such as a video processor. This signal processor performs the signal processing for the image signal picked-up by the CCD 176 in order to generate the image signal. The signal processor outputs the generated image signal to a monitor (not shown).

A channel 177 is formed along the longitudinal direction of the insertion part 172 of the endoscope 171 in order to insert instruments and the like. Through the channel 177, the optical fiber 152b is inserted.

A scanning device 179 is mounted on the front surface of the front end part 173 in the endoscope 171 of the second modification example. The ultrasound transducer 178 is mounted on the driving surface of the scanning device 179.

On applying the scanning signal generated by the scanning signal generating circuit 124 to the scanning device 179, the scanning operation of the ultrasound transducer 178 is executed two- or three-dimensionally.

The ultrasound transducer 178 in the second modification example is composed of an ultrasound transducer of a scanning electron type, for example. This ultrasound transducer is composed of, for example, plural transducer elements placed along x direction in the configuration shown in Fig. 51, and outputs the ultrasound wave by driving the transducer elements by controlling the delay time through the delay elements (not shown) so that the ultrasound wave is focused at the convergence point F.

As shown in Fig. 51, the convergence point F of the ultrasound wave is set in the area to which the output light from the front end surface of the optical fiber 152b is radiated. In this situation, the scanning device 179 is performed responsively to the scanning signal, so that the convergence point F is scanned.

Other components of the second modification example of the tenth embodiment are the same of those of the configuration shown in Fig. 47, the explanation of the same components is omitted here.

According to the second modification example, it is possible to examine the biological tissue 107 in the object by using the endoscope 171. The optical fiber 152b is inserted into the channel 177 shown in Fig. 51 for the detailed examination to a lesion region, and the front end surface of the optical fiber 152b is faced to the target region to be observed.

The pulsed ultrasound wave driving signal is generated through the power amplifier 121 by the pulse generator 121, and the scanning signal is also generated in order to obtain the two-dimensional imaging information to the peripheral region of the target region to be observed and display the imaging information on the monitor 135.

Because the configuration of the second modification example of the tenth example is equipped with the light radiation part to radiate the light to the endoscope 171, the ultrasound wave radiation means for radiating and focusing the ultrasound wave, and the scanning means of scanning the focused region of the ultrasound wave in two dimensions, it is possible to obtain the optical imaging Information for the biological tissue 107 as a desired site.

Accordingly, it is possible to obtain and then display on the monitor 135 the optical imaging information of a lesion region when the operator needs further detailed examination for the specific region such as the lesion region while performing the optical examination using an endoscope for the inside of the cavity of an object. This manner according to the second modification example described above enables to obtain the useful information for examining the lesion region with high accuracy when compared with the manner of using only the endoscope.

<Third modification>

The third modification example is a modification of the second modification example. As will be explained below, it is acceptable to further mount the light radiation and receptor 154b, which has been disclosed in the eighth example, on the front end aperture part of the channel 177. The light radiation and receptor 154b has the functions of focusing, radiating the ultrasound wave, and scanning.

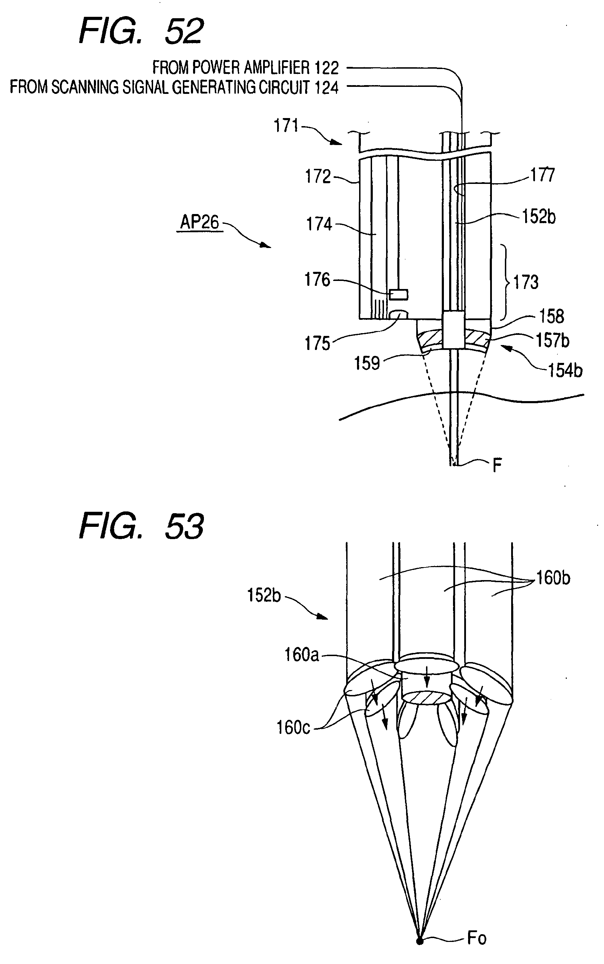

Fig. 52 shows a configuration of the area near the front end part of the endoscope 171 in an endoscope apparatus AP26 according to the third modification example of the tenth example. In the third modification example, the light radiation and receptor 154b shown in Fig. 47 is detachably mounted on the front end part of the channel 177. This configuration enables to obtain the optical imaging information by attaching the light radiation and receptor 154b to the channel of an endoscope which is commercially available. It is also acceptable to apply the optical imaging apparatus having the configuration shown in Fig. 50 to an endoscope. This case can also obtain the same effect.

<Fourth modification>

Fig. 53 shows an example of the configuration of the optical fiber 152b according to the fourth modification example shown in Fig. 53. As shown in Fig. 48, this optical fiber 152b is composed of a first optical fiber part 160a placed at the center position of the optical fiber 152b and a second optical fiber part 160b composed of plural optical fibers.

In the optical fiber 152b shown in Fig. 53, the end surface of the second optical fiber part 160b is so formed that it has an concave surface such as a paraboloid of symmetry of rotation around the axis of the first optical fiber part 160a located at the center position of the optical fiber 152b. Further, the collimator lens (a condenser lens or a convex lens) capable of collecting light is mounted on the end surface of the optical fiber 152b.

The optical fiber 152b Is capable of focusing the light output from the end surface of each second optical fiber part 160b to the convergence point Fo. This configuration enables to obtain the optical imaging information with a superior S/N ratio.

In each embodiment and each modification example previously described, It is possible to use either the pulsed ultrasound wave or the continuous ultrasound wave, and to use a point detector such as a photodiode and a photomultiplier, a one-dimensional line sensor, or a two dimensional detector such as a CCD as the photo detector 104a.

In each embodiment and each modification example described above, it is acceptable to detect the Doppler-shift amount based of the manner using Fourier transform.

**(Eleventh example)**

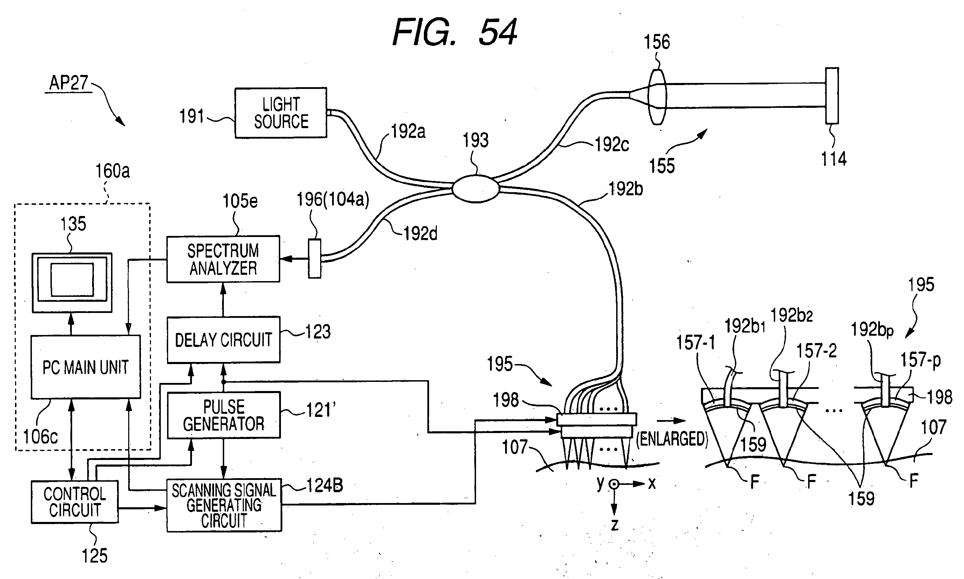

Referring to Fig. 54, an eleventh example will now be described.

Next, a description will be given of the eleventh example with reference to Fig. 54.

Fig. 54 shows an optical imaging apparatus AP27 according to the eleventh example. The eleventh example uses an ultrasound wave radiation-light radiation-detection array 195 composed of a plurality of ultrasound wave radiation-light radiation-detection parts which are arranged in line. Each ultrasound wave radiation-light radiation-detection part has a configuration in which an ultrasound wave radiation part, a light radiation part and a light detection part are integrated or built in a single unit. This configuration enables to obtain a one-dimensional optical imaging information by performing the scan using one ultrasound wave radiation-light radiation-detection array 195. This configuration can obtain the one dimensional optical imaging information in a short time.

This configuration further enables to obtain second-dimensional optical imaging information by performing the scanning using the ultrasound wave radiation-light radiation-detection array 195 in one-dimension.

As will be explained later, the use of an ultrasound wave radiation-light radiation-detection array 195B composed of a plurality of the ultrasound wave radiation-light radiation-detection parts which are arranged in two-dimensions. This configuration enables to get the two dimensional optical imaging information without performing the scanning of the ultrasound transducer and the like.

The eleventh embodiment has the configuration of calculating the scattering information by extracting the Doppler-shift amount $\Delta f$.

The light of visible or near infrared wavelength supplied by a light source 191 is radiated to each optical fiber forming an optical fiber array 192a composed of a plurality of optical fibers (for example, "p" optical fibers). The light radiated each optical fiber is divided into two lights by an optical coupler 193. One of the two lights divided is transmitted to an optical fiber in an optical fiber array 192c, and the other is transmitted to optical fibers 192bi (i = 1 to p) in an optical fiber array 192b.

The reference light is generated based on the light transmitted through the optical fiber array 192c by the reference light generator 155, similar to the case shown in Fig. 47. This light is returned as the reference light to the optical coupler 193.

The light transmitted through the optical fiber 192bi in the optical fiber array 192b is radiated to the biological tissue 107 to be examined through an aperture formed in a ultrasound transducer 157-i in the ultrasound wave radiation-light radiation-detection part (forming the ultrasound wave radiation-light radiation-detection array 195).

As shown in the right bottom part of the enlarged view shown in Fig. 54, the ultrasound wave radiation-light radiation-detection array 195 is composed of a plurality of the ultrasound wave radiation-light radiation-detection parts are arranged in $\times$ direction, for example.

The ultrasound transducer 157-i which is a component of each ultrasound wave radiation-light radiation-detection part focuses the ultrasound wave at the convergence point F. The radiation-detection part placed at the end surface of the optical fiber 192bi forming the optical fiber array 192b radiates the light to the convergence point F and receives the Doppler-shift light reflected from the vicinity region R101 near the convergence point F.

The light received by the optical fiber 192bi in the optical fiber array 192b is mixed, as the observation light, with the reference light from the optical fiber array 192c by the optical coupler 193 in order to generate the Doppler-shift interfered light.

Each interfered light is transmitted through the optical fiber in the optical fiber array 192d. Each photo detector 104a forming the light detection array 196 receives the interfered light output from the end surface of the transmitted through this optical fiber, and performs the photoelectric conversion of converting the received one to the electric signal.

The output signal obtained by the photoelectric conversion of each photo detector 104a forming the light detection array 196 is inputted into a spectrum analyzer 105e of plural channels, for example.

The spectrum analyzer 105e extracts the interfered light of the Doppler-shift amount $\Delta f$ output from each photo detector 104a simultaneously.

The plural interfered signals output from this spectrum analyzer 105e are stored into a memory and the like through input channels in the PC106a.

The scanning signal generating circuit 124B in the eleventh example drives the scanning device 198 equipped with the ultrasound wave radiation-light radiation-detection array 195 so that it scans in y direction, for example, in order to obtain the two dimensional optical imaging information. The pulse generator 121' in the eleventh example has the function of both of the pulse generator 121 and the power amplifier 122 shown in Fig. 47.

As previously explained with reference to Fig. 21, the two dimensional (2D) optical imaging information is obtained by scanning the ultrasound wave radiation-light radiation-detection array 195 in y direction, for example.

The one-dimensional (1D) optical imaging information in x direction is obtained by the ultrasound wave radiation-light radiation-detection parts arranged in line, and the two dimensional optical imaging information can be obtained by scanning the ultrasound wave radiation-light radiation-detection part in y direction.

In order to obtain the three-dimensional optical imaging information, it is acceptable that the scanning signal generating circuit 124B drives the ultrasound wave radiation-light radiation-detection array 195 in order to perform the two dimensional scanning in x and y directions

The configuration of the eleventh example enables to obtain the two or three dimensional optical imaging information at high speed.

Because the resolution of the optical Imaging information in x direction depends on the number of the optical fibers forming the optical fiber array 192b, its resolution can be drastically increased by performing the scanning in x direction.

**(Modifications of the eleventh example)**

<First modification>

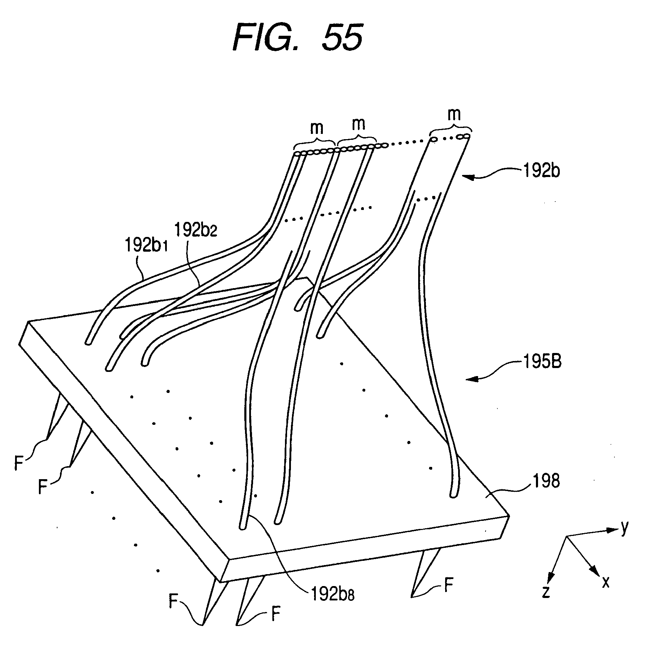

Fig. 55 shows a schematic configuration of an ultrasound wave radiation-light radiation-detection array 195B according to the first modification example.

This modification example uses the optical fiber arrays 192a and 192b composed of a plurality of optical fibers whose number is larger (integral multiples of the case shown in Fig. 54, for example) than the number of those in the optical imaging apparatus AP27 shown in Fig. 54. This configuration enables to increase the number of the ultrasound wave radiation-light radiation-detection parts.

The modification example discloses the ultrasound wave radiation-light radiation-detection array 195B which is so formed that the ultrasound wave radiation-light radiation-detection parts at the end surface of the optical fiber array 192b are arranged in two dimensional directions, x and y directions.

As shown in Fig. 55, the optical fiber array 192b is composed of a plurality of the optical fibers arranged in two dimensions on a scanning device 198 where "m" optical-fiber groups (or bands) are arranged in columns and each optical-fiber group is composed of "p" optical fibers 192b1 to 192bp arranged in rows. In other words, as clearly shown in Fig. 55, each optical fiber 192bi is placed at each aperture formed in the scanning device 198 in two dimensions.

The ultrasound transducer (omitted from Fig. 55), which has been shown in Fig. 54, is mounted on the bottom of the scanning device 197. The configuration of the modification example enables to obtain the two dimensional optical imaging Information (see Fig. 23) without scanning the ultrasound wave radiation-light radiation-detection arrays 195B.

In this configuration of the modification example, the PC106a stores the output signals from the spectrum analyzer 105e into the memories whose addresses correspond to the two dimensional arrangement of the ultrasound wave radiation-light radiation-detection parts that forms the ultrasound wave radiation-light radiation-detection array 195.

The configuration of the modification example is capable of obtain the three dimensional optical imaging information, as described previously referring to Fig. 23, by driving the scanning device 198 in z direction based on the scanning signal.

As previously explained in the eighth example, it is possible to perform the scanning in x- and y-directions in order to obtain the optical imaging information of higher resolution. This modification example enables to obtain the two- or three-dimensional optical imaging information at high speed.

It is possible to have a configuration of focusing the light in each embodiment described previously. It is also acceptable for each embodiment and each modification to use either the pulsed ultrasound wave or the continuous ultrasound wave and to detect the Doppler-shift amount based on Fourier transform without using the signal processing circuit such as the spectrum analyzer.

As described above, each configuration of the fifth to eleventh examples and the modification examples thereof radiates the light to a biological tissue as an object while radiating the ultrasound wave to this same area simultaneously, detects the observation light which is Doppler-shift at the same region to which the ultrasound wave is radiated, and detects and images, at least, the optical scattering information corresponding to the real part of the complex refractive index. It is therefore possible to get the information regarding a significant correlation with the structural change of the lesion of the biological tissue and to effectively use such information in the examination for the lesion of the biological tissue.

**(Twelfth example)**

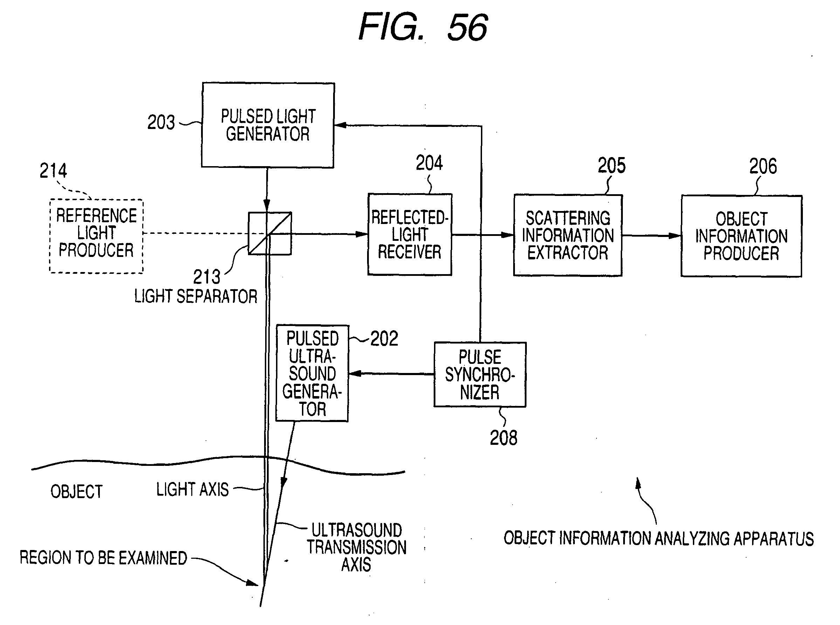

Referring to Figs. 56 - 60, a twelfth example will now be described.

Fig. 56 is a block diagram showing the basic structure of an object information analyzing apparatus according to another example.

As shown in Fig. 56, the object information analyzing apparatus includes a pulsed ultrasound generator 202, and a pulsed light generator 203. The pulsed ultrasound generator 202 is capable of generating an ultrasound wave such that It transmits through an object to be diagnosed along a predetermined ultrasound wave transmission axis. The pulsed light generator 203 operates to generate a pulsed light such that the pulsed light reaches a region of interest in the object at the same timing at which the pulsed ultrasound wave generated by the pulsed ultrasound generator 202 reaches this region of interest. A light axis of the pulsed light is set nearly coaxial with the ultrasound wave transmission axis.

The object information analyzing apparatus further includes a reflected-light receiver 204, and a scattering information extractor 205. The reflected-light receiver 204 is disposed on the side of the pulsed light generator 203 (or the pulsed ultrasound generator 202) to be able to receive the pulsed light generated by the pulsed light generator 203 and reflected at the region of interest. The scattering information extractor 205 detects frequency information and phase information of a light reception signal outputted from the reflected-light receiver 204 in order to extract light scattering information equivalent at least to a real part of a complex refractive index of the region of interest.

The object information analyzing apparatus further includes an object information producer 206 that produces characteristic information of the region of interest of the object on the basis of the scattering information received from the scattering information extractor 205.

As shown in Fig. 56, the reflected-light receiver 204 receives the pulsed light reflected at the region of interest through a light separator (or light dividing section) 213.

The solid line in Fig. 56 shows a typical case where the pulsed light that has been Doppler-shifted at the region of interest is received to extract the scattering information.

On the other hand, the dotted line in Fig. 56 shows another typical case where an interfered pulsed light interfered with a reference light generated by a reference light producer 214 is received to extract the phase information. It should be noted that in the case of receiving the Doppler-shifted pulsed light, it may be interfered with the reference light as well,

The object information analyzing apparatus further includes a pulse synchronizer 208 operating to synchronize the generation of the pulsed ultrasound wave and the generation of the pulsed light such that the pulse ultrasound wave and the pulsed light reaches the region of interest at the same timing. The pulse synchronizer 208 may be so configured to determine the timing to generate (or emit) one of the pulsed light and the pulsed ultrasound wave on the basis of the timing at which the other of them has been generated.

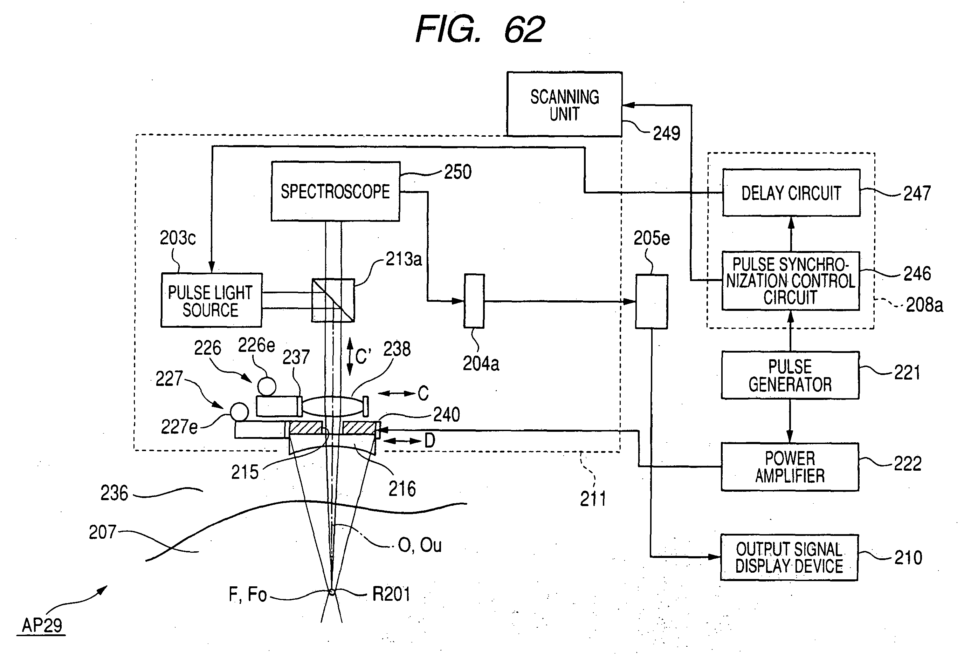

The configuration of Fig. 56 is directed to a case where the frequency Information is electrically detected to extract the scattering information from the received light signal. However, the frequency information may be optically detected from the Doppler-shifted light by use of a spectroscope as described later in a modification with reference to Fig. 62. In this case, the spectroscope as an optical frequency-information detecting section detects, from the Doppler-shifted light, the frequency information of this Doppler-shifted light (the frequency component that has been Doppler shifted) before it is received by the reflected-light receiver 204.

The object information producer 206 includes an image forming part operating to scan the region of interest of the object two-dimensionally, or three dimensionally, and display the scattering information etc. at each position In an image form.

As explained above, the object Information analyzing apparatus shown In Fig. 56 is configured to apply the pulsed ultrasound wave and the pulsed light to the region of interest of the object in such a state that the pulsed ultrasound wave and the pulsed light are nearly coaxial with each other, and the pulsed light reflected at the region of interest is received by use of the same optical path used for applying the pulsed light to the region of interest.

Accordingly, the object information analyzing apparatus can be made compact in size. Since the pulse synchronizer 208 synchronizes the pulsed ultrasound wave and the pulsed light, it is possible to obtain the scattering information in high resolution.

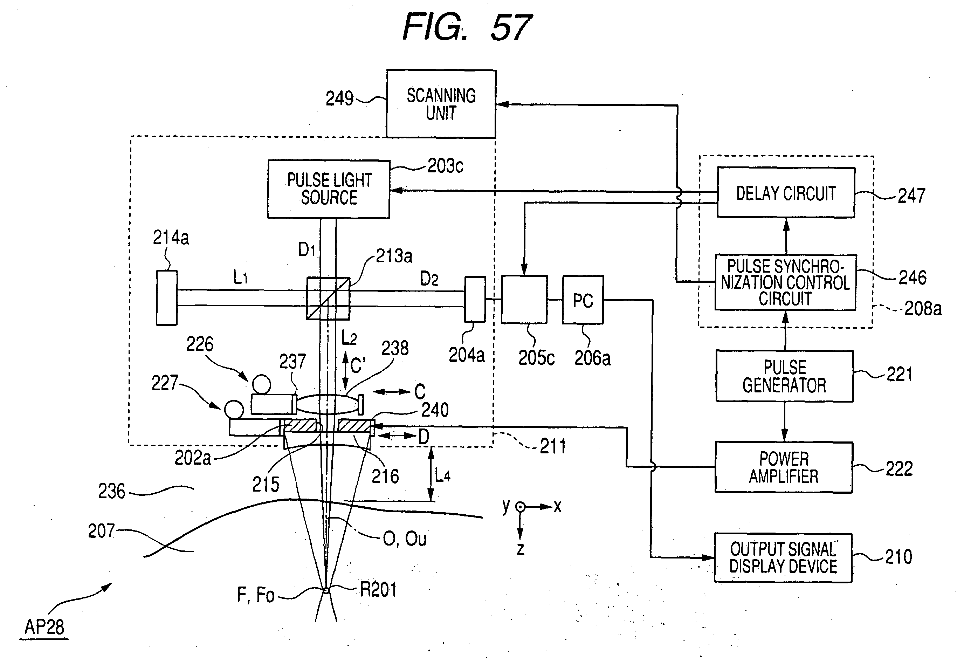

Fig. 57 is a diagram showing a structure of an optical tomographic device AP28 including a pulse light source 203c as the pulsed light generator 203 disposed in a housing unit 211 thereof operating to generate the pulsed light. The pulsed light generated by the pulsed light source 203c enters a half mirror 213a serving as the light separator 213, and is split into two, one of which transmits through the half mirror 213a, and the other of which is reflected at the half mirror 213a.

The pulsed light transmitting through the half mirror 213a is converged by a collimate lens (or a condenser lens) 238 mounted to a lens frame 237, and passes through an opening 215 formed in an ultrasound transducer 202a to be applied to a living tissue 207 as the object.

The ultrasound transducer 202a constituting the pulsed ultrasound generator 202 is mounted to an end face of the housing unit 211 such that an optical axis O of the collimate lens 238 and the ultrasound wave transmission axis are in a coaxial relationship or in nearly a coaxial relationship. The ultrasound transducer 202a is provided with an acoustic lens 216 as an ultrasound wave focusing means.

As shown in Fig. 57, the pulsed light applied to the living tissue 207 is partly in the vicinity region R201 in which a focus point F is located in the direction of the optical axis O. At this time, the pulsed light is Doppler-shifted by the action of the pulsed ultrasound wave arriving at the vicinity region R201, and returns to the side of the collimate lens 238.

The pulsed light returning to the side of the collimate lens 238 is partly reflected at the half mirror 213a, and received by an optical detector 204a serving as the reflected-light receiver 204.

The pulsed light reflected at the half mirror 213a is reflected at a reference mirror 214a serving as the reference light producer 214 disposed on a reference light path so as to be opposed to the half mirror 213a, and enters, as the reference pulsed light, the half mirror 213a again. This reference pulsed light partly transmits through the half mirror 213a, and received by the optical detector 204a in a state of being mixed (interfered) with the Doppler-shifted pulsed light.

Since the optical axis O and the ultrasound wave transmitting axis Ou are set so as to coincide with each other, the optical tomographic device AP28 can be made compact in size.

This embodiment further includes a pulse generator 221 generating a pulse for driving the ultrasound transducer 202a. This pulse is amplified by an amplifier 222, and then applied to the ultrasound transducer 202a as an ultrasound drive pulse.

The pulsed ultrasound wave generated by the ultrasound transducer 202a is transmitted to the side of the living tissue 207 immersed in water while being focused by the acoustic lens 216.

The pulse generated by the pulse generator 221 is also inputted to a pulse synchronization control circuit 208a constituting the pulse synchronizer 208. The pulse synchronization control circuit 208a controls the timing in accordance with which the pulse light source 203c generates the pulsed light by use of a delay circuit 247 capable of variably setting a delay time.

As described later, the pulse synchronization control circuit 208a performs a synchronization control in order that the pulsed light reaches the vicinity region R201 of the focus point F as the region of interest at the same timing at which the pulsed ultrasound wave reaches this vicinity region R201.

The pulse synchronization control circuit 208a also sends a control signal to a scanning unit 249 operating to displace the housing unit 211. The scanning unit 249 is capable of displacing the housing unit 211 two-dimensionally in the x- and y- directions, or in the x- and z- directions, or three-dimensionally in the x-, y-, and z-directions in Fig. 57, so that two-dimensional or three dimensional object information is produced and displayed.

The scanning unit 249 may be configured to displace the unit 211 only one-dimensionally. The lens frame 237 to which the collimate lens 238 is mounted is held in a frame body (not shown) of the housing unit 211 to constitute a lens holding section 226. The lens holding section 226 is provided with a knob 226e, so that a user can move the collimate lens 238 together with the lens frame 237 in a direction C perpendicular to the optical axis O by turning this knob 226e.

The housing unit 211 further includes a transducer holding section (acoustic lens holding section) 227 holding a movable frame body 240 to which the acoustic lens 216 for focusing the ultrasound wave is mounted. The transducer holding section 227 is provided with a knob 227e, so that the user can move the acoustic lens 216 together with the movable frame body 240 in a direction D perpendicular to the ultrasound wave transmitting axis Ou by turning this knob 227e. Hence, the optical axis O and the ultrasound wave transmitting axis Ou can be set coaxial with each other.

The lens frame 237 is fitted to a tubular body (not shown), so that the lens frame 237 can be moved in a direction C' of the optical axis O, so that a focus position Fo on which the pulsed light focuses can be adjusted. It is possible to make adjustment such that the pulsed light is focused on the focus point F on which the ultrasound wave focuses. The collimate lens 238 and the lens frame 237 constitute an application position adjusting section.

The ultrasound transducer 202a and the movable frame body 240 to which the acoustic lens 216 is mounted may be configured to be movable in the ultrasound wave transmitting axis Ou, so that they constitute an ultrasound wave focus point adjusting section enabling to adjust the focus point F on which the ultrasound wave focuses.

As shown in Fig. 57, in this embodiment, it is possible to make such an adjustment that the ultrasound wave is focused on the position on which the pulsed light is focused by the collimate lens 238. This makes it possible to extract the scattering information in high spatial resolution and in high S/N.

The output signal of the optical detector 204a constituting the reflected-light receiver 204 is supplied to a signal processing circuit 205c such as a spectrum analyzer constituting the frequency information detecting section or the scattering information extractor.

This signal processing circuit 205c detects the Doppler-shift frequency as the frequency information. From this frequency information, the scattering information equivalent to the real part of the complex refractive index of the vicinity region R201 of the focus point F in the living tissue 207 can be extracted.

The extracted scattering information is supplied to a personal computer (abbreviated as "PC" hereinafter) 206a constituting the object information producer 206, and is stored in a memory included in the PC 206a together with information concerning scanning position. The PC 206a produces the object information by converting the scattering information into image form. This object information is displayed on an output signal display device 210 included in the PC 206a, or provided separately from the PC 206a.

Although the signal processing circuit 205c extracts the scattering information, and the PC 206a produces the object information in this example, both of the extraction of the scattering information and the production of the object information may be performed by one of the signal processing circuit 205c and the PC 206a. The frequency information extracting section may be included in the scattering information extractor 205.

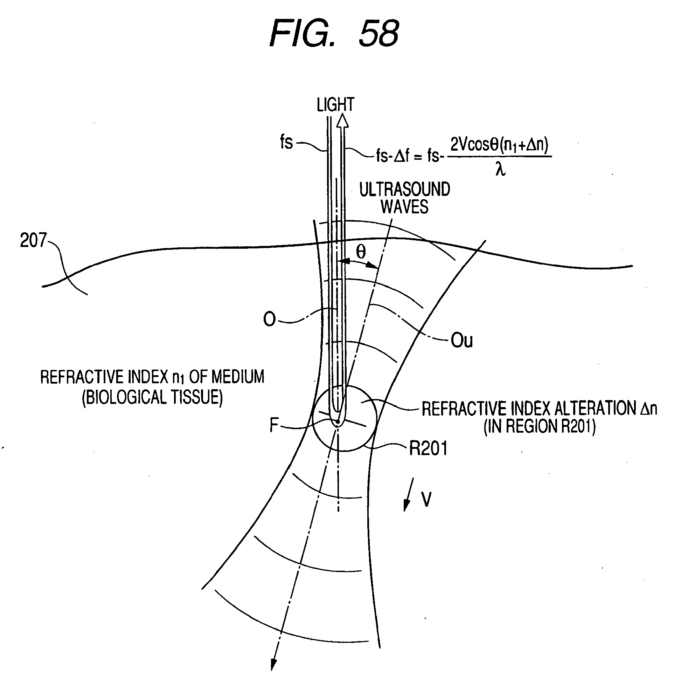

Fig. 58 is a diagram explaining how the light is Doppler-shifted by the action of the ultrasound wave in the vicinity region R201 of the focus point F. Here, it is assumed that the optical axis O is at an angle of $\theta$ with respect to the ultrasound wave transmitting axis Ou, a frequency of the light is $f_s$, a wavelength of the ultrasound wave is $\lambda$, a propagation velocity of the ultrasound wave in the living tissue 207 is V, a refractive index of the living tissue 207 is $n_1$, and a refractive index shift in the vicinity region R201 caused by the action of the ultrasound wave is $\Delta n$.

To be exact, the complex refraction index m (= $m_r + im_l$, $m_r$ being a real part, $m_l$ being an imaginary part) of the living tissue changes in its real part and imaginary part, respectively. However, to detect the first-order variation (Doppler-shifted component) of the light as in this example, it is sufficient to detect the change of the real part. Accordingly, the $\Delta n$ represents a variation $\Delta m_r$ of the real part $m_r$.

As shown in Fig. 58, the frequency of the light scattered at the vicinity region R201 and returning to the entrance side is given by the formula (41).

$$f_s - \Delta f = f_s - 2V\cos\theta(n_1 + \Delta n)/\lambda \quad (41)$$

In this example, the Doppler-shifted component $\Delta f$ of the second term of the formula (41) is detected. The formula (41) is the same as the foregoing formula (21).

If the optical axis O and the ultrasound wave transmitting axis Ou are set coaxial to each other as shown in Fig. 57, $\theta$ is 0, and accordingly $\cos\theta = 1$.

Next, the operation of this example is explained.

First, explanation is made to the signal detection by the optical detector 204a. The ultrasound wave emitted from the ultrasound transducer 202a propagates in the living tissue 207 as a compression wave. The spatial density of an organism's constituents (scatterer or absorber) changes depending on a sound pressure. As shown in Fig. 58, in an ultrasound wave focusing region in which the sound pressure becomes high, the refractive index variation $\Delta n$ becomes large at a high density portion.

When the light is applied to the high density portion, a strong Fresnel reflection occurs at a refractive index changing position. That is, the high density portion serves as a mirror.

Since the refractive index changing position moves in the direction in which the ultrasound wave propagates, the frequency of the reflected light is Doppler-shifted by $\Delta f$.

In a case where the optical tomographic device AP28 in which a Michelson interferometer is formed as shown in Fig. 57 is used, the electric field $E_r(t)$ of the light on the reference mirror 214a side, which enters the optical detector 204a is given by the formula (42), while the electric field $E_0(t)$ of the light on detecting side is given by the formula (43).

$$E_r(t) = E_r \exp i\{2\pi f_s t - k_0(D_1 + L_1 + D_2)\} \quad (42)$$

$$E_0(t) = E_0 \exp i\{2\pi(f_s - \Delta f)t - k_0(D_1 + 2L_2 + 2n_0L_4 + 2n_1z + D_2)\} \quad (43)$$

In these formulae, $D_1$ is a distance between the pulse light source 203c and the half mirror 213a, $D_2$ is a distance between the half mirror 213a and the optical detector 204a, $L_1$ is a distance between the half mirror 213a and the reference mirror 214a, $L_2 + L_4$ is a distance between the half mirror 213a and the surface of the living tissue 207, $L_4$ is a distance between the collimate lens 238 and the surface of living tissue 207, z is a distance between the surface of the organism, the vicinity region R201 of the focus point F, and no is a refractive index of an ultrasound propagating medium such as water 236.

The Doppler-shift frequency $\Delta f$ is given by the formula (41). It can be understood from the formula (41) that Doppler-shift frequency $\Delta f$ depends on the refractive Index variation $\Delta n$.

Since the refractive index is a major parameter of a light-scattering phenomenon (refer to Mie theory, for example), it is possible to determine a local scattering characteristic by measuring the Doppler-shift frequency.

The intensity I(t) of the light received by the optical detector 204a is given by the following formula (44).

$$I(t) = <|E_r + E_o|^2>$$
$$= <|E_r|^2 + |E_o|^2> + 2<|E_r||E_o|>\cos\{2\pi(2V\cos\theta(n_1 + \Delta n_1)/\lambda)t$$
$$+ 2n_1 k_o z\} \quad (44)$$

In this formula, it is assumed that $(L_2 + n_0 L_4) \cong L1$.

By detecting the frequency of the AC component in the formula (44) by use of a spectrum analyzer, or the like, it becomes possible to determine the Doppler-shift frequency, and to obtain the scattering information of a local region.

The above described explanation on the signal detection by the optical detector 204a on the basis of the formulae (42) to (44) is similar to the foregoing explanation on the basis of the formulae (22) to (24).

In this embodiment, since the ultrasound wave and the light are used in the form of pulse, it is possible to detect only an intended signal light component in the vicinity region R201 of the focus point F in high spatial resolution and in high S/N.

Each time one-frame of picture of the scattering information is accumulated in the PC 206a, it is outputted to the output signal display device 210 to be displayed as optical tomogram.

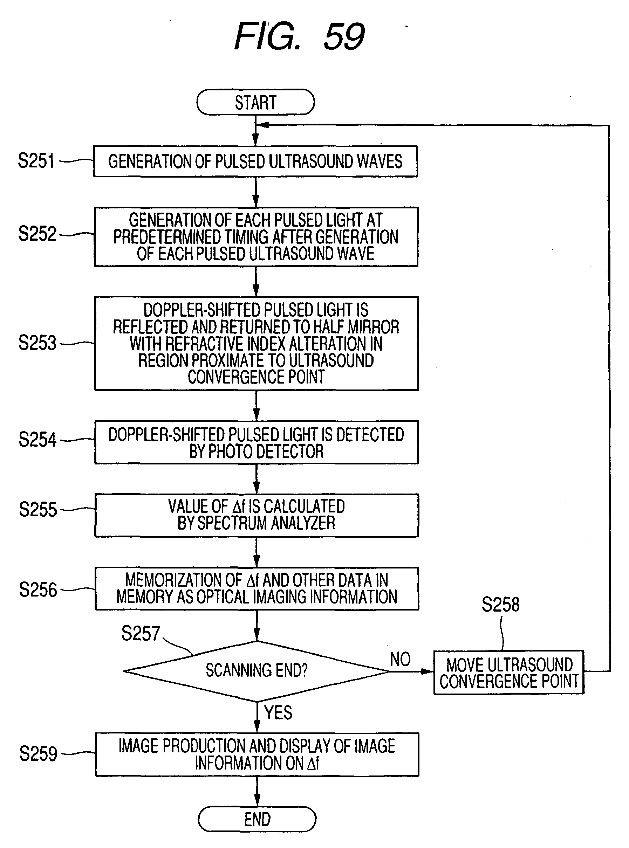

Next, the operation of this example is explained with reference to the flowchart of Fig. 59.

At first step S251, the pulsed drive signal generated by the pulse generator 221 and amplified by the power amplifier 222 is applied to the ultrasound transducer 202a, so that the ultrasound transducer 202a generates the pulsed ultrasound wave. This pulsed ultrasound wave is applied to the living tissue 207 while being focused by the acoustic lens 216.

At following step S252, the pulse synchronization control circuit 246 performs control to generate the pulsed light after an elapse of a certain time from the generation of the pulsed ultrasound wave.

More specifically, the pulse synchronization control circuit 246 adjusts the delay time from the time of the generation of the pulsed ultrasound wave set in the delay circuit 247 to such a value that the pulsed light reaches the focus point F at the same timing at which the pulsed ultrasound wave reaches this focus point F. And the pulse synchronization control circuit 246 sends a control signal to the pulse light source 203c after an elapse of a certain time equivalent to this delay time from the time of generation of the pulsed of the pulsed ultrasound wave, to cause the pulse light source 203c to generate the pulsed light.

At step S253, this pulsed light undergoes a Doppler shift by the refractive index variation in the living tissue 207 caused by the pulsed ultrasound wave focused in the vicinity region R201 of the focus point F. The pulsed light reflected backward returns to the half mirror 213a, and interfered with the reference pulsed light to make an interfered pulsed light having a Doppler shift frequency component. This interfered pulsed light is detected by the optical detector 204a at step 5254.

At following step S255, the value of the Doppler-shift frequency $\Delta f$ is detected by a spectrum analyzer or the like from an output signal of the optical detector 204a.

Subsequently, the detected value of the Doppler-shift frequency $\Delta f$ is stored in the memory included in the PC 206a together with the scanning position information as tomographic information at step S256.

Next, the pulse synchronization control circuit 246 judges at step S257 whether or not the current scanning position is at a terminating position. If the judgment is negative, the pulse synchronization control circuit 246 moves the housing unit 211 to move the focus point F at step S258.

After that, the control process returns to step S251 to repeat the above described processings.

When the scanning is completed for the entire scan region, the control process proceeds from step S257 to step S259. At stepS259, one frame of picture is produced. This one frame of picture is sent to the output signal display device 210 where information concerning $\Delta n$, for example, is displayed in the form of image.

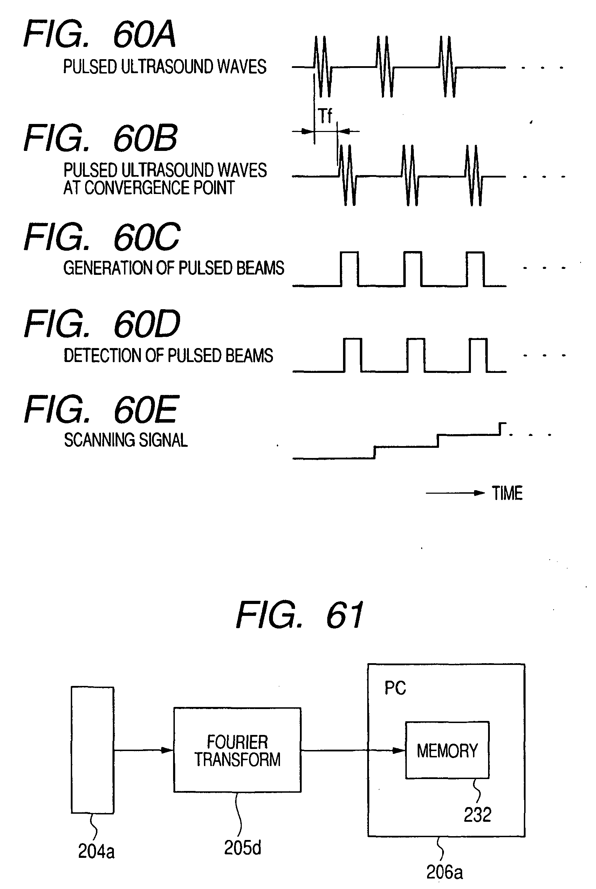

Fig. 60 is a timing chart explaining the operation of this example. At the beginning, the ultrasound transducer 220a emits the pulsed ultrasound wave at a certain timing ((A) in Fig. 60). This pulsed ultrasound wave reaches the focus point F after an elapse of a time Tf ((B) in Fig. 60).

The pulse light source 203c emits the pulsed light when time Tf minus a time needed for the pulsed light to travel a distance from the pulse source device 203c to the focus point F has elapsed from the emission of the pulsed ultrasound wave ((C) in Fig. 60).

The optical detector 204a detects the pulsed light reflected at the focus point F and returning as an observation pulsed light in synchronization with a gate pulse having a short duration ((D) in Fig. 60).

The scanning unit 249 moves the housing unit 211 by a one-step distance after the optical detector 204a detects the pulsed light ((E) in Fig. 60).

In this way, the interfered pulsed light that has undergone the Doppler shift is successively detected, while the focus point F is moved two-dimensionally, or three-dimensionally.

As explained above, the Doppler-shift frequency Δf is calculated on the basis of the pulsed light detected by the optical detector 204a by use of a spectrum analyzer, or the like, and stored in the memory included in the PC 206a. The object information analyzing apparatus of this embodiment can be made compact in size, because the ultrasound wave transmission axis Ou and the optical axis O are set so as to coincide with each other.

In addition, since the pulse synchronization control is performed in order that the pulsed light reaches the vicinity region R201 of the focus point F at the same timing at which the pulsed ultrasound wave reaches this vicinity region R201, it is possible to perform the signal detection in high S/N and in high spatial resolution. That is, since noise light is suppressed from occurring in this embodiment, it is possible to obtain high quality scattering information.

**(Modifications of the twelfth example)**

<First modification>

In the twelfth example, although the Doppler-shift frequency Δf is calculated from the output signal of the optical detector 204a by use of a spectrum analyzer, or the like, it may be calculated through Fourier transform processing by use of a Fourier transform circuit 205d as shown in Fig. 61.

In this case, the Doppler-shift frequency Δf calculated through Fourier transform processing is stored in a memory 232 included in the PC 206a while being associated with the scanning position information. According to the first modification, it is possible to calculate the scattering information equivalent to the real part of the complex refraction index by software. This makes it possible to reduce the production cost of the object information analyzing apparatus.

<Second modification>

Fig. 62 is a diagram showing a structure of a second modification of the twelfth example. The second modification has a configuration in which the pulsed light to be detected is not interfered with the reference pulsed light. Accordingly in the second modification, the reference light producer 214 is eliminated.

Unlike the optical tomographic device AP28 shown in Fig. 57, in an optical tomographic device AP29 of the second modification, the pulsed light emitted from the pulse source 203c is reflected at the half mirror 213a, and then applied to the living tissue 207 along the optical axis O.

The pulsed light reflected at the living tissue 207 to undergo the Doppler shift and transmitting through the half mirror 213a enters a spectroscope 250 to be optically separated in wavelength.

The pulsed light separated in wavelength by the spectroscope 250 is received by the optical detector 204a where it is converted into an electric signal. This electric signal is supplied to a signal processing circuit 205e. This signal processing circuit 205e includes an A/D converter circuit, a computation circuit, and a memory. The computation circuit calculates the Doppler-shift frequency Δf and the refractive index variation Δn from the electric signal converted into a digital signal by the A/D converter circuit and from information used for performing the wavelength separation in the spectroscope 250. The information concerning the refractive index variation Δn is stored in the memory together with the scanning position information.

The information stored in this memory is outputted to the output signal display device 210. As for the rest, the second modification is the same as the twelfth embodiment shown in Fig. 57 in structure. The second modification can be made further compact in size, because it does not need the reference light producer 214.

In this modification, the Doppler-shift frequency etc. may be calculated by performing Fourier transform on the output signal of the optical detector 204a without use of the spectroscope 250.

<Third modification>

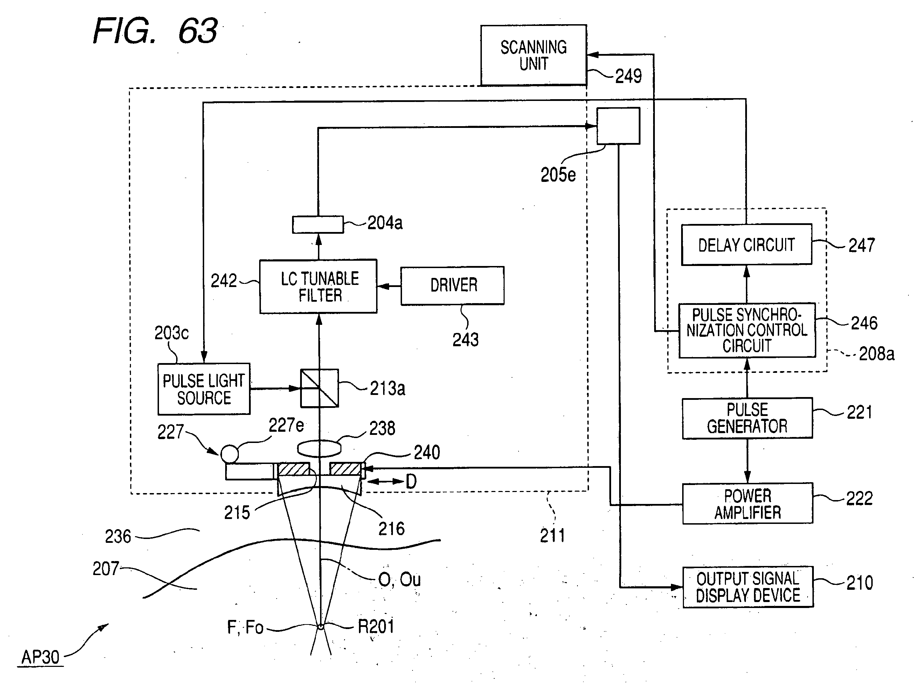

In the configuration shown in Fig. 62, the wavelength separation is performed by the spectroscope 250 which includes a prism or a diffraction grating to extract an intended wavelength of the light utilizing the fact that refraction angle in the prism or diffraction grating depends on the wavelength. However, it may be performed by use of a liquid crystal tunable filter. As shown in Fig. 63, the third modification is provided with an optical tomographic device AP30 including a liquid crystal tunable filter 242.

The transmissive wavelength of this liquid crystal tunable filter 242 varies in accordance with a drive signal applied thereto by a driver 243. The pulsed light transmitted through the liquid crystal tunable filter 242 is received by the optical detector 204a. The output signal of the optical detector 204a is stored in the memory through the computation circuit of the signal processing circuit 205e while being associated with the scanning position information. The collimate lens 238 is fixed to the housing unit 211 to offer reduction of production cost and weight.

As for the rest, the third modification is the same as the second modification shown in Fig. 62. The third modification offers substantially the same advantages offered by the second modification.

The liquid crystal tunable filter 242 may be replaced by an acoustic-optical diffraction grating to optically detect (extract) the Doppler-shifted pulsed light.

The configuration shown in Fig. 63 may be so modified that information in relation to the Doppler-shift frequency and the refractive index variation $\Delta n$ are calculated by performing Fourier transform on the output signal of the optical detector 204a without use of the liquid crystal tunable filter 242.

Although the optical axis O and the ultrasound wave transmitting axis Ou have been described as being coaxial with each other, they may form a slight angle to each other, or have a slight distance from each other.

**(Thirteenth embodiment)**

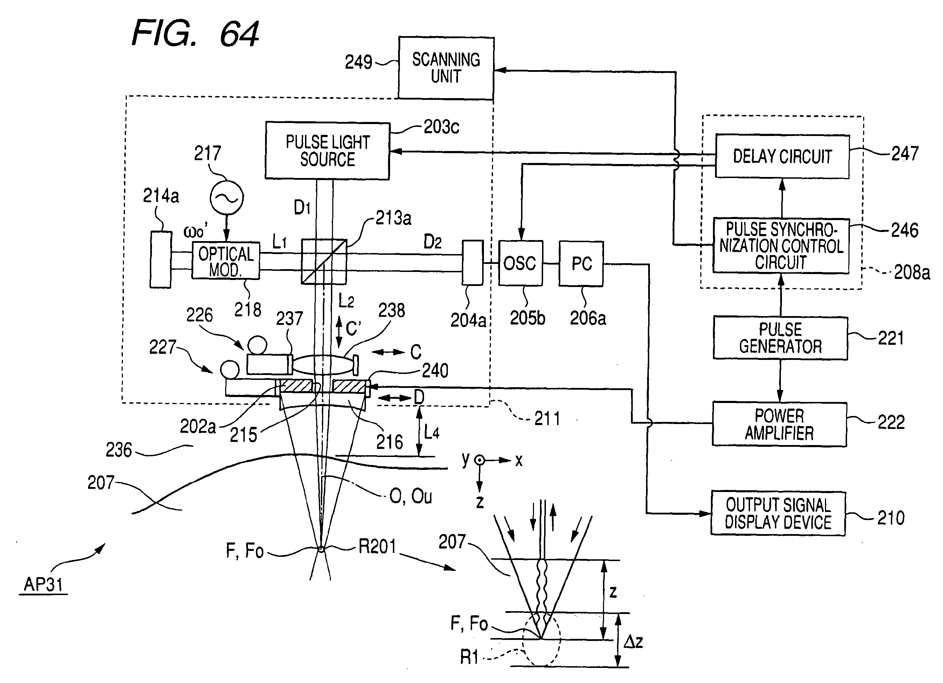

Referring to Figs. 64 - 65, a thirteenth embodiment of the present invention will now be described.

In the above described twelfth example, the scattering information equivalent to the real part of the complex refraction index is extracted by detecting the Doppler-shifted pulsed light, while on the other hand, in this embodiment, the scattering information equivalent to the real part of the complex refraction index is extracted by detecting the phase information of the reflected light.

Fig. 64 is a diagram showing a structure of an optical tomographic device AP 31 included in the object information analyzing apparatus of the thirteenth embodiment. The optical tomographic device AP 31 is different from the optical tomographic device AP28 of the twelfth example shown in Fig. 57 in that an optical modulator 218 is additionally disposed on the optical path between the half mirror 213a and the reference mirror 214a.

This optical modulator 218 is configured to vary its refractive index in proportion to an AC electric field applied thereto by an oscillator 217. The optical modulator 218 may be constituted by a ferroelectric crystal having a strong electro-optical effect such as $LiNbO_3$. When the oscillation signal generated by the oscillator 217 has an angular frequency of $\omega'_0$, the pulsed light reflected at the half mirror 213a and entering the optical modulator 218 undergoes optical modulation at this angular frequency of $\omega'_0$.

This optically modulated pulsed light is reflected at the reference mirror 214a, undergoes the optical modulation in the optical modulator 218 again, and returns to the half mirror 213a

where the reference pulsed light and the observation pulsed light coming from the living tissue 207 are interfered (or mixed) with each other. The interfered pulsed light is received by the optical detector 204a, and converted into an electric interfered signal there.

The interfered signal outputted from the optical detector 204a is supplied to an oscilloscope 205b constituting the scatteing information extractor 205, where the phase component and the amplitude component of the observation pulsed light are extracted. Instead of the oscilloscope 205b, a lock-in amplifier may be used.

The output signal of the oscilloscope 205b is converted into a digital signal, and stored in the memory Included in the PC 206a while being associated with the scanning position information.

As explained above, in the thirteenth embodiment, the scattering information equivalent to the real part of the complex refractive index is extracted by detecting the phase information of the light scattered due to the complex refractive index variation caused by the action of the ultrasound wave.

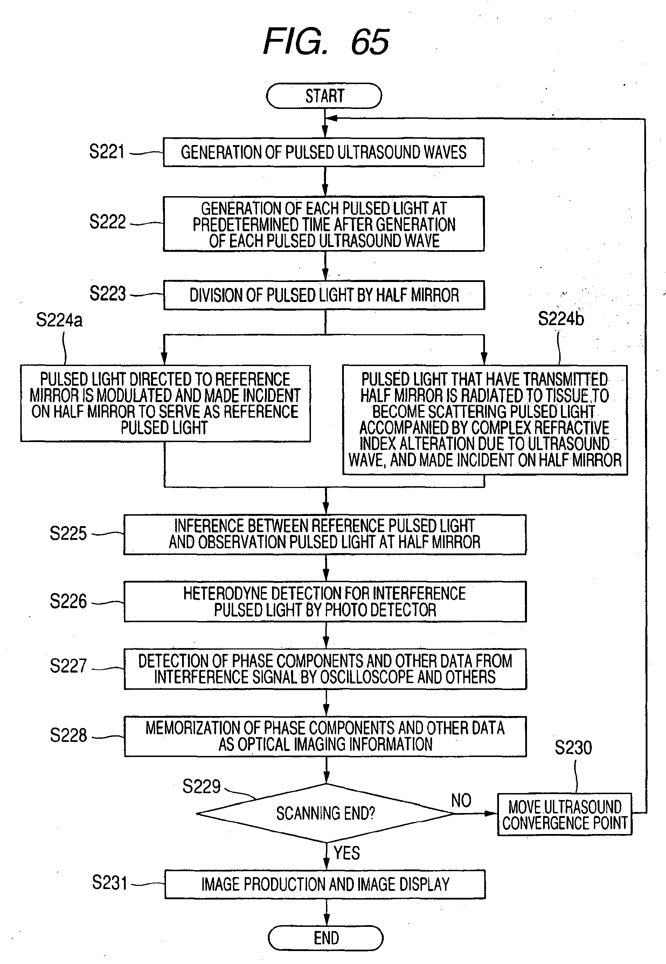

Next, the operation of the thirteenth embodiment is explained with reference to Fig. 65.

At first step S221, the pulse-like ultrasound drive signal generated by the pulse generator 221 and amplified by the power amplifier 222 is applied to the ultrasound transducer 202a, so that the ultrasound transducer 202a generates the pulsed ultrasound wave. This pulsed ultrasound wave is applied to the living tissue 207 while being focused by the acoustic lens 216.

At following step S222, the pulse synchronization control circuit 246 performs control to generate the pulsed light after an elapse of a certain time from the generation of the pulsed ultrasound wave.

More specifically, the pulse synchronization control circuit 246 adjusts the delay time from the time of the generation of the pulsed ultrasound wave set in the delay circuit 247 to such a value that the pulsed light reaches the focus point F at the same timing at which the pulsed ultrasound wave reaches this focus point F. And the pulse synchronization control circuit 246 sends a control signal to the pulse light source 203c after a certain time equivalent to this delay time from the time of the generation of the pulsed ultrasound wave, to cause the pulse light source 203c to generate the pulsed light.

At step S223, this pulsed light splits into two at the half mirror 213a. At step S224a, the pulsed light going to the reference mirror 214a undergoes the optical modulation in the optical modulator 218, reflected at the reference mirror 214a, and then enters the half mirror 213a again as the reference pulsed light.

On the other hand, the pulsed light transmitting through the half mirror 213a is applied to the living tissue 207 to be scattered depending on the complex refractive index variation caused by the action of the pulse ultrasound wave. This scattered pulsed light again enters the half mirror 213a as the observation pulsed light at step S224b.

At step S225, the reference pulsed light and the observation pulsed light are interfered with each other in the half mirror 213a. At step S226, this interfered pulsed light is received by the optical detector 204a, and heterodyne-detected there.

At step S227, the phase information and amplitude information of the heterodyne-detected interfered signal are detected by the oscilloscope 205b. Details of the detection of the phase information and amplitude information are explained later. At step S228, these pieces of detected information are stored in the memory included in the PC 206a while being associated with the scanning position information.

Finally, at step S229, the pulse synchronization control circuit 246 judges whether or not the current scanning position is at a terminating position. If the judgment is negative, the pulse synchronization control circuit 246 moves the housing unit 211 to move the focus point F. After that, the control process returns to step S221 to repeat the above described processings.

If the judgment is affirmative, since it means that one frame of the scattering information has been obtained, the control process proceeds to step 5231. At this step S231, the PC 206a performs a processing to produce an image from the one frame of scattering information, and displays this image to terminate the control process.

The timing chart shown in Fig. 60 can be used to explain the operation of this embodiment.

Next, explanation is given to the detection of the phase component and the amplitude component of the scattered light. In the following explanation, distance relationships among the components constituting the optical tomographic device shown in Fig. 64 are the same as the optical tomographic device shown in Fig. 57. In the lower part of Fig. 64, there is shown an enlarged view around the vicinity region R201. The vicinity region R201 has a size (a length along the z-axis) of $\Delta z$. Here, it is assumed that the complex refractive index of the vicinity region R201 changes from m to $m+\Delta m$ by the action of the focused ultrasound wave.

The electric field $E_s(z', t)$ passing through the living tissue 207 can be approximated by the following formula (45).

$$E_s(z',t) \square E_s = E_{s0} \exp\{i(\omega_s t - k_s z')\} \quad (45)$$

In this formula, z' is a optical path length, which is given by the following formula (46).

$$
\begin{aligned}
z' &= D_2 + D_1 + 2L_2 + 2n_0 L_4 + 2m(z - \Delta z/2) + (m + \Delta m)\Delta z \\
&= D_2 + D_1 + 2L_2 + 2n_0 L_4 + 2(m_r - im_l)z - (m_r - im_l)\Delta z + \\
&\qquad\qquad\qquad \{(m_r + \Delta m_r) - i(m_l + \Delta m_l)\}\Delta z \\
&= D_2 + D_1 + 2L_2 + 2n_0 L_4 + 2(m_r z - \Delta m_r \Delta z) - i(2m_l z + \Delta m_l \Delta z) \\
&= D_1 + D_2 + 2L_2 + 2n_0 L_4 + (2m_r z + \Delta m_r \Delta z) - i(2m_l z + \Delta m_l \Delta z)
\end{aligned}
$$

$$(46)$$

Substituting the formula (46) into the formula (45) to eliminate z', the following formula (47) is obtained.

$$
\begin{aligned}
E_s(z,t) &= E_{s0} \exp[i\{(\omega_s t - k_0\{(D_1 + D_2 + 2L_2 + 2n_0 L_4 + 2m_r z + \Delta m_r \Delta z) - \\
&\qquad\qquad\qquad i(2m_l z + \Delta m_l \Delta z)\}\}] \\
&= E_{s0} \exp[i\{\omega_s t - k_0(D_1 + D_2 + 2L_2 + 2n_0 L_4 + 2m_r z + \Delta m_r \Delta z)\}] \\
&\qquad\qquad\qquad \exp\{-k_0(2m_l z + \Delta m_l \Delta z)\} \\
&= E_{s0} \exp\{-k_0(2m_l z + \Delta m_l \Delta z)\}
\end{aligned}
$$

$$\exp[i\{\omega_s t - k_0(D_1 + D_2 + 2L_2 + 2n_0 L_4 + 2m_r z + \Delta m_r \Delta z)\}]$$

$$(47)$$

The reference pulsed light $E_r(t)$ is given by the following formula (48).

$$E_r(t) = E_{ro}\exp[i\{(\omega_s - 2\omega'_o)t - k_0\{D_1 + 2L_1 + D_2\}] \quad (48)$$

Here, $I(z, t) = |E_5(z, t) + E_r(t)|^2$ is calculated. When putting

$$E_s(z,t) = E'_s\exp\{i(\omega_s t + \phi_1)\}, \quad E_r(t) = E_{ro}\exp[i\{(\omega_s - 2\omega'_o)t + \phi_2\}]$$

$$E = E_s(z,t) + E_r(t)$$

$$= \{E'_{so}\exp(i\phi_1) + E_{ro}\exp(-i2\omega'_o t + \phi_2)\}]\exp(i\omega_s t)I(z,t)$$

$$= |EE^*|$$

$$= \{E'_{so}\exp(i\phi_1) + E_{ro}\exp(-i2\omega'_o t + i\phi_2)\}$$

$$\exp(i\omega_s t) \times \{E'_{so}\exp(-i\phi_1) + E_{ro}\exp(i2\omega'_o t - i\phi_2)\}\exp(-i\omega_s t)I(z,t)$$

$$= E'^2_{so} + E^2_{ro} + E'_{so}E_{ro}\exp\{i2\omega'_o t + i\phi_1 - i\phi_2\} +$$

$$E'_{so}E_{ro}\exp\{-i2\omega'_o t - i\phi_1 + i\phi_2\}$$

$$= D.C. + E'_{so}E_{ro}\exp\{-i(2\omega'_o t - \phi_2 + \phi_1)\} +$$

$$E'_{so}E_{ro}\exp\{i(2\omega'_o t - \phi_2 + \phi_1)\}$$

$$= D.C. + 2E'_{so}E_{ro}\cos(2\omega'_o t - \phi_2 + \phi_1) - \phi_2 + \phi_1$$

$$= k_0(D_1 + D_2 + 2L_1) - k_0(D_1 + D_2 + 2L_2 + 2n_0L_4 + 2m_r z + \Delta m_r \Delta z)$$

$$= -k_0(2L_2 - 2L_1 + 2n_0L_4 + 2m_r z + \Delta m_r \Delta z)$$

Accordingly,

$$I(z,t) = |EE^*|$$

$$= D.C. + 2E_{so}E_{ro}\exp\{-k_0(2m_l z + \Delta m_l \Delta z)\}$$

$$\cos\{2\omega'_o t - k_0(2L_2 - 2L_1 + 2n_0L_4 + 2m_r z + \Delta m_r \Delta z)\},$$

where $n_0$ is a refractive index of water 236.

Here, if the reference arm length and the optical path length between the half mirror 213a and the living tissue 207 are adjusted such that $L_2 - L_1 + n_0L_4 = 0$, the above formula can be transformed as follows.

$$I(z,t)$$

$$= D.C. + 2E_{so}E_{ro}\exp\{-k_0(2m_l z + \Delta m_l \Delta z)\}$$

$$\cos\{2\omega'_o t - k_0(2m_r z + \Delta m_r \Delta z)\}$$

$$(49)$$

This formula (49) shows that if both the real part $m_r + \Delta m_r$ and the imaginary part $m_l + \Delta m_l$ of the complex refractive index $m + \Delta m$ of the medium of the ultrasound wave focusing region change, each of the phase term and the amplitude term of the returning light or the scattered light passing through the ultrasound wave focusing region undergoes the optical modulation.

In this embodiment, the local scattering and absorption characteristics in the medium of the ultrasound wave focusing region are obtained by detecting the phase-modulated component and the amplitude-modulated component of the light returning from this medium.

The imaginary part ($m_I + \Delta m_I$) of the complex refractive index $m+\Delta m$, which is a value representing the amount of light absorption as already explained, is also called an extinction factor. Between this extinction factor and the absorption factor a, the following relationship exists.

$$\alpha = 4n(m_I + \Delta m_I)/\lambda \quad (50)$$

The absorption factor $\alpha$ is equivalent to a reciprocal of a propagation distance at which the intensity of incident light reduces to 1/e.

The formula (49) holds when the incident pulsed light is applied to the living tissue 207, and the observation pulsed light returning from the ultrasound wave focusing region is directly detected. However, since it is difficult to detect the phase component at high S/N in this manner, the reference pulsed light optically modulated by the optical modulator 218 and the observation pulsed light returning from the ultrasound wave focusing region are optically mixed to be interfered with each other, in order to perform the heterodyne-detection as shown in Fig. 64. This interfered light equivalent to a heterodyne-detected, light is received by the optical detector 204a where it is converted into an interfered signal.

In this case, the first term of the formula (49) representing the optical current component detected by the optical detector 204a becomes a DC component term, while the second term becomes an AC component term representing an AC. component varying at a beat angular frequency of $2\omega'_0$. Accordingly, it is possible to obtain information closely related to the absorption characteristic and the scattering characteristic at a depth position of z from the amplitude component and the phase component (phase difference to be exact) of the AC component in the formula (49).

Hence, inputting the signal represented by the formula (49) to the oscilloscope 205b or a lock-in amplifier having a function of a phase meter makes it possible to detect the scattering amount and the absorption amount from the phase difference and the amplitude of the AC component.

As explained below, the phase difference etc. can be calculated by use of a signal processing circuit instead of the phase meter.

The following explanation is directed to a case where the phase difference etc. are calculated by subjecting the output signal of the optical detector 204a to a signal processing.

The data given by the formula (49) is A/D converted, and stored in the memory included in the PC 206a. The CPU included in the PC 206a performs computation on the data stored in the memory to calculate the phase difference (the real part of the complex refractive index) of the AC component shown in formula (49).

To calculate the value of the phase difference largely affecting the scattering characteristic, the CPU performs a Fourier transform processing on the data stored in the memory. By Fourier transforming the I(z,t), the following formula (51) is obtained.
Here, $2\omega'_0 = 2\pi f_0$.

$$F\{I(z,t)\}$$
$$= a\delta(f) + (b/2)\exp(ik_0(2m_r z + \Delta m_r \Delta z))\delta(f-f_0) +$$
$$(b/2)\exp(-ik_0(2m_r z + \Delta m_r \Delta z))\delta(f+f_0)$$
$$(51)$$

In this formula, a is a DC component, and $b = 2E_{s0}E_{r0}\exp\{k_0 (2m_I z + \Delta m_I \Delta z)\}$.

It is possible to determine the phase-difference term of $\{k_0 (2m_r z + \Delta m_r \Delta z)\}$ from the ratio between the real part and the imaginary part of the complex amplitude having a frequency spectrum $f_0$ of the second term of the formula (51). More specifically,

$$\{k_0(2m_r z + \Delta m_r \Delta z)\}$$
$$= \tan^{-1}[Im\{(b/2)\exp(ik_0(2m_r z + \Delta m_r \Delta z))\}/$$
$$Re\{(b/2)\exp(ik_0(2m_r z + \Delta m_r \Delta z))\}]$$
$$(52)$$

According to this embodiment, it is possible to obtain the optical tomographic information of light scattering and light absorption, and to display the optical tomographic information in the form of an image. This makes it possible to provide a diagnostic material effective to make a comprehensive diagnosis on a lesioned region.

Since the object information analyzing apparatus of this embodiment is the type to detect the reflected light, it can be made compact in size.

Furthermore, since the ultrasound wave transmission axis Ou and the optical axis O are set so as to coincide with each other, the object information analyzing apparatus of this embodiment can be made further compact in size.

In addition, since the pulse synchronization control is performed in order that the pulsed light reaches the vicinity region R201 of the focus point F at the same timing at which the pulsed ultrasound wave reaches this vicinity region R201, it is possible to perform the signal detection in high S/N and in high spatial resolution. That is, since noise light is suppressed from occurring, it is possible to obtain high quality scattering information.

Modification of the thirteenth embodiment

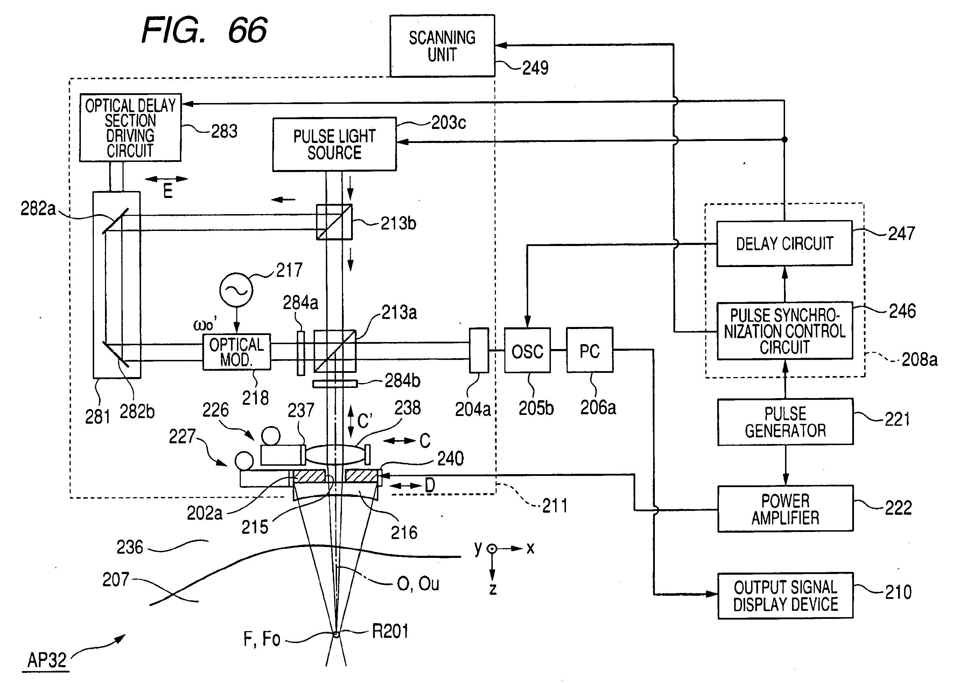

Fig. 66 is a diagram showing a structure of an optical tomographic device AP32 included In a modification of the thirteenth embodiment. This modification includes an optical delay means. The optical tomographic device AP32 is different from the optical tomographic device AP31 shown in Fig. 64 in that a half mirror 213b serving as a second beam splitter is additionally disposed between the pulse source 203c and the half mirror 213a serving as a first beam splitter.

This half mirror 213b partly reflects the pulsed light coming from the pulse source 203c toward a mirror 282a constituting an optical delay section 281.

This optical delay section 281 includes the mirror 282a and a mirror 282b disposed in a housing such that they are opposed to each other across a certain distance. The pulsed light reflected at the mirror 282a is further reflected at the mirror 282b, undergoes modulation in the optical modulator 218, and enters the half mirror 213a as the reference pulsed light.

In this modification, a polarization plate 284a is disposed on the optical path between the optical modulator 218 and the half mirror 213a, so that the reference pulsed light polarized in a predetermined direction enters the half mirror 213a. Also, a polarization plate 284b is disposed on the optical path between the half mirror 213a and the collimate lens 238.

This polarization plate 284b is so disposed that the polarization direction thereof is the same as that of the polarization plate 284a. This modification has such a configuration that the pulsed light applied to the living tissue 207 is polarized, and only a component having a predetermined polarization direction of the observation pulsed light reflected from the living tissue 207 is allowed to enter the half mirror 213a in order to improve coherency.

The travel amount of the housing containing the optical delay section 281 is adjusted in the direction indicated by the double-ended arrow E in Fig. 66 by an optical delay section driving circuit 283. This optical delay section driving circuit 283 is controlled in accordance with a control signal sent from the pulse synchronization control circuit 246 through the delay circuit 247.

The travel amount of the housing containing the optical delay section 281 is controlled by the optical delay section driving circuit 283 such that the timing at which the pulsed light that has transmitted through the half mirror 213a toward the living tissue 207 and reflected at the focus point F enters back the half mirror 213a is the same as the timing at which the reference pulsed light that has reflected at the half mirror 213b and undergone the optical modulation enters the half mirror 213a. As for the rest, the optical tomographic device AP32 of this modification is the same as the optical tomographic device AP31 shown in Fig. 64 in structure.

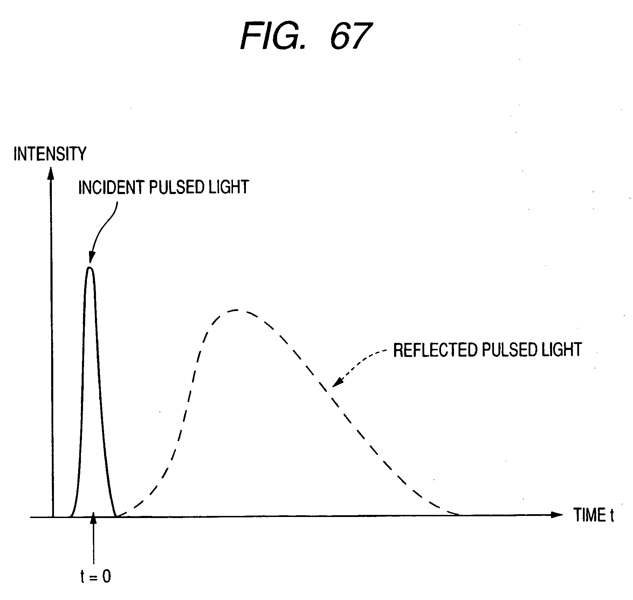

Fig. 67 shows an example of a profile (the dotted line) of the pulsed light reflected from the living tissue 207 after an elapse of time t from the time t=0 at which the pulsed light shown by the solid line is applied to the living tissue. In Fig. 67, the horizontal axis represents time t and the vertical axis represents intensity (amplitude) of light.

As shown in Fig. 67, the pulsed light applied to the living tissue 207 makes the reflected pulsed light shown by the dotted line, which enters the half mirror 213a.

In this modification, the optical delay section 281 adjusts the delay time (the path length of the reference light) such that the pulsed light reflected at the focus point F of the pulsed ultrasound wave enters the half mirror 213a at the same timing at which the reference pulsed light enters the half mirror 213a.

By this adjustment, the reference pulsed light entering the half mirror 213a and the pulsed light reflected from the living tissue 207 interfere with each other, and this interfered pulsed light is received by the optical detector 204a.

When the focus point F of the pulsed ultrasound wave is shifted in the depth direction of the living tissue 207 by the scanning unit 249, the time needed for the pulsed light reflected at the focus point F to reach the half mirror 213a changes.

Accordingly, the optical delay section driving circuit 283 adjusts the delay time of the optical delay section 281 depending on this change.

This modification offers substantially the same advantages offered by the thirteenth embodiment, and in addition, it facilitates obtaining the light scattering information with respect to the depth direction of the living tissue 207. Furthermore, since it adopts the polarizing means, the S/N can be improved.

In the configuration shown in Fig. 66, the optical modulator 218 may be disposed also on the optical path between the half mirror 213a and the collimate lens 238 to further improve the S/N.

The reference mirror 282b may be configured to be moved in the direction E by the optical delay section driving circuit 283 in order to eliminate the use of the half mirror 213b, and the mirror 282a. This configuration can offer the same advantages offered by the configuration of Fig. 66.

**(Fourteenth embodiment)**

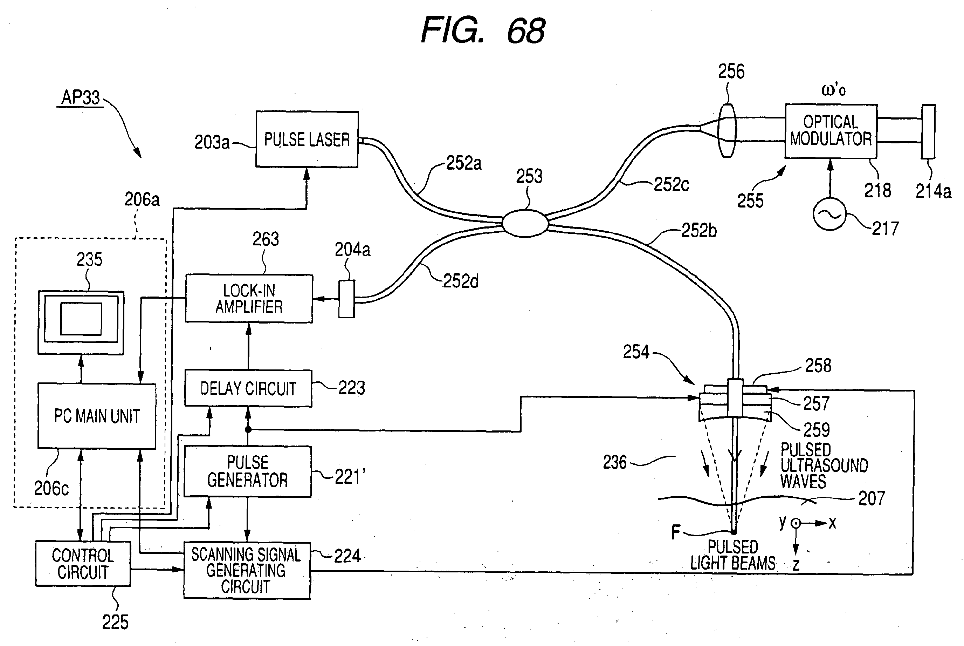

Referring to Figs. 68 - 70, a fourteenth embodiment of the present invention will now be described.

Fig. 68 is a diagram showing a structure of an optical tomographic device AP33 included in an object information analyzing apparatus of a fourteenth embodiment of the invention. This embodiment is characterized by using optical fiber to further make the part for performing the two-dimensional scanning compact in size.

The optical tomographic device AP33 includes a pulse laser device 203a for emitting a pulsed light to the living tissue 207. In place of the pulse laser device 203a, any light source capable of generating pulsed light, such as LED, or SLD may be used.

The pulsed light generated by the pulse laser device 203a enters an end surface of an optical fiber 252a provided for guiding the pulsed light, and splits into two pulsed lights at an optical coupler 253. On of the two pulsed lights is guided to a pulsed light emitting/receiving section 254 through an optical fiber 252b, and the other is guided to a reference light producer 255 through an optical fiber 252c.

The pulsed light applied to the living tissue 207 through water 236 that transmits ultrasound wave, and returning from the living tissue 207 enters the optical fiber 252b as the observation pulsed light, and interferes with the reference pulsed light at the optical coupler 253 to make the interfered pulsed light.

This interfered pulsed light passes through an optical fiber 252d, and is received by the optical detector 204a disposed at an end surface of the optical fiber 252d where it undergoes photoelectric conversion.

The reference light producer 255 collimates the pulsed light emitted from an end surface of the optical fiber 252c by use of a collimate lens 256. The collimated pulsed light is reflected by the reference mirror 214a fixed in position, and again enters the end surface of the optical fiber 252c through the collimate lens 256.

Like the thirteenth embodiment, the optical modulator 218 driven by the oscillation output of the oscillator 217 is disposed between the collimate lens 256 and the reference mirror 214a, so that the pulsed light emitted from the end surface of the optical fiber 252c undergoes the optical modulation.

The pulsed light emitting/receiving section 254 includes an ultrasound transducer 257 and a scanning device 258 disposed in the vicinity of an end surface of the optical fiber 252b.

The ultrasound transducer 257 generates the pulsed ultrasound wave when applied with the pulse-like ultrasound drive signal by a pulse generator 221'. This pulsed ultrasound wave is focused by an acoustic lens 259 and applied to the living tissue 207. This acoustic lens 259 serves as both the pulse generator 221 and the power amplifier 222 shown in Fig. 57.

The scanning device 258 moves the pulsed light emitting/receiving section 254 two-dimensionally in accordance with a scanning signal supplied from a scanning signal generating circuit 224 in synchronization with the pulse-like ultrasound drive signal. For example, the scanning device 258 moves the pulsed light emitting/receiving section 254 in the depth direction of the living tissue 207 (or the z-direction), and the x-direction perpendicular to the z-direction.

The optical fibers 252a, 252b, 252c, 252d may be a single-mode fiber or the like transmitting a single mode light only for the purpose of suppressing dispersion of the pulsed light.

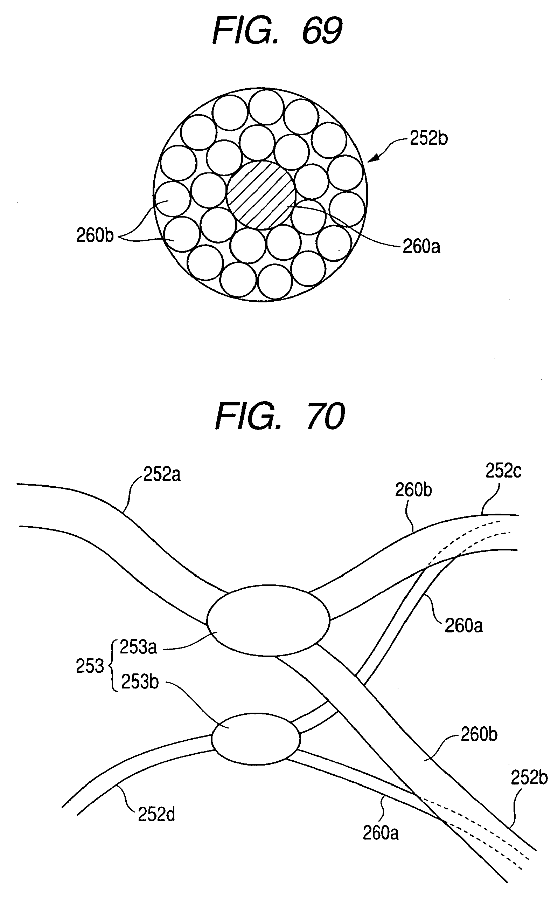

Fig. 69 is an enlarged view of the end surface of the optical fiber 252b. As shown in this figure, the optical fiber 252b is constituted by a first optical fiber portion 260a including one or more optical fibers located in the center thereof, and a second optical fiber portion (fiber bundle) 260b including a plurality of optical fibers located surrounding the first optical fiber portion 260a.

Fig. 70 is an enlarged view around the optical coupler 253. As shown in this figure, the pulsed light generated by the pulse laser device 203a and entering the optical fiber 252a is split into two parts at a first coupler part 253a constituting the optical coupler 253, one of which is guided to the second optical fiber portion 260b of the optical fiber 252b, and the other of which is guided to the second optical fiber portion 260b of the optical fiber 252c.

The pulsed light is emitted from the end surface of the second fiber portion 260b of the optical fiber 252b toward the living tissue 207. The pulsed light returning from the living tissue 207 is received by the first optical fiber portion 260a of the optical fiber 252b. This pulsed light received by the first optical fiber portion 260a of the optical fiber 252b is mixed with the reference pulsed light guided through the first optical fiber portion 260 of the optical fiber 252c to make the interfered pulsed light at a second coupler part 253b constituting the optical coupler 253.

This interfered pulsed light is guided through the optical fiber 252d, and received by the optical detector 204a.

The detection signal of the optical detector 204a is applied to a signal input terminal of a lock-in amplifier 263.

This lock-in amplifier 263 performs a signal detecting operation in synchronization with the signal generated by the pulse generator 221' and delayed by a delay circuit 223.

The signal detected by the lock-in amplifier 263 is converted into a digital signal by an A/D converter circuit included in a PC body 206c constituting the PC 206a, and stored in the memory. A monitor 235 for displaying an optical tomographic information in the form of image is connected to the PC body 206c.

The CPU included in the PC body 206c calculates the scanning position information on the basis of the scanning signal received from the scanning signal generating circuit 224.

This embodiment includes a control circuit 225 doubling as the pulse synchronizer 208. This control circuit 225 controls operations of the pulse generator 221', scanning signal generating circuit 224, delay circuit 223, and pulse laser device 203a. This control circuit 225 has a function of transmitting control signals with the CPU included in the PC body 206c, so that the pulse generator 221' etc. can be controlled by both the control circuit 225, and the PC 206a directly or through the control circuit 225.

In the fourteenth embodiment, the optical tomographic information equivalent to the real part and the imaginary part of he complex refractive index can be obtained by performing the scanning in the vicinity of the end surface of the optical fiber 252b. Accordingly, a scanning device small in size and driving power can be used as the scanning device 258. Other than the above, the advantages offered by the thirteenth embodiment can be offered also by this fourteenth embodiment.

To further improve the S/N, another optical modulator may be disposed between the optical coupler 253 and the scanning device 258.

**(Modifications of the fourteenth embodiment not forming part of the claimed invention**)

<First modification>

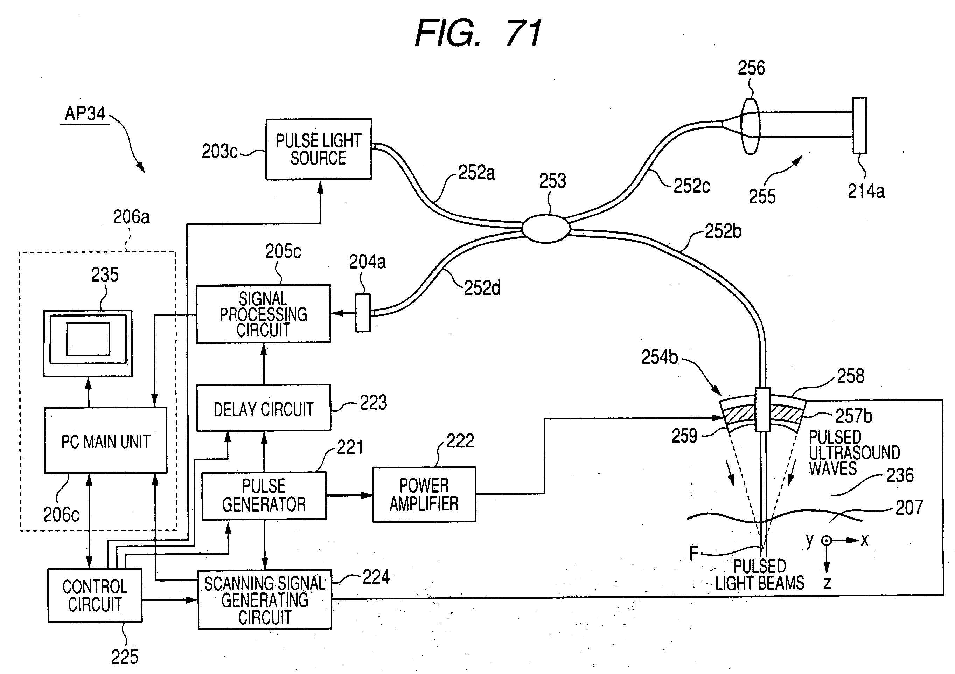

The reference light producer 255 shown in Fig. 68 may be so configured to obtain the scattering information by detecting the Doppler-shifted pulsed light to eliminate the optical modulator 218 and the oscillator 217. Fig. 71 is a diagram showing a structure of an optical tomographic device AP34 including the reference light producer 255 having such a configuration.

Unlike the optical tomographic device AP33 shown in Fig. 68, the optical tomographic device AP34 does not include the optical modulator 218 and the oscillator 217.

This modification is provided with a pulsed light emitting/receiving section 254b including an ultrasound transducer 257b, the scanning device 258, and the acoustic lens, 259 mounted to an ultrasound wave emitting surface of the ultrasound transducer 257b for converging the ultrasound wave. Instead of the ultrasound transducer 257b, the ultrasound transducer 257 having a plate-like shape may be used.

The output signal of the optical detector 204a is supplied to the signal processing circuit 205c such as a spectrum analyzer to detect the Doppler shift frequency Δf. The output signal of the signal processing circuit 205c is inputted to the PC body 206c, and stored in the memory as the optical tomographic information after being A/D converted. The optical fibers 252a, 252b, 252c, 252d may be a single-mode fiber or the like transmitting a single mode light only for the purpose of suppressing dispersion of the pulsed light.

This modification offers substantially the same advantages offered by the fourteenth embodiment.

Additionally, the signal processing circuit 205c may be formed to obtain the Doppler-shift component Δf through execution of Fourier transform.

<Second modification>

Fig. 42 is a diagram showing a structure of an optical tomographic device AP35 of a second modification of the fourteenth embodiment. In this optical tomographic device AP35, any interferometer is not used. That is, this optical tomographic device AP35 does not include the optical coupler 253 which is used in the optical imaging device AP34 shown in Fig. 71 for performing light separation and light coupling. More specifically, the pulsed light generated by the pulse source 203c is guided through an optical fiber bundle 252a' to the light emitting/receiving section 254b, and emitted from an end surface of the pulsed light emitting/receiving section 254b to the living tissue 207.

This optical fiber bundle 252a' forms an optical fiber bundle section 252b' together with an optical fiber 252d' for light receiving use.

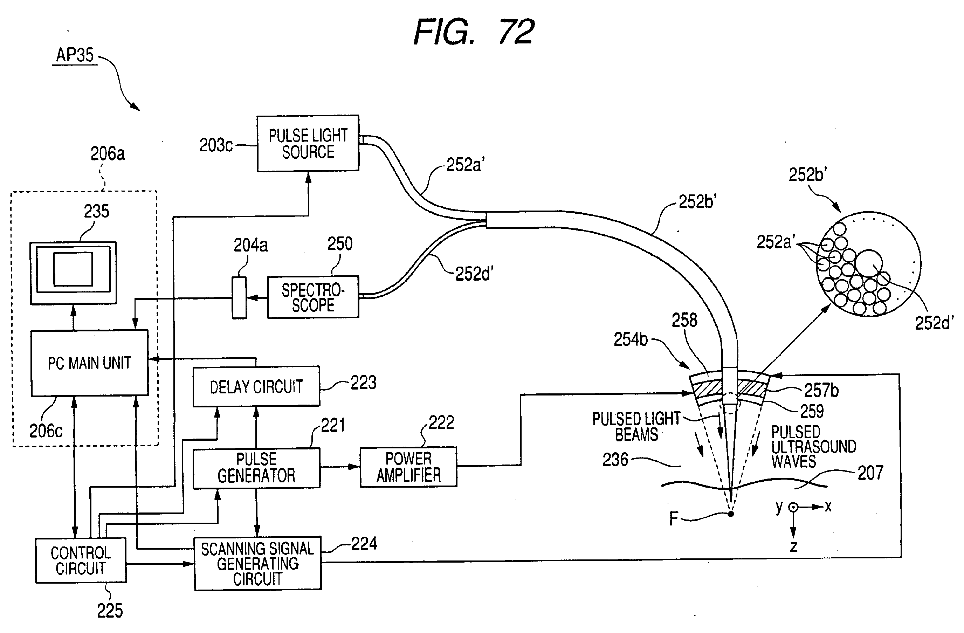

This optical fiber bundle section 252b' has a structure similar to the structure shown in Fig. 69. That is, as shown in an enlarged view of the end surface of the optical fiber bundle section 252b' in Fig. 72, the optical fiber 252d' is located at the center, and optical fibers constituting the optical fiber bundle 252a' are located surrounding the optical fiber 252d'.

The ultrasound transducer 257b having a concave shape, and the scanning device 258 are disposed around the end portion of the optical fiber bundle 252a'. The acoustic lens 259 is mounted to the ultrasound wave emitting surface of the ultrasound transducer 257b for focusing the ultrasound wave.

The centrally located optical fiber 252d' receives the Doppler-shifted pulsed light reflected from the living tissue 207 and guides it to its rear end surface at which the spectroscope 250 is disposed for optically extracting the Doppler-shift frequency component of this pulsed light.

This extracted Doppler-shift frequency component is inputted to the optical detector 204a where it is converted into an electric signal. This electric signal is stored In the memory included in the PC body 206c together with the scanning position information and the information concerning frequency separation in the spectroscope 250.

In this modification, the output signal of the optical detector 204a is directly inputted to the PC body 206c without through the signal processing circuit 205c, because it does not electrically separate the Doppler-shift frequency component unlike in the configuration shown in Fig. 71. Accordingly, the signal outputted from the delay circuit 223 is inputted to the PC body 206c.

As for the rest, the optical tomographic device AP35 of this modification is the same as the optical tomographic device AP34 shown in Fig. 71 in structure. This modification offers substantially the same advantages offered by the first modification shown in Fig. 71. This modification can be made further compact in size, because the optical coupler 253 and the reference light producer 255 can be eliminated. This modification enables to obtain the scattering information (two-dimensional information, for example) equivalent to the real part of the refractive index as the characteristic information of the object.

The optical fiber 252d' of the optical fiber bundle 252a' may be a single-mode fiber or other elements transmitting a single mode light only for the purpose of suppressing dispersion of the pulsed light.

As the spectroscope 250, a liquid crystal tunable filter, or an acoustic-optical element, or a diffraction grating may be used.

Further, when the pulsed light whose frequency is Doppler-shifted is extracted, the spectroscopy 250 may not be used, if the signal detected by the photo detector 204a is subjected to Fourier transform and the resultant transformed signal is used.

**(Fifteenth embodiment)**

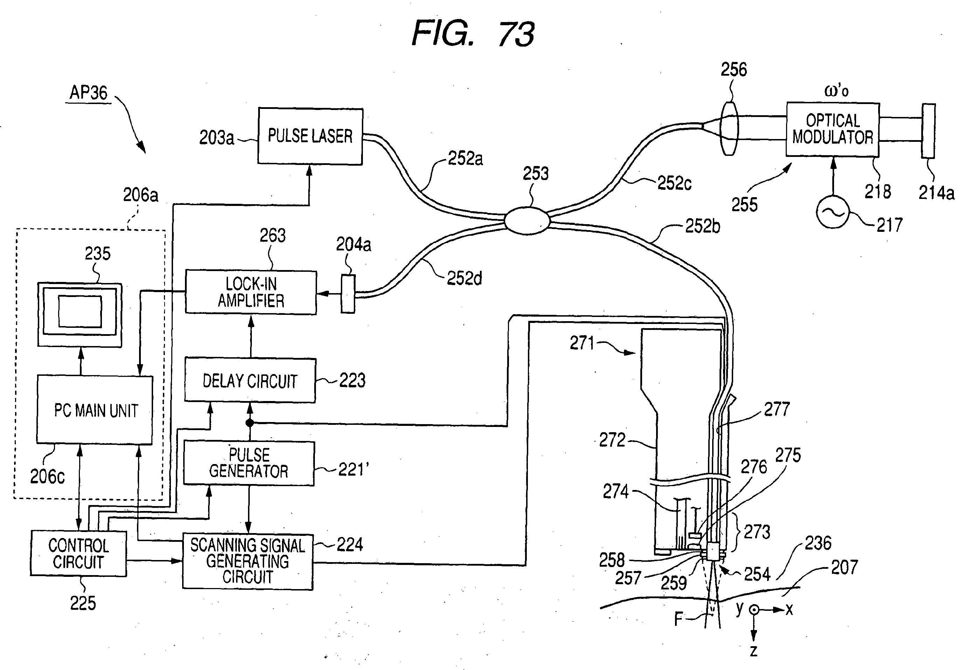

Referring to Fig. 73, a fifteenth embodiment of the present invention will now be described. This embodiment includes an endoscopic device AP36.

Fig. 73 is a diagram showing a structure of the endoscopic device AP36. This endoscopic device AP36 is equivalent to the optical tomographic device AP33 shown in Fig. 68 provided with an endoscope 271 in which the pulsed light emitting/receiving section 254 is disposed.

This endoscope 271 includes an inserting portion 272 and a leading end portion 273 provided in the front end of the inserting portion 272. The leading end portion 273 is provided with an illumination window through which illumination light is emitted, and an observation window for observation use or image pick-up use. The illumination light is emitted through one end surface (front end surface) of a light guide 274 mounted to the illumination window.
The illumination light generated by an endoscope light source (not shown) enters the other end surface (not shown) of the light guide 274.

An objective lens 275 is mounted to the observation window. A CCD 276 is disposed at an image focus point of the objective lens 275 as an image pick-up device. This CCD 276 is connected to a signal processing device (not shown) such as a video processor which performs signal processing on an image signal picked up by the CCD 276 to form a picture signal, and outputs it to a monitor (not shown).

The inserting portion 272 is formed with a channel 277 extending in its longitudinal direction which allows a treatment device to pass therethrough. The optical fiber 252b is inserted through this channel 277. The pulsed light emitting/receiving section 254b shown in Fig. 68 is detachably mounted to a front opening end of the channel 277.

A signal line for transmitting the pulse-like ultrasound drive signal from the pulse generator 221'and the scanning signal from the scanning signal generating circuit 224 is also inserted through the channel 277, so that they are applied to the ultrasound transducer 257 and the scanning device 258, respectively.

According to this embodiment in which the pulsed light emitting/receiving section 254 can be detachably mounted to the channel 277 of the endoscope 271, the scattering information can be easily obtained. This embodiment is advantageous especially in a case where it is necessary to make a detailed diagnosis on a lesioned region in a coelom under optical observation. This makes it easy to make a comprehensive diagnosis in such a case.

**(Modifications of the fifteenth embodiment)**

<First modification>

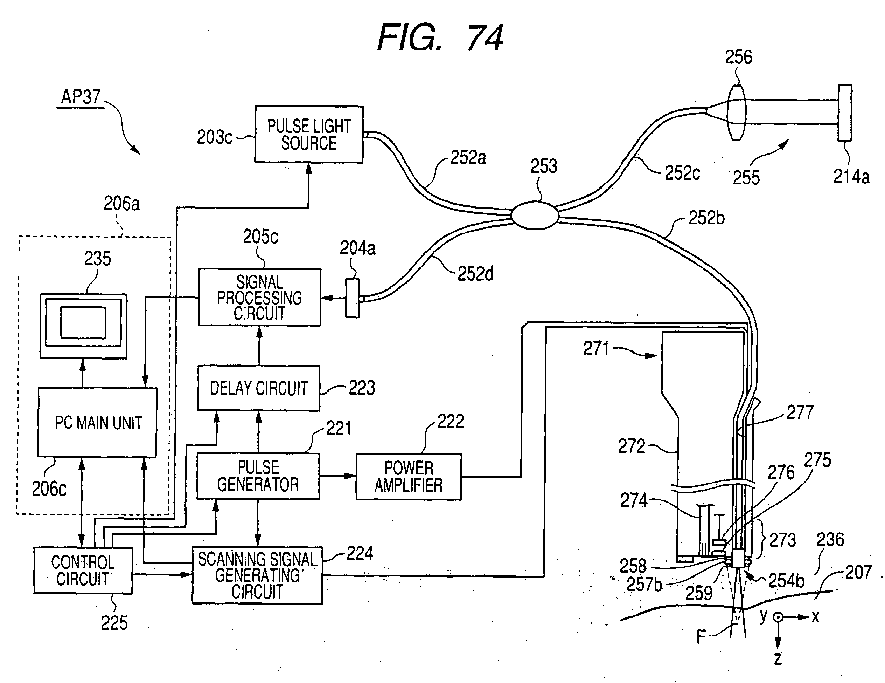

Fig. 74 is a diagram showing an endoscopic device AP37 included in a first modification of the fifteenth embodiment. This endoscopic device AP37 is equivalent to the optical tomographic device AP 34 shown in Fig. 71 provided with the endoscope 271.
In this endoscopic device AP37, the pulsed light emitting/receiving section 254b is included in the endoscope 271.

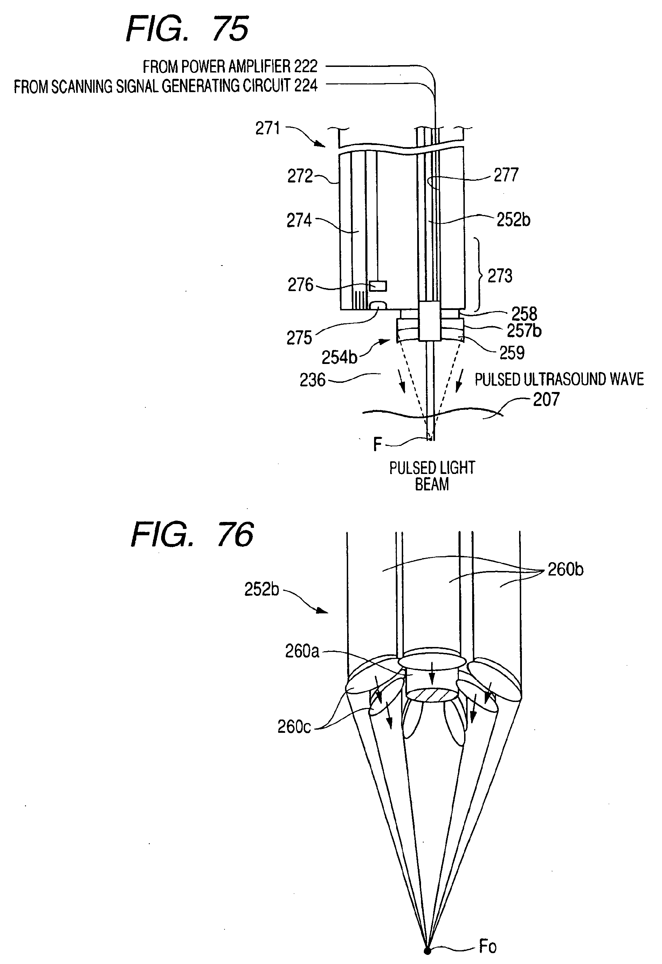

Fig. 75 is a diagram showing a configuration of the front end portion of the endoscope 271 shown in Fig. 74. The pulsed light emitting/receiving section 254b is mounted to the front opening end of the channel 277 through which the optical fiber 252b etc. are inserted. This modification offers substantially the same advantage offered by the fifteenth embodiment.

<Second modification>

Fig. 76 is a diagram showing a configuration of the front end portion of the optical fiber 252b in a second modification of the fifteenth embodiment. As explained with reference to Fig. 69, the optical fiber 252b has such a structure that the first optical fiber portion 260a is located in the center thereof, and the second fiber portion 260b constituted by a plurality of fibers is located surrounding the first optical fiber portion 260a.
As shown in Fig. 76, the end surfaces of the fibers constituting the second fiber portion 260b are worked to form concave surfaces rotationally symmetrical with respect to the axis of the first optical fiber portion 260a. A collimate lens (or a condenser lens) is mounted to each of these end surfaces, so that the light emitted from these end surfaces of the fibers constituting the second fiber portion 260b is focused on the focus position Fo.
This makes it possible to obtain the optical tomographic information in high spatial resolution and in high S/N.
Each of the first optical fiber portion 260a and the fibers constituting the second optical fiber portion 260b may be a single-mode fiber or other elements for the purpose of suppressing pulse dispersion.
As already explained in foregoing each example, in the configuration where the electric signal outputted from the optical detector 204a is subjected to electrical signal processing in a spectrum analyzer or a Fourier transformer to electrically separate the Doppler-shift frequency component (Fig. 57, for example), it may be so modified that the Doppler-shift frequency component is optically separated by use of an optical separation means.
Conversely, in the configuration where the Doppler-shift frequency component is optically separated by use of an optical separation means (Fig. 62, for example), it may be so modified that the Doppler-shift frequency component is electrically separated by use of an electrical signal processing means.
According to the twelfth example and thirteenth to fifteenth embodiments and their modifications where the pulse synchronization control Is performed in order that the pulsed light reaches a region of interest at the same timing at which the pulsed ultrasound wave reaches this region, and the pulsed light reflected from this region is detected to extract, at least, the scattering information equivalent to the real part of the complex refractive index of the region of interest, it is possible to obtain information having a high correlation to conformational change of a lesioned tissue, which can be effectively used to make a diagnosis on the lesioned tissue.

**Claims**

1. An apparatus for analyzing information indicative of a characteristic of an object to be diagnosed, comprising:

   an ultrasound generator (2) configured for generating a beam-formed ultrasound wave toward a region to be examined of the object along a direction given by a desired spatial axis;
   a light generator (3) configured for generating a beam of light toward the region of the object;
   a light receiver (4) configured for:

      receiving light obtained from the region of the object; and
      outputting an electric signal corresponding to the received light; and

   an information acquiring unit (5) configured to detect phase modulation components and amplitude modulation components of the electric signal,

   **characterized by**

an information producer (6) configured to calculate a real part and an imaginary part of the complex refractive index of the region based on the phase modulation components and the amplitude modulation components acquired by the information acquiring unit (5), wherein
the real part of the complex refractive index reflects scattering characteristics of the region, and the imaginary part of the complex refractive index reflects absorption characteristics of the region.

2. The apparatus of claim 1, further comprising a scanning unit (12a,12b) that scans both the beam-formed ultrasound wave and the beam of light in at least two dimensions.

3. The apparatus of claim 2, wherein the light generator (3) is configured to generate a central axis of the beam of light in the same direction as a central axis of the beam-formed ultrasound wave.

4. The apparatus of claim 2, further comprising an interference unit (13b) that produces interfered light produced by making the light obtained from the region and the beam of light generated by the light generator (3) which has been passed through an optical modulator (18) to modulate the obtained light interfere with each other.

5. The apparatus of claim 3, wherein the information acquiring unit (5) comprises an oscilloscope (5a) for extracting the information indicative of the phase and amplitude information from the electric signal outputted from the light receiver (4).

6. The apparatus of claim 5, wherein
the light generator (3) is spatially located on the same side as the ultrasound generator (2) to the object, and
the light receiver (4) is spatially located on the same side as the ultrasound generator (2) to the object and is formed as a reflected-light receiver to receive light reflected from the region to be examined.

7. The apparatus of claim 6, wherein
the ultrasound generator (2) comprises a focus adjustor adjusting a focus of the ultrasound wave to enable the focus to be produced at a predetermined position within the object;
the light generator (3) comprises a light adjustor adjusting the generated light to enable the light to be radiated to the position on which the ultrasound wave generated by the ultrasound generator (7) is focused; and
the apparatus further comprising a calculator (33a) that is configured to extract the information reflective of the scattering of the light in the region positionally corresponding to the focus, on the basis of the signal outputted from the reflected-light receiver.

8. The apparatus of claim 1, comprising
a first holder holding the ultrasound generator (2) so that the axis along which the ultrasound wave is transmitted is located at a predetermined spatial position aid
a second holder holding the light generator (3) so that an axis of the light generated from the light generator (3) agrees with the axis along which the ultrasound wave is transmitted.

9. The apparatus of claim 8, wherein
the first holder comprises a holding member movably holding the axis along which the ultrasound wave is transmitted, and
the second holder comprises an adjusting member adjusting the light axis in agreement with the axis along which the ultrasound wave is transmitted, depending on a held state of the first holder.

10. The apparatus of claim 1, comprising an image forming member forming an image based on the information indicative of the characteristic of the region which is produced by the information producer (6).

11. The apparatus of claim 1, wherein a PC functions as the information acquiring unit (5) and the information producer (6).

12. The apparatus of claim 1, wherein
the light generator (3) comprises a light converging unit enabling the generated light to converge onto a predetermined position within at least the region to which the ultrasound wave is radiated.

13. The apparatus of claim 1, wherein
the ultrasound generator (2) comprises an ultrasound converging unit making the ultrasound wave converge into the region to be examined within the object.

**14.** The apparatus of claim 1, wherein

the ultrasound generator (2) is a pulsed ultrasound generator (202) generating a pulsed ultrasound wave serving as the ultrasound wave and

the light generator (3) is a pulsed light generator (203) generating a pulsed light along an axis which is set at a spatially different angle from the axis along which the pulsed ultrasound wave is transmitted toward the object, the angle being equal to or less than a predetermined amount.

**15.** The apparatus of claim 14, comprising

a synchronizer (208) allowing the pulsed ultrasound wave and the pulsed light to be generated in synchronism at a predetermined timing.

**16.** The apparatus of claim 15, wherein

the synchronizer (208) is configured to perform the synchronization such that at a timing when the pulsed ultrasound wave reaches the region to be examined, the pulsed light reaches the region to be examined.

**17.** The apparatus of claim 1, wherein

the ultrasound generator (2) is a pulsed ultrasound generator (202) generating a pulsed ultrasound wave serving as the ultrasound wave,

the light generator (3) is a pulsed light generator (203) generating a pulsed light, serving as the beam of light,

a central axis along which the pulsed ultrasound wave is transmitted and a central axis along which the pulsed light is radiated are set as a substantially the same axis.

**18.** The apparatus of claim 15, comprising

a scanning unit (249) scanning both the pulsed ultrasound generator (202) and the pulsed light generator (203) at least one-dimensionally.

**19.** The apparatus of claim 15, wherein

the synchronizer (208) is configured to control scanning timing at which the scanning unit (249) scans both the pulsed ultrasound generator (202) and the pulsed light generator (203) in synchronism with the timing at which both the pulsed ultrasound wave and the pulsed light are generated.

**20.** The apparatus of claim 15, comprising

a pulsed ultrasound converging unit enabling the pulsed ultrasound wave to converge onto a predetermined position within the object.

**21.** The apparatus of claim 15, comprising

a pulsed light converging unit enabling the pulsed light to converge onto a predetermined position within the object.

**22.** An endoscope system, comprising:

an endoscope insertable into an object and provided with an apparatus for analyzing information indicative of a characteristic of an object to be diagnosed according to claim 1.

**23.** A method of analyzing information indicative of a characteristic of an object to be diagnosed, comprising

a first step (S1) for generating a pulsed beam-formed ultrasound wave toward a region to be examined of the object along a direction given by a desired spatial axis;

a second step (S2) for generating a pulsed beam of light toward the region of the object;

a third step for receiving light obtained from the region of the object and outputting an electric signal corresponding to the received light; and

a fourth step for detecting phase modulation components of the electric signal and amplitude modulation components of the electric signal,

**characterized by** further comprising:

a fifth step for calculating a real part and an imaginary part of the complex refractive index of the region based on the phase modulation components and the amplitude modulation components, wherein

the real part of the complex refractive index reflects scattering characteristics of the region, and the imaginary part of the complex refractive index reflects absorption characteristics of the region.

24. The method of claim 23, wherein the fourth step includes a process to extract the information indicative of the phase of the light obtained from the region from the electric signal, the information indicative of the phase serving as the information indicative of the changes in the real part of the complex refractive index of the medium, and a process to calculate the information indicative of the scattering of the light at the region on the basis of the extracted information indicative of the phase.

25. The method of claim 23, comprising a step for scanning the beam of light so that positions in the object at which the beam of light reaches changes at least one-dimensionally.

26. The method of claim 25, comprising a step for forming an image using the information indicative of the characteristic of the region.

27. The method of claim 23, comprising a step for mutually synchronizing the generation of the ultrasound wave and the beam of light.

**Patentansprüche**

1. Vorrichtung zum Analysieren einer Information, die eine Eigenschaft eines zu diagnostizierenden Objekts anzeigt, umfassend:

   einen Ultraschallgenerator (2), der dazu eingerichtet ist, eine strahlförmige Ultraschallwelle in Richtung eines zu untersuchenden Bereichs des Objekts entlang einer Richtung zu erzeugen, die durch eine gewünschte räumliche Achse gegeben ist;
   einen Lichtgenerator (3), der dazu eingerichtet ist, einen Lichtstrahl in Richtung des Bereichs des Objekts zu erzeugen;
   einen Lichtempfänger (4) der dazu eingerichtet ist:

   Licht zu empfangen, das von dem Bereich des Objekts erhalten wird;
   und
   ein elektrisches Signal auszugeben, das dem empfangenen Licht
   entspricht; und

   eine Informationsaufnahmeeinheit (5), die dazu eingerichtet ist, Phasenmodulationskomponenten und Amplitudenmodulationskomponenten des elektrischen Signals zu erfassen,

   **gekennzeichnet durch**

   einen Informationserzeuger (6), der dazu eingerichtet ist, einen Realteil und einen Imaginärteil des komplexen Brechungsindexes des Bereichs auf der Basis der von der Informationsaufnahmeeinheit (5) aufgenommenen Phasenmodulationskomponenten und Amplitudenmodulationskomponenten zu berechnen, wobei der Realteil des komplexen reflektierten Brechungsindexes Streueigenschaften des Bereichs reflektiert und der Imaginärteil des komplexen Brechungsindexes Absorptionseigenschaften des Bereichs reflektiert.

2. Vorrichtung nach Anspruch 1, die ferner eine Abtasteinheit (12a, 12b) umfasst, die sowohl die strahlförmige Ultraschallwelle als auch den Lichtstrahl in mindestens zwei Dimensionen abtastet.

3. Vorrichtung nach Anspruch 2, wobei der Lichtgenerator (3) dazu eingerichtet ist, eine zentrale Achse des Lichtstrahls in der gleichen Richtung zu erzeugen, wie eine zentrale Achse der strahlförmigen Ultraschallwelle.

4. Vorrichtung nach Anspruch 2, die ferner eine Interferenzeinheit (13b) umfasst, die Interferenzlicht erzeugt, das erzeugt wird, indem veranlasst wird, dass sich das von dem Bereich erhaltene Licht und das von dem Lichtgenerator (3) erzeugte Licht, das durch einen optischen Modulator (18) geleitet worden ist, um das erhaltene Licht zu modulieren, überlagern.

**5.** Vorrichtung nach Anspruch 3, wobei die Informationsaufnahmeeinheit (5) ein Oszilloskop (5a) zum Extrahieren der die Phasen- und Amplitudeninformation anzeigenden Information aus dem von dem Lichtempfänger (4) ausgegebenen elektrischen Signal umfasst.

**6.** Vorrichtung nach Anspruch 5, wobei
der Lichtgenerator (3) bezüglich des Objekts räumlich auf der gleichen Seite angeordnet ist wie der Ultraschallgenerator (2) und
der Lichtempfänger (4) bezüglich des Objekts auf der gleichen Seite angeordnet ist wie der Ultraschallerzeuger (2) und als ein Reflexionslichtempfänger zum Empfangen von Licht ausgebildet ist, das von dem zu untersuchenden Bereichs reflektiert wird.

**7.** Vorrichtung nach Anspruch 6, wobei
der Ultraschallgenerator (2) eine Fokuseinstelleinrichtung umfasst, die einen Fokus der Ultraschallwelle einstellt, um die Erzeugung des Fokus an einer vorbestimmten Position innerhalb des Objekts zu ermöglichen;
der Lichtgenerator (3) eine Lichteinstelleinrichtung umfasst, die das erzeugte Licht einstellt, um die Abstrahlung des Lichts zu der Position zu ermöglichen, auf die die von dem Ultraschallgenerator (7) erzeugte Ultraschallwelle fokussiert ist; und
die Vorrichtung ferner eine Berechnungseinrichtung (33a) umfasst, die dazu eingerichtet ist, auf der Basis des von dem Reflexionslichtempfänger ausgegebenen Signals die Information zu extrahieren, die die Streuung des Lichts in dem hinsichtlich der Position dem Fokus entsprechenden Bereich reflektiert.

**8.** Vorrichtung nach Anspruch 1, die umfasst
einen ersten Halter, der den Ultraschallgenerator (2) hält, so dass die Achse, entlang derer die Ultraschallwelle übertragen wird, an einer vorbestimmten räumlichen Position angeordnet ist, und
einen zweiten Halter, der den Lichtgenerator (3) hält, so dass eine Achse des von dem Lichtgenerator (3) erzeugten Lichts mit der Achse zusammenfällt, entlang derer die Ultraschallwelle übertragen wird.

**9.** Vorrichtung nach Anspruch 8, wobei
der erste Halter ein Halteelement umfasst, das die Achse, entlang derer die Ultraschallwelle übertragen wird, bewegbar hält, und
der zweite Halter ein Einstellelement umfasst, das die Lichtachse in Übereinstimmung mit der Achse, entlang derer die Ultraschallwelle übertragen wird, in Abhängigkeit eines gehaltenen Zustands des ersten Halters einstellt.

**10.** Vorrichtung nach Anspruch 1, die ein Bilderzeugungselement umfasst, das auf der Basis der die Eigenschaft des Bereichs anzeigenden Information, die von dem Informationserzeuger (6) erzeugt wird, ein Bild erzeugt.

**11.** Vorrichtung nach Anspruch 1, wobei ein PC als die Informationsaufnahmeeinheit (5) und der Informationserzeuger (6) fungiert.

**12.** Vorrichtung nach Anspruch 1, wobei
der Lichtgenerator (3) eine Lichtsammeleinheit umfasst, die es dem erzeugten Licht ermöglicht, auf eine vorbestimmte Position innerhalb zumindest des Bereichs zu konvergieren, auf den die Ultraschallwelle abgestrahlt wird.

**13.** Vorrichtung nach Anspruch 1, wobei
der Ultraschallgenerator (2) eine Ultraschallsammeleinheit umfasst, die veranlasst, dass die Ultraschallwelle in den zu untersuchenden Bereich innerhalb des Objekts konvergiert.

**14.** Vorrichtung nach Anspruch 1, wobei
der Ultraschallgenerator (2) ein gepulster Ultraschallgenerator (202) ist, der eine als die Ultraschallwelle dienende gepulste Ultraschallwelle erzeugt, und
der Lichtgenerator (3) ein gepulster Lichtgenerator (203) ist, der ein gepulstes Licht entlang einer Achse erzeugt, die von der Achse, entlang derer die gepulste Ultraschallwelle in Richtung des Objekts übertragen wird, in einem räumlich anderen Winkel festgelegt ist, wobei der Winkel gleich einem vorbestimmten Betrag oder weniger als vorbestimmter Betrag ist.

**15.** Vorrichtung nach Anspruch 14, die umfasst
eine Synchronisationseinrichtung (208), die es ermöglicht, dass die gepulste Ultraschallwelle und das gepulste Licht synchronisiert zu einem bestimmten Zeitpunkt erzeugt werden können.

**16.** Vorrichtung nach Anspruch 15, wobei
die Synchronisationseinrichtung (208) dazu eingerichtet ist, die Synchronisation so auszuführen, dass zu einem Zeitpunkt, zu dem die gepulste Ultraschallwelle den zu untersuchenden Bereich erreicht, das gepulste Licht den zu untersuchenden Bereich erreicht.

**17.** Vorrichtung nach Anspruch 1, wobei
der Ultraschallgenerator (2) ein gepulster Ultraschallgenerator (202) ist, der eine als die Ultraschallwelle dienende gepulste Ultraschallwelle erzeugt,
der Lichtgenerator (3) ein gepulster Lichtgenerator (203) ist, der ein als der Lichtstrahl dienendes gepulstes Licht erzeugt,
wobei eine Achse, entlang derer die gepulste Ultraschallwelle übertragen wird, und eine zentrale Achse, entlang derer das gepulste Licht abgestrahlt wird, als eine im Wesentlichen gleiche Achse festgelegt sind.

**18.** Vorrichtung nach Anspruch 15, die umfasst
eine Abtasteinheit (249), die sowohl den gepulsten Ultraschallgenerator (202) als auch den gepulsten Lichtgenerator (203) zumindest eindimensional abtastet.

**19.** Vorrichtung nach Anspruch 15, wobei
die Synchronisationseinrichtung (208) dazu eingerichtet ist, einen Abtastzeitpunkt, zu dem die Abtasteinheit (249) sowohl den gepulsten Ultraschallgenerator (202) als auch den gepulsten Lichtgenerator (203) abtastet, synchronisiert mit dem Zeitpunkt zu steuern, zu dem sowohl die gepulste Ultraschallwelle als auch das gepulste Licht erzeugt werden.

**20.** Vorrichtung nach Anspruch 15, die umfasst
eine gepulste Ultraschallsammeleinheit, die es der gepulsten Ultraschallwelle ermöglicht auf eine vorbestimmte Position innerhalb des Objekts zu konvergieren.

**21.** Vorrichtung nach Anspruch 15, die umfasst
eine gepulste Lichtsammeleinheit, die es dem gepulsten Licht ermöglicht, auf eine vorbestimmte Position innerhalb des Objekts zu konvergieren.

**22.** Endoskopsystem, das umfasst:

ein Endoskop, das in ein Objekt einführbar ist und mit einer Vorrichtung zum Analysieren einer Information, die eine Eigenschaft eines zu diagnostizierenden Objekts anzeigt, nach Anspruch 1 versehen ist.

**23.** Verfahren zum Analysieren einer Information, die eine Eigenschaft eines zu diagnostizierenden Objekts anzeigt, umfassend:

einen ersten Schritt (S1) zum Erzeugen einer gepulsten strahlförmigen Ultraschallwelle in Richtung eines zu untersuchenden Bereichs des Objekts entlang einer Richtung, die durch eine gewünschte räumliche Achse gegeben ist;
einen zweiten Schritt (S2) zum Erzeugen eines gepulsten Lichtstrahls in Richtung des Bereichs des Objekts;
einen dritten Schritt zum Empfangen von Licht, das von dem Bereich des Objekts erhalten wird, und zum Ausgeben eines elektrischen Signals, das dem empfangenen Licht entspricht; und
einen vierten Schritt zum Erfassen von Phasenmodulationskomponenten und Amplitudenmodulationskomponenten des elektrischen Signals,

**dadurch gekennzeichnet, dass** es ferner umfasst:

einen fünften Schritt zum Berechnen eines Realteils und eines Imaginärteils des komplexen Brechungsindexes des Bereichs auf der Basis der Phasenmodulationskomponenten und Amplitudenmodulationskomponenten, wobei

der Realteil des komplexen reflektierten Brechungsindexes Streueigenschaften des Bereichs reflektiert und der Imaginärteil des komplexen Brechungsindexes Absorptionseigenschaften des Bereichs reflektiert.

**24.** Verfahren nach Anspruch 23, wobei der vierte Schritt einen Prozess zum Extrahieren der Information, die die Phase des von dem Bereich erhaltenen Lichts anzeigt, aus dem elektrischen Signal, wobei die Information, die die Phase anzeigt, als die Information dient, die die Änderungen in dem Realteil des komplexen Brechungsindexes des Mediums anzeigt, und einen Prozess zum Berechnen der Information, die die Streuung des Lichts an dem Bereich auf der Basis der extrahierten Information, die die Phase anzeigt, umfasst.

25. Verfahren nach Anspruch 23, das einen Schritt zum Abtasten des Lichtstrahls umfasst, so dass sich Positionen in dem Objekt, an die der Lichtstrahl reicht, zumindest eindimensional verändern.

26. Verfahren nach Anspruch 25, das einen Schritt zum Erzeugen eines Bilds unter Verwendung der Information umfasst, die die Eigenschaft des Bereichs anzeigt.

27. Verfahren nach Anspruch 23, das einen Schritt zum wechselseitigen Synchronisieren der Erzeugung der Ultraschallwelle und des Lichtstrahls umfasst.

**Revendications**

1. Appareil d'analyse d'informations indicatrices d'une caractéristique d'un objet à diagnostiquer, comprenant :

   un générateur d'ultrasons (2) configuré pour générer une onde ultrasonique formée en faisceau en direction d'une région à examiner de l'objet le long d'une direction donnée par un axe spatial souhaité ;
   un générateur de lumière (3) configuré pour générer un faisceau lumineux en direction de la région de l'objet ;
   un récepteur de lumière (4) configuré pour :

      recevoir la lumière obtenue de la région de l'objet ; et
      émettre un signal électrique correspondant à la lumière reçue ; et

   une unité d'acquisition d'informations (5) configurée pour détecter des composantes de modulation de phase et des composantes de modulation d'amplitude du signal électrique,

   **caractérisé par**

   un producteur d'informations (6) configuré pour calculer une partie réelle et une partie imaginaire de l'indice de réfraction complexe de la région en se basant sur les composantes de modulation de phase et les composantes de modulation d'amplitude acquises par l'unité d'acquisition d'informations (5), dans lequel

   la partie réelle de l'indice de réfraction complexe reflète des caractéristiques de diffusion de la région, et la partie imaginaire de l'indice de réfraction complexe reflète des caractéristiques d'absorption de la région.

2. Appareil selon la revendication 1, comprenant en outre une unité de balayage (12a, 12b) qui balaye à la fois l'onde ultrasonique formée en faisceau et le faisceau de lumière dans au moins deux dimensions.

3. Appareil selon la revendication 2, dans lequel le générateur de lumière (3) est configuré pour générer un axe central du faisceau de lumière dans la même direction qu'un axe central de l'onde ultrasonique formée en faisceau.

4. Appareil selon la revendication 2, comprenant en outre une unité d'interférence (13b) qui produit une lumière interférée produite en faisant en sorte que la lumière obtenue de la région et le faisceau de lumière généré par le générateur de lumière (3) qui est passé à travers un modulateur optique (18) pour moduler la lumière obtenue interfèrent l'un avec l'autre.

5. Appareil selon la revendication 3, dans lequel l'unité d'acquisition d'informations (5) comprend un oscilloscope (5a) pour extraire les informations indicatrices des informations de phase et d'amplitude du signal électrique délivrées en sortie du récepteur de lumière (4).

6. Appareil selon la revendication 5, dans lequel
   le générateur de lumière (3) est spatialement situé sur le même côté que le générateur d'ultrasons (2) par rapport à l'objet, et
   le récepteur de lumière (4) est spatialement situé sur le même côté que le générateur d'ultrasons (2) par rapport à l'objet et est formé comme un récepteur de lumière réfléchie pour recevoir une lumière réfléchie de la région à examiner.

7. Appareil selon la revendication 6, dans lequel
   le générateur d'ultrasons (2) comprend un système d'ajustement de focalisation ajustant une focalisation de l'onde ultrasonique afin de permettre que la focalisation soit produite en une position prédéterminée à l'intérieur de l'objet ;
   le générateur de lumière (3) comprend un système d'ajustement de lumière ajustant la lumière générée afin de permettre que la lumière soit projetée sur la position sur laquelle l'onde ultrasonique générée par le générateur d'ultrasons (2) est focalisée ; et
   l'appareil comprenant en outre un calculateur (33a) qui est configuré pour extraire les informations reflétant la diffusion de la lumière dans la région correspondant en position au foyer, en se basant sur le signal délivré en sortie du récepteur de lumière réfléchie.

8. Appareil selon la revendication 1, comprenant
   un premier dispositif de maintien maintenant le générateur d'ultrasons (2) de telle manière que l'axe le long duquel l'onde ultrasonique est transmise est situé en une position spatiale prédéterminée et
   un deuxième dispositif de maintien maintenant le générateur de lumière (3) de telle manière qu'un axe de la lumière générée par le générateur de lumière (3) coïncide avec l'axe le long duquel l'onde ultrasonique est transmise.

9. Appareil selon la revendication 8, dans lequel
   le premier dispositif de maintien comprend un élément de maintien maintenant de façon mobile l'axe le long duquel l'onde ultrasonique est transmise, et
   le deuxième dispositif de maintien comprend un élément d'ajustement ajustant l'axe de lumière en accord avec l'axe le long duquel l'onde ultrasonique est transmise, en fonction d'un état maintenu du premier dispositif de maintien.

10. Appareil selon la revendication 1, comprenant un élément de formation d'image formant une image en se basant sur les informations indicatrices de la caractéristique de la région qui sont produites par le producteur d'informations (6).

11. Appareil selon la revendication 1, dans lequel un PC fonctionne comme l'unité d'acquisition d'informations (5) et le producteur d'informations (6).

12. Appareil selon la revendication 1, dans lequel
    le générateur de lumière (3) comprend une unité de convergence de lumière permettant que la lumière générée converge sur une position prédéterminée à l'intérieur au moins de la région sur laquelle les ultrasons sont projetés.

13. Appareil selon la revendication 1, dans lequel
    le générateur d'ultrasons (2) comprend une unité de convergence d'ultrasons faisant que l'onde ultrasonique converge dans la région à examiner à l'intérieur de l'objet.

14. Appareil selon la revendication 1, dans lequel
    le générateur d'ultrasons (2) est un générateur d'ultrasons pulsés (202) générant une onde ultrasonique pulsée servant comme l'onde ultrasonique et
    le générateur de lumière (3) est un générateur de lumière pulsée (203) générant une lumière pulsée le long d'un axe qui est réglé à un angle spatialement différent de l'axe le long duquel l'onde ultrasonique pulsée est transmise en direction de l'objet, l'angle étant égal ou inférieur à une quantité prédéterminée.

15. Appareil selon la revendication 14, comprenant
    un synchroniseur (208) permettant que l'onde ultrasonique pulsée et la lumière pulsée soient générées en synchronisme à un instant prédéterminé.

16. Appareil selon la revendication 15, dans lequel
    le synchroniseur (208) est configuré pour exécuter la synchronisation de telle manière que, à un instant où l'onde ultrasonique pulsée atteint la région à examiner, la lumière pulsée atteint la région à examiner.

17. Appareil selon la revendication 1, dans lequel
    le générateur d'ultrasons (2) est un générateur d'ultrasons pulsés (202) générant une onde ultrasonique pulsée servant comme l'onde ultrasonique et

le générateur de lumière (3) est un générateur de lumière pulsée (203) générant une lumière pulsée, servant comme le faisceau de lumière,

un axe central le long duquel l'onde ultrasonique pulsée est transmise et un axe central le long duquel la lumière pulsée est projetée sont réglés comme sensiblement le même axe.

**18.** Appareil selon la revendication 15, comprenant
une unité de balayage (249) balayant à la fois le générateur d'ultrasons pulsés (202) et le générateur de lumière pulsée (203) de manière au moins unidimensionnelle.

**19.** Appareil selon la revendication 15, dans lequel
le synchroniseur (208) est configuré pour commander l'instant de balayage auquel l'unité de balayage (249) balaye à la fois le générateur d'ultrasons pulsés (202) et le générateur de lumière pulsée (203) en synchronisme avec l'instant auquel l'onde ultrasonique pulsée et la lumière pulsée sont générées.

**20.** Appareil selon la revendication 15, comprenant
une unité de convergence d'ultrasons pulsés permettant que l'onde ultrasonique pulsée converge sur une position prédéterminée à l'intérieur de l'objet.

**21.** Appareil selon la revendication 15, comprenant
une unité de convergence de lumière pulsée permettant que la lumière pulsée converge sur une position prédéterminée à l'intérieur de l'objet.

**22.** Système d'endoscope, comprenant :

un endoscope insérable dans un objet et prévu avec un appareil d'analyse d'informations indicatrices d'une caractéristique d'un objet à diagnostiquer selon la revendication 1.

**23.** Procédé d'analyse d'informations indicatrices d'une caractéristique d'un objet à diagnostiquer, comprenant

une première étape (S1) pour générer une onde ultrasonique pulsée formée en faisceau en direction d'une région à examiner de l'objet le long d'une direction donnée par un axe spatial souhaité ;
une deuxième étape (S2) pour générer un faisceau lumineux pulsé en direction de la région de l'objet ;
une troisième étape pour recevoir une lumière obtenue de la région de l'objet et émettre un signal électrique correspondant à la lumière reçue ; et
une quatrième étape pour détecter des composantes de modulation de phase du signal électrique et des composantes de modulation d'amplitude du signal électrique,

**caractérisé en ce que** comprenant en outre :

une cinquième étape pour calculer une partie réelle et une partie imaginaire de l'indice de réfraction complexe de la région en se basant sur les composantes de modulation de phase et les composantes de modulation d'amplitude, dans lequel

la partie réelle de l'indice de réfraction complexe reflète des caractéristiques de diffusion de la région, et la partie imaginaire de l'indice de réfraction complexe reflète des caractéristiques d'absorption de la région.

**24.** Procédé selon la revendication 23, dans lequel la quatrième étape inclut un processus pour extraire les informations indicatrices de la phase de la lumière obtenue de la région à partir du signal électrique, les informations indicatrices de la phase servant comme les informations indicatrices des changements dans la partie réelle de l'indice de réfraction complexe du support, et un processus pour calculer les informations indicatrices de la diffusion de la lumière au niveau de la région en se basant sur les informations indicatrices de la phase extraites.

**25.** Procédé selon la revendication 23, comprenant une étape de balayage du faisceau de lumière de telle manière que des positions dans l'objet que le faisceau de lumière atteint changent de façon au moins unidimensionnelle.

**26.** Procédé selon la revendication 25, comprenant une étape de formation d'une image en utilisant les informations indicatrices de la caractéristique de la région.

**27.** Procédé selon la revendication 23, comprenant une étape de synchronisation mutuelle de la génération de l'onde ultrasonique et du faisceau de lumière.

## FIG. 1

## FIG. 3

# FIG. 2

EP 1 810 610 B1

# FIG. 4

START

S1 — GENERATION OF LIGHT BEAM FROM LASER

S2 — GENERATION OF PULSED ULTRASOUND WAVESWHICH CONVERGE

S3 — DIVISION OF LIGHT BEAM BY HALF MIRROR

S4a — LIGHT BEAM DIRECTED TO REFERENCE MIRROR IS MODULATED BY OPTICAL MODULATOR AND MADE INCIDENT ON HALF MIRROR TO SERVE AS REFERENCE LIGHT BEAM

S4b — LIGHT BEAM DIRECTED TO MIRROR IS RADIATED TO TISSUE TO BECOME SCATTERING LIGHT ACCOMPANIED BY COMPLEX REFRACTIVE INDEX ALTERATION DUE TO ULTRASOUND WAVE, AND THEN MADE INCIDENT ON HALF MIRROR

S5 — INTERFERENCE BETWEEN REFERENCE LIGHT AND OBSERVATION LIGHT AT HALF MIRROR

S6 — HETERODYNE DETECTION FOR INTERFERENCE LIGHT BY PHOTO DETECTOR

S7 — DETECTION OF PHASE COMPONENTS AND OTHER DATA FROM INTERFERENCE SIGNAL BY OSCILLOSCOPE AND OTHERS

S8 — MEMORIZATION OF PHASE COMPONENTS AND OTHER DATA AS OPTICAL IMAGING INFORMATION

S9 — SCANNING END?

NO → S10 — MOVE ULTRASOUND CONVERGENCE POINT

YES

S11 — IMAGE PRODUCTION AND IMAGE DISPLAY

END

**FIG. 5A** LASER BEAM

**FIG. 5B** PULSED ULTRASOUND DRIVING SIGNAL

**FIG. 5C** GATE PULSE

**FIG. 5D** EXTRACTION OF PHASE & AMPLITUDE COMPONENTS

**FIG. 5E** SCANNING SIGNAL

$Ta$

$Tf$

TIME

EP 1 810 610 B1

FIG. 6

# FIG. 7

```
                    ( START )
                        │
                        ▼
        S21 ──  GENERATION OF LIGHT FROM
                    LIGHT SOURCE
                        │
                        ▼
        S22 ──  GENERATION OF PULSED ULTRASOUND
                  WAVES WHICH CONVERGE
                        │
                        ▼
        S23 ──  DIVISION OF LIGHT BY HALF MIRROR
```

S24a                                                 S24b

| LIGHT DIRECTED TO REFERENCE MIRROR IS MODULATED BY OPTICAL MODULATOR AND MADE INCIDENT ON HALF MIRROR TO SERVE AS REFERENCE LIGHT | LIGHT WHICH HAVE TRANSMITTED HALF MIRROR IS RADIATED TO TISSUE TO BECOME SCATTERING LIGHT ACCOMPANIED BY COMPLEX REFRACTIVE INDEX ALTERATION DUE TO ULTRASOUND WAVES, AND THEN MADE INCIDENT ON HALF MIRROR |

```
        S25 ──  INTERFERENCE BETWEEN REFERENCE LIGHT AND
                  OBSERVATION LIGHT AT HALF MIRROR
                        │
                        ▼
        S26 ──  HETERODYNE DETECTION FOR INTERFERENCE LIGHT
                     BY PHOTO DETECTOR
                        │
                        ▼
        S27 ──  DETECTION OF PHASE COMPONENTS AND OTHER
                  DATA FROM INTERFERENCE SIGNAL
                        │
                        ▼
        S28 ──  MEMORIZATION OF PHASE COMPONENTS AND OTHER
                  DATA AS OPTICAL IMAGING INFORMATION
                        │                              S30
                        ▼                               │
        S29 ──◇ SCANNING END? ◇── NO ──▶ MOVE ULTRASOUND
                        │                   CONVERGENCE POINT
                      YES
                        │
                        ▼
        S31 ──  IMAGE PRODUCTION AND IMAGE DISPLAY
                        │
                        ▼
                    ( END )
```

# FIG. 8

SCANNING UNIT ~12

SCANNING SIGNAL GENERATING CIRCUIT ~24

LIGHT SOURCE ~3b

17 ω₀'

14

18 OPTICAL MODULATOR

$L_1$ → $D_1$ ↓ 13 $D_2$ →

2ω₀' 2ωb₀'

$L_2$

4a

18b ωb'₀ ~17b

2a

16

15

Ou ~36

$L_4$

7

y ⊙ → x, z ↓

F

R1 R2

AP3

PC ~6a

SIGNAL PROCESSING CIRCUIT ~5b

DELAY CIRCUIT ~23

PULSE GENERATOR ~21

POWER AMPLIFIER ~22

11

EP 1 810 610 B1

# FIG. 9

EP 1 810 610 B1

# FIG. 10A
CONTINUOUS LIGHT

# FIG. 10B
ULTRASOUND
DRIVING SIGNAL

# FIG. 10C
EXTRACTION OF PHASE &
AMPLITUDE COMPONENTS

# FIG. 10D
SCANNING SIGNAL

Tf

→ TIME

# FIG. 11

**FIG. 12**

AP5

**FIG. 13**

AP6

FIG. 14

EP 1 810 610 B1

## FIG. 15

## FIG. 16

FIG. 17

EP 1 810 610 B1

## FIG. 18

FROM PULSE GENERATOR 21'
FROM SCANNING SIGNAL GENERATING CIRCUIT 24

## FIG. 19

*FIG. 20*

## FIG. 21

1D OPTICAL IMAGING
INFORMATION

2D OPTICAL IMAGING
INFORMATION

SCANNING

## FIG. 23

2D OPTICAL IMAGING
INFORMATION

SCANNING

3D OPTICAL
IMAGING
INFORMATION

# FIG. 22

# FIG. 24

103 — LIGHT GENERATOR

113

REFLECTED-LIGHT RECEIVER
104

FREQUENCY INFORMATION EXTRACTOR
105

SCATTERING INFORMATION EXTRACTOR
106

OBJECT INFORMATION PRODUCER
106'

ULTRASOUND GENERATOR
102

LIGHT AXIS

REGION TO BE EXAMINED

ULTRASOUND TRANSMISSION AXIS

OBJECT INFORMATION ANALYZING APPARATUS

OBJECT

EP 1 810 610 B1

FIG. 25

SCANNING SIGNAL GENERATING CIRCUIT — 124

PC — 106a

SIGNAL PROCESSING CIRCUIT (SPECTRUM ANALYZER) — 105c

SIGNAL GENERATOR — 139

POWER AMPLIFIER — 122

SCANNING UNIT — 112

111

104a

LIGHT SOURCE — 103b

113a

138

137

140

102a

116

Ou

R101

F

θ

O

L1

L2

L4

D1

114

136

107

x

y

z

AP11

## FIG. 26

# FIG. 27

```
                    ┌──── START ────┐

S141 ──    GENERATION OF LIGHT

S142 ──    GENERATION OF ULTRASOUND WAVES

S143 ──    DOPPLER-PHENOMENON IS CAUSED IN A REGION
           PROXIMATE TO CONVERGENCE POINT

S144 ──    DETECTION AT PHOTO DETECTOR

S145 ──    DETECTION OF DOPPLER SHIFT
           Δf BY SPECTRUM ANALYZER

S146 ──    MEMORIZATION OF Δf Or "n1+Δn" IN MEMORY
           AS OPTICAL IMAGING INFORMATION
                                              S148

S147 ──    SCANNING END?    NO    MOVE ULTRASOUND
                                   CONVERGENCE POINT
              YES

S149 ──    IMAGE PRODUCTION AND IMAGE DISPLAY

S150 ──    SCREENING OF LESION SITE OF CANCER
           OR OTHER UTILIZATION

                      END
```

# FIG. 28

# FIG. 29

# FIG. 30

# FIG. 31

EP 1 810 610 B1

90

## FIG. 32

## FIG. 35

# FIG. 33

EP 1 810 610 B1

# FIG. 34

START

S141 — GENERATION OF LIGHT

S142 — GENERATION OF ULTRASOUND WAVE

S143 — DOPPLER-PHENOMENON IS CAUSED IN REGION PROXIMATE TO CONVERGENCE POINT

S144' — TRANSMISSION WAVELENGTH IS CHANGED BY LC TUNABLE FILTER

S145' — DETECTION AT PHOTO DETECTOR

S146 — MEMORIZATION OF Δf AND OTHER DATA IN MEMORY AS OPTICAL IMAGING INFORMATION

S147 — SCANNING END?

NO → S148 — MOVE ULTRASOUND CONVERGENCE POINT

YES

S149 — IMAGE PRODUCTION AND IMAGE DISPLAY

S150 — SCREENING OF LESION SITE OF CANCER OR OTHER UTILIZATION

END

# FIG. 36

EP 1 810 610 B1

# FIG. 37

EP 1 810 610 B1

FIG. 38

EP 1 810 610 B1

# FIG. 39

```
                    ( START )
                        │
                        ▼
S151 ─── GENERATION OF CONTINUOUS LIGHT
                        │
                        ▼
S152 ─── GENERATION OF PULSED ULTRASOUND WAVES ◄──────┐
                        │                              │
                        ▼                              │
        DOPPLER-SHIFTED REFLECTED LIGHT IS INCIDENT    │
        ON LC TUNABLE FILTER WITH REFRACTIVE INDEX     │
S153 ─── ALTERATION IN REGION PROXIMATE TO             │
        ULTRASOUND CONVERGENCE POINT                   │
                        │                              │
                        ▼                              │
        TRANSMISSION WAVELENGTH OF LC TUNABLE          │
S154 ─── FILTER IS CHANGED BY DRIVER                   │
                        │                              │
                        ▼                              │
        TRANSMITTED LIGHT FROM LC TUNABLE              │
S155 ─── FILTER IS RECEIVED BY PHOTO DETECTOR          │
                        │                              │
                        ▼                              │
        MEMORIZATION OF OUTPUT SIGNAL                  │
S156 ─── OF PHOTO DETECTOR IN MEMORY          S158     │
                        │                              │
          S157          ▼              NO    MOVE ULTRASOUND
             ◄  SCANNING END? ►─────────────► CONVERGENCE POINT ┘
                        │ YES
                        ▼
        IMAGE PRODUCTION AND DISPLAY OF IMAGE
S159 ─── INFORMATION BASED OF AMOUNT OF
        MODULATED FREQUENCY
                        │
                        ▼
                    ( END )
```

FIG. 40

# FIG. 41

EP 1 810 610 B1

## FIG. 42A
GENERATION OF CONTINUOUS LIGHT

## FIG. 42B
PULSED ULTRASOUND WAVES

## FIG. 42C
PULSED ULTRASOUND WAVES AT CONVERGENCE POINT

## FIG. 42D
DETECTION OF DOPPLER-SHIFTED LIGHT

## FIG. 42E
SCANNING SIGNAL

TIME

# FIG. 43

EP 1 810 610 B1

SCANNING UNIT — 149

LIGHT SOURCE (CONTINUOUS LIGHT) — 103b

114

113a

104a

148

148a 148b

138

DELAY CIRCUIT — 147

CONTROL CIRCUIT — 146

PULSE GENERATOR — 121

POWER AMPLIFIER — 122

OUTPUT SIGNAL DISPLAY DEVICE — 110

136

107

O

Fo

102a

111

Ou

AP19

PULSED ULTRASOUND WAVES

## FIG. 44

SCANNING UNIT — 149

LIGHT SOURCE (CONTINUOUS LIGHT) — 103b

114

150

113a

104a

138

148
148a  148b

DELAY CIRCUIT — 147

CONTROL CIRCUIT — 146

PULSE GENERATOR — 121

POWER AMPLIFIER — 122

OUTPUT SIGNAL DISPLAY DEVICE — 110

136

107

O

Fo

Ou

102a  111

PULSED ULTRASOUND WAVES

AP20

EP 1 810 610 B1

FIG. 45

## FIG. 46

103b

LIGHT
SOURCE

113a

102a

AP22

136

107

Ou

PULSED ULTRASOUND WAVES

FIG. 47

EP 1 810 610 B1

# FIG. 48

152b

160b

160a

# FIG. 49

152b

152c

160b

160a

153 { 153a
153b

160b

152d

160a

152b

FIG. 50

EP 1 810 610 B1

# FIG. 51

AP25

LIGHT SOURCE — 103b

152a

153

152c

114

156

106a

135

105c

SIGNAL PROCESSING CIRCUIT

104a

152d

152b

PC MAIN UNIT

106c

DELAY CIRCUIT — 123

171

172

177

176

175

173

136

107

PULSE GENERATOR — 121

POWER AMPLIFIER — 122

174

179

CONTROL CIRCUIT

SCANNING SIGNAL GENERATING CIRCUIT — 124

178

F

125

y

x

z

EP 1 810 610 B1

## FIG. 52

FROM POWER AMPLIFIER 122
FROM SCANNING SIGNAL GENERATING CIRCUIT 124

171

172

AP26

174

176

175

177

152b

173

158

157b

154b

159

F

## FIG. 53

152b

160b

160a

160c

Fo

*FIG. 54*

EP 1 810 610 B1

## FIG. 55

*FIG. 56*

PULSED LIGHT GENERATOR — 203

OBJECT INFORMATION PRODUCER — 206

SCATTERING INFORMATION EXTRACTOR — 205

REFLECTED-LIGHT RECEIVER — 204

PULSE SYNCHRO-NIZER — 208

PULSED ULTRA-SOUND GENERA-TOR — 202

213 — LIGHT SEPARATOR

REFERENCE LIGHT PRODUCER — 214

OBJECT INFORMATION ANALYZING APPARATUS

ULTRASOUND TRANSMISSION AXIS

LIGHT AXIS

OBJECT

REGION TO BE EXAMINED

# FIG. 57

EP 1 810 610 B1

# FIG. 58

LIGHT

fs

fs-Δf = fs- $\dfrac{2V\cos\theta(n_1+\Delta n)}{\lambda}$

ULTRASOUND
WAVES

207

θ

O

Ou

REFRACTIVE INDEX $n_1$ OF MEDIUM
(BIOLOGICAL TISSUE)

REFRACTIVE INDEX ALTERATION Δn
(IN REGION R201)

F

R201

V

# FIG. 59

```
                        ( START )
                           │
                           ▼
S251 ──  GENERATION OF PULSED ULTRASOUND WAVES
                           │
                           ▼
          GENERATION OF EACH PULSED LIGHT AT
S252 ──  PREDETERMINED TIMING AFTER GENERATION
             OF EACH PULSED ULTRASOUND WAVE
                           │
                           ▼
           DOPPLER-SHIFTED PULSED LIGHT IS
        REFLECTED AND RETURNED TO HALF MIRROR
S253 ──   WITH REFRACTIVE INDEX ALTERATION IN
           REGION PROXIMATE TO ULTRASOUND
                  CONVERGENCE POINT
                           │
                           ▼
S254 ──  DOPPLER-SHIFTED PULSED LIGHT IS DETECTED
                  BY PHOTO DETECTOR
                           │
                           ▼
S255 ──       VALUE OF Δf IS CALCULATED
                 BY SPECTRUM ANALYZER
                           │
                           ▼
S256 ──  MEMORIZATION OF Δf AND OTHER DATA IN          S258
         MEMORY AS OPTICAL IMAGING INFORMATION
                           │
    S257                   ▼                  NO    MOVE ULTRASOUND
        <  SCANNING END?  >  ─────────────────────  CONVERGENCE POINT
                           │
                          YES
                           ▼
S259 ──  IMAGE PRODUCTION AND DISPLAY OF IMAGE
                 INFORMATION ON Δf
                           │
                           ▼
                        ( END )
```

*FIG. 60A*

PULSED ULTRASOUND WAVES

*FIG. 60B*

PULSED ULTRASOUND WAVES
AT CONVERGENCE POINT

Tf

*FIG. 60C*

GENERATION OF PULSED BEAMS

*FIG. 60D*

DETECTION OF PULSED BEAMS

*FIG. 60E*

SCANNING SIGNAL

→ TIME

*FIG. 61*

PC

FOURIER
TRANSFORM

MEMORY

232

204a          205d

206a

# FIG. 62

# FIG. 63

EP 1 810 610 B1

FIG. 64

EP 1 810 610 B1

# FIG. 65

```
            ┌─────────────┐
            │    START    │
            └─────────────┘
                   │
S221 ──  ┌──────────────────────────────────────┐
         │  GENERATION OF PULSED ULTRASOUND WAVES │
         └──────────────────────────────────────┘
                   │
S222 ──  ┌──────────────────────────────────────┐
         │  GENERATION OF EACH PULSED LIGHT AT    │
         │  PREDETERMINED TIME AFTER GENERATION   │
         │  OF EACH PULSED ULTRASOUND WAVE        │
         └──────────────────────────────────────┘
                   │
S223 ──  ┌──────────────────────────────────────┐
         │  DIVISION OF PULSED LIGHT BY HALF MIRROR │
         └──────────────────────────────────────┘
```

S224a

PULSED LIGHT DIRECTED TO REFERENCE MIRROR IS MODULATED AND MADE INCIDENT ON HALF MIRROR TO SERVE AS REFERENCE PULSED LIGHT

S224b

PULSED LIGHT THAT HAVE TRANSMITTED HALF MIRROR IS RADIATED TO TISSUE TO BECOME SCATTERING PULSED LIGHT ACCOMPANIED BY COMPLEX REFRACTIVE INDEX ALTERATION DUE TO ULTRASOUND WAVE, AND MADE INCIDENT ON HALF MIRROR

S225 — INFERENCE BETWEEN REFERENCE PULSED LIGHT AND OBSERVATION PULSED LIGHT AT HALF MIRROR

S226 — HETERODYNE DETECTION FOR INTERFERENCE PULSED LIGHT BY PHOTO DETECTOR

S227 — DETECTION OF PHASE COMPONENTS AND OTHER DATA FROM INTERFERENCE SIGNAL BY OSCILLOSCOPE AND OTHERS

S228 — MEMORIZATION OF PHASE COMPONENTS AND OTHER DATA AS OPTICAL IMAGING INFORMATION

S229 — SCANNING END? — NO — S230 MOVE ULTRASOUND CONVERGENCE POINT

YES

S231 — IMAGE PRODUCTION AND IMAGE DISPLAY

END

FIG. 66

# FIG. 67

INTENSITY

INCIDENT PULSED LIGHT

REFLECTED PULSED LIGHT

TIME t

t = 0

## FIG. 68

EP 1 810 610 B1

# FIG. 69

252b

260b

260a

# FIG. 70

252a

252c

260b

260a

253 { 253a
      253b

260b

252b

252d

260a

# FIG. 71

EP 1 810 610 B1

FIG. 72

FIG. 73

EP 1 810 610 B1

## FIG. 74

EP 1 810 610 B1

## FIG. 75

FROM POWER AMPLIFIER 222
FROM SCANNING SIGNAL GENERATING CIRCUIT 224

271
272
274
276
275
254b
236
277
252b
273
258
257b
259

PULSED ULTRASOUND WAVE
207

F

PULSED LIGHT
BEAM

## FIG. 76

252b
260b
260a
260c
Fo

**EP 1 810 610 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2006012898 A
- JP 2007005680 A
- JP 2007005678 A
- JP 2007005679 A
- JP 2005224399 A
- JP 2000197635 A

### Non-patent literature cited in the description

- **MAKI HISAKA et al.** Optical tomogram observation at deep part of a scatterer utilizing interaction of pulsed ultrasound wave and light. *Optics,* 2000, vol. 29 (10), 631-634